(19) Europäisches Patentamt
European Patent Office
Office européen des brevets

(11) EP 2 226 315 A1

(12) EUROPEAN PATENT APPLICATION
published in accordance with Art. 153(4) EPC

(43) Date of publication:
**08.09.2010 Bulletin 2010/36**

(21) Application number: **08869161.3**

(22) Date of filing: **26.12.2008**

(51) Int Cl.:
***C07D 239/84*** (2006.01) ***A61K 31/517*** (2006.01)
***A61K 31/5377*** (2006.01) ***A61P 3/00*** (2006.01)
***A61P 5/00*** (2006.01) ***A61P 9/00*** (2006.01)
***A61P 11/06*** (2006.01) ***A61P 19/08*** (2006.01)
***A61P 25/00*** (2006.01) ***A61P 25/28*** (2006.01)
***A61P 29/00*** (2006.01) ***A61P 35/00*** (2006.01)
***A61P 37/06*** (2006.01) ***A61P 37/08*** (2006.01)
***A61P 43/00*** (2006.01) ***C07D 403/12*** (2006.01)
***C07D 405/12*** (2006.01) ***C07D 413/12*** (2006.01)
***C07D 417/12*** (2006.01)

(86) International application number:
**PCT/JP2008/073866**

(87) International publication number:
**WO 2009/084695 (09.07.2009 Gazette 2009/28)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **28.12.2007 JP 2007341248**

(71) Applicants:
- **Carna Biosciences Inc.**
  **Kobe-shi,**
  **Hyogo 650-0047 (JP)**
- **Crystalgenomics, Inc.**
  **Songpa-gu, Seoul 138-736 (KR)**

(72) Inventors:
- **SAWA, Masaaki**
  **Kobe-shi**
  **Hyogo 650-0047 (JP)**
- **YOKOTA, Koichi**
  **Kobe-shi**
  **Hyogo 650-0047 (JP)**
- **MORIYAMA, Hideki**
  **Kobe-shi**
  **Hyogo 650-0047 (JP)**
- **SHIN, Myoungyoup**
  **Seoul 138736 (KR)**
- **RO, Seonggu**
  **Seoul 138-736 (KR)**
- **CHO, Joong Myung**
  **Seoul 138736 (KR)**

(74) Representative: **TBK-Patent**
**Bavariaring 4-6**
**80336 München (DE)**

(54) **2-AMINOQUINAZOLINE DERIVATIVE**

(57) An object of the present invention is to provide compounds which are useful as protein kinase inhibitors. Disclosed is a 2-aminoquinazoline derivative represented by the following formula (I):

EP 2 226 315 A1

[Chemical Formula 1]

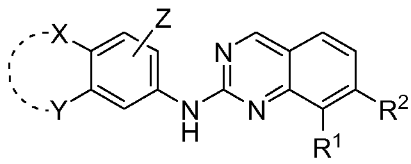

(I)

wherein $R^1$ represents a lower alkyl group which may be substituted with a halogen atom, or a halogen atom; $R^2$ represents a hydrogen atom, a substituted or unsubstituted lower alkyl group, a halogen atom, a hydroxyl group, a substituted or unsubstituted lower alkoxy group, a substituted or unsubstituted amino group, a substituted or unsubstituted acylamino group, a carboxyl group, a lower alkoxycarbonyl group, a carbamoyl group, or a substituted or unsubstituted lower alkylureido group; and X, Y and Z each independently represents a hydrogen atom, a substituted or unsubstituted lower alkyl group, a halogen atom, a hydroxyl group, a carboxyl group, a lower alkoxycarbonyl group, a cyano group, a carbamoyl group, a substituted or unsubstituted lower alkoxy group, a substituted or unsubstituted amino group, a substituted or unsubstituted lower alkoxycarbonylamino group, a substituted or unsubstituted lower alkylaminocarbonyl group, a lower alkylsulfonylamino group, a substituted or unsubstituted lower alkylureido group, or a substituted or unsubstituted acylamino group, or X and Y may be combined to form a 5- to 6-membered ring forming a bicyclic fused ring, wherein the 5- to 6-membered ring may optionally have a substituent, provided that when X and Y are not combined to form a fused ring, $R^2$ represents a hydrogen atom and, when X and Y are combined to form a fused ring, a saturated or unsaturated, bicyclic alicyclic or heterocyclic fused ring can be formed.

**Description**

BACKGROUND OF THE INVENTION

Field of the Invention

The present invention relates to 2-aminoquinazoline derivatives which are useful as pharmaceuticals particularly protein kinase inhibitors.

Description of the Related Art

Protein kinases are responsible for signal transduction involving proliferation, intercellular communication, and matters of subsistence by phosphorylation of tyrosine, serine, or threonine residues in its own sequence or in other protein. These signals have important roles in proliferation and matters of subsistence in normal cells (see, for example, Shchemelinin et al., Folia Biol (Praha), 2006, 52, 81).

These phosphorylation phenomena are induced in response to different stimuli including extracellular stimuli, for example, environmental and chemical stress signals (for example, osmotic stimulus, thermal stimulus, UV irradiation, bacterial endotoxin, etc.), cytokine (for example, interleukin-1 (IL-1), tumor necrosis factor-alpha (TNF-alpha), and growth factors (for example, granulocyte-macrophage colony-stimulating factor (GM-CSF) and fibroblast growth factor (FGF))).

Extracellular stimuli may affect more than one cell response involved in cell proliferation, migration, differentiation, hormone secretion, activation of transcription factor, muscular contraction, glucose metabolism, protein synthesis regulation, and cell cycle control.

In the past, it has been known that many diseases, such as autoimmune diseases, inflammatory diseases, bone diseases, metabolic disorders, neurological and neurodegenerative disorders, cancers, cardiovascular diseases, allergies and asthma, Alzheimer's disease, and hormonal-related disorders, relate to abnormal cell response mediated by the above-described protein kinases.

As protein kinases related with the above diseases, for example, it has been reported that the class III receptor tyrosine kinase family (Flt3, c-Kit, and PDGF receptors, and c-Fms) plays key roles in maintenance, proliferation and growth of hematopoietic and non-hematopoietic cells (see Scheijen, B., Griffin J. D., Oncogene, 2002, 21, 3314).

Flt3 and c-Kit receptors regulate maintenance of stem and early precursor cells, and growth of mature lymphocyte and myeloid cells (see Lyman, S., Jacobsen, S., Blood, 1998, 91, 1101). As downstream regulators of Flt3 and c-Kit receptors, PLCγ, PI3 kinase, Grb-2, SHIP, and Src-related kinases are known (see Scheijen, B., Griffin J. D., Oncogene, 2002, 21, 3314), and are demonstrated that these kinases play key roles in malignant tumors in various hematopoietic and non-hematopoietic cells. It is reported that mutations in Flt3 and c-Kit which induce ligand-independent activation are involved in the onset of acute myeloid leukemia (AML), acute lymphoid leukemia (ALL), mastocytosis, and gastrointestinal stromal tumor (GIST).

More recently, the Aurora kinase family was discovered as important regulators for mitosis involving G2 and M phase in cell division cycle.

The Aurora kinase family consists of tumor-related serine/threonine kinases which are localized in mitotic organs (centrosomes, spindle poles of bipolar-type spindles, or intermediates) and regulates completion of centrosome separation, organization of bipolar-type spindles and chromosome separation.

Three homologues of Aurora kinases have been identified (Aurora A, B, and C (these are referred to as Aurora 2, 1, and 3 in the literature, respectively)) (see Nigg et al., Nat. Rev. Mol. Cell. Biol., 2001, 2, 21). All of them share a highly conserved common catalytic domain located in carboxy termini, but their amino termini have no similarities in their sequence and stretch at various length (see Keen, N., Taylor, S., Nature Review Cancer 2004, 4, 927). Human Aurora kinases are expressed during cell proliferation, and are over expressed in a number of tumor cell lines derived from breasts, ovaries, prostates, pancreases, and colons.

Aurora A kinase is considered as a kinase involved in spindle formation and localized in centrosome in early G2 when it phosphorylates spindle-related proteins. Substantial overexpression of Aurora A is detected in breast cancer, ovary cancer, and pancreas cancer in humans (see Zhou et al., Nat. Genet. 1998, 20, 189; Tanaka et al., Cancer Res. 1999, 59, 2041; and Han et al., Cancer Res. 2002, 62, 2890).

Aurora B kinase functions as an oncogene, and transforms Rat1 fibroblasts and mouse NIH 3T3 cells *in vitro.* Aurora B also transforms NIH 3T3 cells growing as tumors in nude mice. Excess Aurora B can lead aneuploidy (numerical chromosome aberration) by accelerating loss of tumor suppressor genes and/or by magnifying events, which are known to contribute to oncogenes and cell transformation. Cells with excess Aurora B may avoid a mitotic checkpoint; as a result, proto-oncogenes come to be activated inappropriately. Upregulation of Aurora B is observed in many pancreas cancer cell lines. Furthermore, it is observed that prevention of cell cycles and increase of apoptosis are induced by treatment using antisense oligonucleotide of Aurora B kinase.

In cancer cells, as described above, signals are abnormally enhanced without appropriate regulation because of gene mutation, overexpression or activation of molecule with protein kinase activity. Therefore, compounds which inhibit protein kinase may be preventives or remedies by inhibiting growth of cancer cells or blocking survival signaling.
Next, examples include Janus kinases (JAK) (JAK is the tyrosine kinase family including JAK1, JAK2, JAK3, and TYK2.
This JAK plays an important role in cytokine signaling.
An example of downstream substrates of JAK family kinases includes STAT (signal transducer and activator of transcription) proteins.
It is known that JAK/STAT signaling is related with a number of immunological diseases caused by abnormal immune reaction (for example, allergies, asthma, autoimmune diseases (for example, transplantation rejection, chronic rheumatism, amyotrophic lateral sclerosis, and multiple sclerosis), solid tumors, and hematopoietic malignancy (e.g., leukemia and lymphoma) (see Shuai, K., Liu, B., Nature Review Immunol. 2003, 3, 900; and Seidel et al., Oncogene 2000, 19, 2645).
It is known that high-affinity immunoglobulin E receptors expressed on the cell surface of mast cells are cross-linked through sensibilization with allergens and release inflammation-inducibleediators including many vasoactive cytokines leading to an acute allergic reaction or an immediate (type I) hypersensitivity reaction (see Gordon et al., Nature 1990, 346, 274; and Galli, N., Engl. J. Med. 1993, 328, 257).
For example, Syk is a tyrosine kinase which plays an important role in degranulation of mast cells and activation of eosinophils mediated by the high-affinity immunoglobulin E receptor. Therefore, Syk kinase is involved in various allergic diseases including asthma (see Taylor et al., Mol. Cell. Biol., 1995, 15, 4149).
Apoptosis suppression of eosinophils is proposed as a primary mechanism involved in increase of blood and tissue eosinophils in the development of asthma. It is believed that IL-5 and GM-CSF cause the increase of blood and tissue eosinophils by being upregulated in asthma and inhibiting apoptosis of eosinophils. It is reported that inhibition of Syk kinase can block apoptosis suppression of eosinophils by cytokines through experiments using antisense (see Yousefi et al., J. Exp. Med. 1996, 183, 1407).
As described above, aberration of protein kinases is also involved in various diseases besides cancers. Therefore, compounds which inhibit these protein kinases may be preventives or remedies for these diseases and so on.
Furthermore, compounds provided by the present invention are also useful for research of kinases in biological and pathological phenomena, and intracellular signal transduction pathway mediated by such kinases; and for comparative evaluations of novel kinase inhibitors.
On the other hand, various 2-aminoquinazoline derivatives having a protein kinase inhibitory action, for example, 2-aminoquinazoline derivatives having a cyclin- dependent kinase inhibitory action (Cdks) (see WO2001/038315), 2-aminoquinazoline derivatives having an Raf protein kinase inhibitory action (see WO2005/037285), 2-aminoquinazoline derivatives having a protein kinase regulatory action (see WO2006/039718), 2-aminoquinazoline derivatives having a p38 MAP kinase inhibitory action (see WO2006/118256), and 2-aminoquinazoline derivatives having a PDK1 kinase inhibitory action (see WO2007/117607) are known.

DISCLOSURE OF INVENTION

The present inventors have synthesized various compounds and intensively studied about their pharmacological activities so as to solve the problems described above, and found that specific 2-aminoquinazoline derivatives or pharmaceutically acceptable salts thereof can achieve the object of the present invention, and thus the present invention has been completed. The object of the present invention is to provide novel compounds which have a protein kinase inhibitory action and are useful as pharmaceuticals.
The above object is achieved by the following first invention to seventeenth invention.

First Invention

A 2-aminoquinazoline derivative represented by the following formula (I):

[Chemical Formula 1]

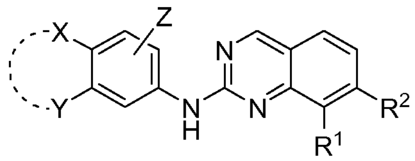

(I)

wherein $R^1$ represents a lower alkyl group which may be substituted with a halogen atom, or a halogen atom; $R^2$ represents a hydrogen atom, a substituted or unsubstituted lower alkyl group, a halogen atom, a hydroxyl group, a substituted or unsubstituted lower alkoxy group, a substituted or unsubstituted amino group, a substituted or unsubstituted acylamino group, a carboxyl group, a lower alkoxycarbonyl group, a carbamoyl group, or a substituted or unsubstituted lower alkylureido group; and X, Y and Z each independently represents a hydrogen atom, a substituted or unsubstituted lower alkyl group, a halogen atom, a hydroxyl group, a carboxyl group, a lower alkoxycarbonyl group, a cyano group, a carbamoyl group, a substituted or unsubstituted lower alkoxy group, a substituted or unsubstituted amino group, a substituted or unsubstituted lower alkoxycarbonylamino group, a substituted or unsubstituted lower alkylaminocarbonyl group, a lower alkylsulfonylamino group, a substituted or unsubstituted lower alkylureido group, or a substituted or unsubstituted acylamino group, or X and Y may be combined to form a 5- to 6-membered ring forming a bicyclic fused ring, wherein the 5- to 6-membered ring may optionally have a substituent, provided that when X and Y are not combined to form a fused ring, $R^2$ represents a hydrogen atom and, when X and Y are combined to form a fused ring, a saturated or unsaturated, bicyclic alicyclic or heterocyclic fused ring can be formed; or a pharmaceutically acceptable salt thereof.

Second Invention

The 2-aminoquinazoline derivative according to the first invention, wherein in the formula (I), $R^1$ is a lower alkyl group which may be substituted with a halogen atom, or a pharmaceutically acceptable salt thereof.

Third Invention

The 2-aminoquinazoline derivative according to the first invention, wherein in the formula (I), $R^1$ is a methyl group, or a pharmaceutically acceptable salt thereof.

Fourth Invention

The 2-aminoquinazoline derivative according to the first invention, wherein in the formula (I), $R^1$ is a halogen atom, or a pharmaceutically acceptable salt thereof.

Fifth Invention

The 2-aminoquinazoline derivative according to the first invention, wherein in the formula (I), $R^1$ is a methyl group and $R^2$ is a hydrogen atom, or a pharmaceutically acceptable salt thereof.

Sixth Invention

The 2-aminoquinazoline derivative according to the first invention, wherein in the formula (I), $R^1$ is a methyl group and $R^2$ is a hydroxyl group or a substituted or unsubstituted amino group, or a pharmaceutically acceptable salt thereof.

Seventh Invention

The 2-aminoquinazoline derivative according to any one of the first invention to the sixth invention, wherein in the formula (I), X and Y are combined to form a bicyclic fused ring, or a pharmaceutically acceptable salt thereof.

Eighth Invention

The 2-aminoquinazoline derivative according to the seventh invention, wherein the bicyclic fused ring is a heterocyclic fused ring, or a pharmaceutically acceptable salt thereof.

Ninth Invention

The 2-aminoquinazoline derivative according to the eighth invention, wherein the heterocyclic fused ring is a 1H-indazol-6-yl group, or a pharmaceutically acceptable salt thereof.

Tenth Invention

The 2-aminoquinazoline derivative according to the eighth invention, wherein the heterocyclic fused ring is a 1H-indol-6-yl group, or a pharmaceutically acceptable salt thereof.

Eleventh Invention

The 2-aminoquinazoline derivative according to the eighth invention, wherein the heterocyclic fused ring is a 1H-benzo[d]imidazol-6-yl group, or a pharmaceutically acceptable salt thereof.

Twelfth Invention

The 2-aminoquinazoline derivative according to any one of the ninth invention to the eleventh invention, wherein hydrogen atoms of the heterocyclic fused ring each independently may be substituted with Z, or a pharmaceutically acceptable salt thereof.

Thirteenth Invention

The 2-aminoquinazoline derivative according to the twelfth invention, wherein Z each independently represents a methyl group, an amino group or a hydroxyl group, or a pharmaceutically acceptable salt thereof.

Fourteenth Invention

A 2-aminoquinazoline derivative represented by the following formula (II):

[Chemical Formula 2]

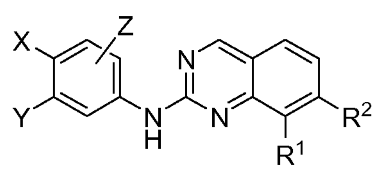

(II)

wherein $R^1$ represents a lower alkyl group which may be substituted with a halogen atom, or a halogen atom; $R^2$ represents a hydrogen atom; and X, Y and Z each independently represents a hydrogen atom, a substituted or unsubstituted lower alkyl group, a halogen atom, a hydroxyl group, a carboxyl group, a cyano group, a carbamoyl group, a substituted or unsubstituted lower alkoxy group, a substituted or unsubstituted amino group, or a substituted or unsubstituted acylamino group; or a pharmaceutically acceptable salt thereof.

Fifteenth Invention

The 2-aminoquinazoline derivative according to the fourteenth invention, wherein in the formula (II), $R^1$ is a lower alkyl group which may be substituted with a halogen atom, or a pharmaceutically acceptable salt thereof.

Sixteenth Invention

The 2-aminoquinazoline derivative according to the fourteenth invention, wherein in the formula (II), $R^1$ is a halogen atom; or a pharmaceutically acceptable salt thereof.

Seventeenth Invention

The 2-aminoquinazoline derivative according to any one of the fourteenth invention to the sixteenth invention, wherein X and Y each independently represents a hydrogen atom, a hydroxyl group, a substituted or unsubstituted lower alkoxy group, a substituted or unsubstituted amino group, or a substituted or unsubstituted acylamino group, or a pharmaceutically acceptable salt thereof.

The 2-aminoquinazoline derivatives or pharmaceutically acceptable salts thereof of the present invention are effective as pharmaceuticals for prevention or treatment of diseases which are known to be related to abnormal cell response mediated by protein kinase for example, autoimmune diseases, inflammatory diseases, bone diseases, metabolic disorders, neurological and neurodegenerative disorders, cancers, cardiovascular diseases, allergies and asthma, Alzheimer's disease, and hormonal-related disorders. They are also useful as inhibitors of protein kinases, and reagents for tests and studies.

BEST MODE FOR CARRYING OUT THE INVENTION

[2-Aminoquinazoline Derivatives of the Present Invention]

The present invention will now be described in detail.

The 2-aminoquinazoline derivatives of the present invention are compounds represented by the following formula (I), or compounds represented by the following formula (II):

[Chemical Formula 3]

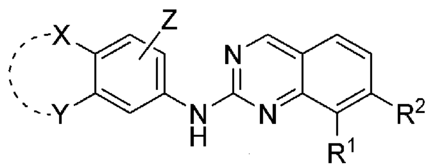

(I)

wherein $R^1$ represents a lower alkyl group which may be substituted with a halogen atom, or a halogen atom; $R^2$ represents a hydrogen atom, a substituted or unsubstituted lower alkyl group, a halogen atom, a hydroxyl group, a substituted or unsubstituted lower alkoxy group, a substituted or unsubstituted amino group, a substituted or unsubstituted acylamino group, a carboxyl group, a lower alkoxycarbonyl group, a carbamoyl group, or a substituted or unsubstituted lower alkylureido group; and X, Y and Z each independently represents a hydrogen atom, a substituted or unsubstituted lower alkyl group, a halogen atom, a hydroxyl group, a carboxyl group, a lower alkoxycarbonyl group, a cyano group, a carbamoyl group, a substituted or unsubstituted lower alkoxy group, a substituted or unsubstituted amino group, a substituted or unsubstituted lower alkoxycarbonylamino group, a substituted or unsubstituted lower alkylaminocarbonyl group, a lower alkylsulfonylamino group, a substituted or unsubstituted lower alkylureido group, or a substituted or unsubstituted acylamino group, or X and Y may be combined to form a 5- to 6-membered ring forming a bicyclic fused ring, wherein the 5- to 6-membered ring may optionally have a substituent, provided that when X and Y are not combined to form a fused ring, $R^2$ represents a hydrogen atom and, when X and Y are combined to form a fused ring, a saturated or unsaturated, bicyclic alicyclic or heterocyclic fused ring can be formed; or a pharmaceutically acceptable salt thereof.

[Chemical Formula 4]

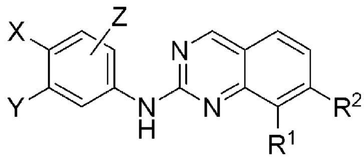

(II)

wherein $R^1$ represents a lower alkyl group which may be substituted with a halogen atom, a halogen atom; $R^2$ represents a hydrogen atom; and X, Y and Z each independently represents a hydrogen atom, a substituted or unsubstituted lower alkyl group, a halogen atom, a hydroxyl group, a carboxyl group, a cyano group, a carbamoyl group, a substituted or unsubstituted lower alkoxy group, a substituted or unsubstituted amino group, or a substituted or unsubstituted acylamino group.

In the present invention, the lower alkyl group which may be substituted with a halogen atom as $R^1$ may be any C1-C3 linear, branched or cyclic alkyl group, and specific examples thereof include a methyl group, an isopropyl group and a cyclopropyl group, of which a methyl group is particularly preferable.

Examples of $R^1$ itself or the halogen atom used as a substituent of the lower alkyl group include a fluorine atom, a chlorine atom and a bromine atom etc.

Therefore, specific examples of the lower alkyl group substituted with a halogen atom include a trifluoromethyl group.

The substituted or unsubstituted lower alkyl group in $R^2$ represents a substituted or unsubstituted C1-C6 linear, branched or cyclic alkyl group.

The substituted lower alkyl group represents an alkyl group which is substituted with a substituted or unsubstituted amino group, or a substituted or unsubstituted alkoxy group etc., and may be substituted with a halogen atom. Specific examples thereof include a morpholinomethyl group, a pyrrolidinoethyl group and a methoxyethyl group.

Examples of the halogen atom in $R^2$ include a fluorine atom, a chlorine atom and a bromine atom etc.

The substituted or unsubstituted lower alkoxy group represents an alkoxy group having a substituted or unsubstituted C1-C6 linear, branched or cyclic alkyl group, or an alkoxy group in which a carbon atom(s) of a linear, branched or cyclic alkyl group may be substituted with a hetero atom(s), and may be substituted with a halogen atom. Specific examples thereof include a methoxy group, a 2-morpholinoethyloxy group, a 2-pyrrolidinoethyloxy group, a 2-dimethylaminoethyloxy group, a 2-ethoxyethyloxy group etc.

The substituted or unsubstituted amino group represents an amino group having a substituted or unsubstituted C1-C6 linear, branched or cyclic alkyl group, or an amino group in which a carbon atom(s) of a linear, branched or cyclic alkyl group may be substituted with a hetero atom(s), and may be substituted with a halogen atom. Specific examples thereof include a 2-(pyrrolidin-1-yl)ethyl group, a 2-morpholinoethylamino group, a 2-morpholinopropylamino group, a 4-methylpiperazin-1-yl group, a 2-methoxyethylamino group, pyrrolidin-1-yl group, a 2-(dimethylamino)ethylamino group, a 2-hydroxyethylamino group, a 2-(piperazin-1-yl)ethylamino group, a dimethylamino group, a 4-hydroxymethylpiperazino group, a piperidin-1-yl group, a methylamino group, a 4-(2-hydroxyethyl)piperidin-1-yl group, an ethylamino group, a diethylamino group etc.

The substituted or unsubstituted acylamino group represents a C1-C6 linear or branched alkylcarboxyamino group.

The lower alkoxycarbonyl group represents a C1-C6 linear, branched or cyclic alkoxycarbonyl group, and specific examples thereof include a methoxycarbonyl group etc.

The substituted or unsubstituted lower alkylureido group represents a substituted or unsubstituted C1-C6 linear, branched or cyclic alkylureido group, and specific examples thereof include an ethylureido group etc.

The substituted or unsubstituted lower alkyl group in X, Y and Z represents a substituted or unsubstituted C1-C6 linear, branched or cyclic alkyl group, and the substituted lower alkyl group represents an alkyl group which may be substituted with a substituted or unsubstituted amino group, or a substituted or unsubstituted alkoxy group, and these groups may be substituted with a halogen atom.

The halogen atom represents a fluorine atom, a chlorine atom or a bromine atom.

The lower alkoxycarbonyl group represents a C1-C6 linear or branched alkyloxycarbonyl group, and specific examples thereof include a methoxycarbonyl group etc.

The substituted or unsubstituted lower alkoxy group represents an alkoxy group having a substituted or unsubstituted C1-C6 linear, branched or cyclic alkyl group, or an alkoxy group in which a carbon atom(s) of a linear,

branched or cyclic alkyl group may be substituted with a hetero atom(s), and these groups may be substituted with a halogen atom.

The substituted or unsubstituted amino group represents an amino group having a substituted or unsubstituted C1-C6 linear, branched or cyclic alkyl group, or an amino group in which a carbon atom(s) of a linear, branched or cyclic alkyl group may be substituted with a hetero atom(s), and these groups may be substituted with a halogen atom.

The substituted or unsubstituted lower alkoxycarbonylamino group represents a substituted or unsubstituted C1-C6 linear, branched or cyclic alkyloxycarbonylamino group, and specific examples thereof include an ethoxycarbonylamino group etc. The substituted or unsubstituted lower alkylaminocarbonyl group represents a substituted or unsubstituted C1-C6 linear, branched or cyclic alkylaminocarbonyl group in which a carbon atom(s) of a linear, branched or cyclic alkyl group may be substituted with a hetero atom(s). Specific examples thereof include a 2-dimethylaminoethylaminocarbonyl group, a 2-morpholinoethylaminocarbonyl group etc.

The lower alkylsulfonyl amino group represents a C1-C6 linear, branched or cyclic alkylsulfonyl amino group.

The substituted or unsubstituted lower alkylureido group represents a substituted or unsubstituted C1-C6 linear, branched or cyclic alkylureido group, and specific examples thereof include an ethylureido group etc.

The substituted or unsubstituted acylamino group represents a substituted or unsubstituted C1-C6 linear, branched or cyclic alkylcarbonylamino group.

When X and Y are combined to form a fused ring, they may be combined to form a 5- to 6-membered ring or they may be combined to form a bicyclic fused ring which optionally has a substituent. When X and Y are combined to form a fused ring, a saturated or unsaturated bicyclic alicyclic or heterocyclic fused ring can be formed. Specific examples thereof include bicyclic fused rings such as indazole, benzotriazole, benzoimidazole, dihydroindene, indole, benzodioxol, dihydrobenzodioxine, benzooxazin-3-one and benzothiazole. More specific examples of the bicyclic fused ring include a 1H-indazol-6-yl group, a 1H-indol-6-yl group, 1H-benzo[d]imidazol-6-yl group etc.

[Pharmaceutically Acceptable Salt of 2-Aminoquinazoline Derivatives of the Present Invention]

The 2-aminoquinazoline derivatives of the present invention can be converted into pharmaceutically acceptable salts by a known method. Examples of salts include alkali metal salts with sodium and potassium; alkali earth metal salts with magnesium and calcium; organic amine salts with a lower alkylamine and a lower alcoholamine; basic amino acid salts with lysine, arginine and ornithine; and ammonium salts. Salts further include inorganic acid salts with hydrochloric acid, sulfuric acid, carbonic acid and phosphoric acid; and organic acid salts with fumaric acid, maleic acid, methanesulfonic acid and p-toluenesulfonic acid.

The compounds of the present invention can be produced by the method described below. In the following method, when defined groups vary under the conditions of the method to be carried out or are not suited for carrying out the method, the compounds of the present invention can be easily produced by adding a conventional method used in organic synthetic chemistry, for example, means such as protection or deprotection of a functional group [T. W. Greene, Protective Groups in Organic Synthesis, 3rd Edition, John Wiley & Sons, Inc., 1999]. If necessary, the order of the reaction process such as introduction of substituents can vary.

Abbreviations and symbols used in the following description are as follows.

NMP: N-methylpyrrolidone
$POCl_3$: Phosphoryl chloride
$CH_2Cl_2$: Dichloromethane
WSC: 1-ethyl-3-(dimethylaminopropyl)carbodiimide hydrochloride
HOBt: 1-hydroxy-1H-benzotriazole
THF: Tetrahydrofuran
DIEA: N,N-diisopropylethylamine
Xantphos: 4,5-bis(diphenylphosphino)-9,9-dimethylxanthene

The compounds represented by the formula (I) of the present invention can be produced, for example, in accordance with Scheme 1:

[Scheme 1]

[Chemical Formula 5]

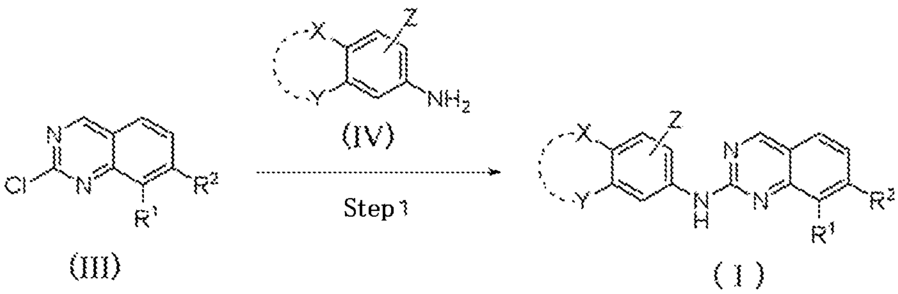


wherein $R^1$, $R^2$, X, Y and Z are as defined above.

The compound (I) can be obtained by reacting a compound (III) and 1 to 5 mol equivalents, preferably 1 to 1.5 mol equivalents of a compound (IV) with heating in a solvent, in the presence of an acid catalyst such as hydrochloric acid, if necessary.

The solvent is not specifically limited as long as it is inert to the reaction and, for example, it is possible to use a lower alcohol, and preferably n-butanol.

The reaction can be carried out by heating at a temperature of 100 to 150°C for 3 to 24 hours. Preferably, the above compound can be synthesized by reacting at a temperature of 120 to 130°C for 10 to 25 minutes using a microwave synthesis apparatus.

If necessary, it is possible to further add a step such as ester decomposition etc.

The compound (IV) as one raw material of Scheme 1 can be obtained as a commercially available product (for example, a product manufactured by SIGMA-ALDRICH Co.) or obtained by a known method or a method analogous thereto.

The compound (III) as the other raw material of Scheme 1 can be produced, for example, by the method shown in Scheme 2:

[Scheme 2]

[Chemical Formula 6]

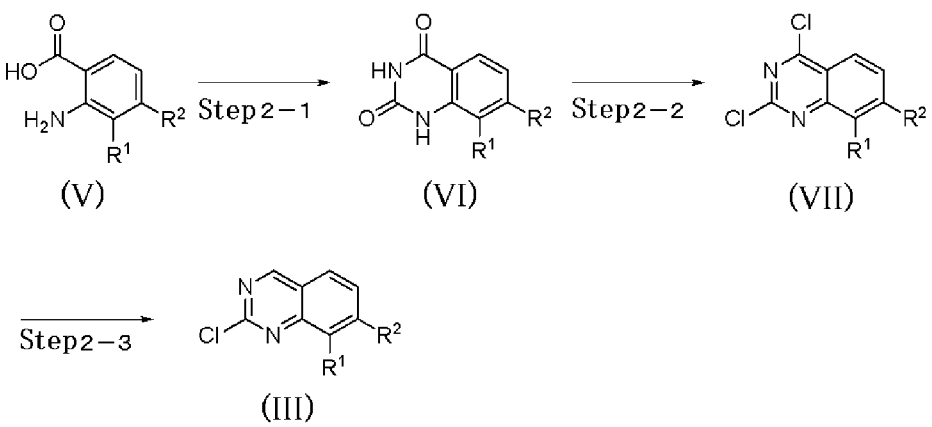

wherein $R^1$ and $R^2$ are as defined above.

Step 2-1

This step can be carried out by a known method [see, for example, WO0121598] or a method analogous thereto.
Namely, the compound (VI) can be obtained by reacting a compound (V) and urea with heating in a solvent.
The solvent is not specifically limited as long as it is inert to the reaction and, for example, it is possible to use NMP, DMF and 1,4-dioxane, and preferably NMP.
The reaction is completed at a temperature of 150 to 200°C, and preferably 175 to 190°C, within 3 to 6 hours.
The compound (V), which is a starting material of Scheme 2, can be obtained as a commercially available product (for example, a product manufactured by SIGMA-ALDRICH Co.) or obtained by a known method [see, for example, J. Med. Chem. 1991, 34, 217] or an analogous method thereof.

Step 2-2

This step can be carried out by a known method [see, for example, Japanese Translation No. 2-502462 of the PCT Application] or a method analogous thereto.
Namely, a compound (VII) can be obtained by reacting the compound (VI) with $POCl_3$ in the presence of dimethylaniline.
The reaction is completed at a temperature of 100 to 150°C, and preferably 100 to 130°C, within 3 to 6 hours.

Step 2-3

This step can be carried out by a known method [see, for example, U.S. Patent No. 6,040,488] or a method analogous thereto.
The compound (III) can be obtained by selectively removing a chloro group at the 4-position of the compound (VII). Namely, it can be synthesized by adding a zinc powder in a two-layer mixed solution of $CH_2Cl_2$ and an ammonia-containing saturated brine solution and reacting the solution under reflux conditions for 3 to 6 hours.
As shown in Scheme 3, the compound (III) can also be produced by treating the compound (VII) obtained in the step 2-2 of Scheme 2 with ammonia and reacting the compound with nitrite esters:

[Scheme 3]

[Chemical Formula 7]

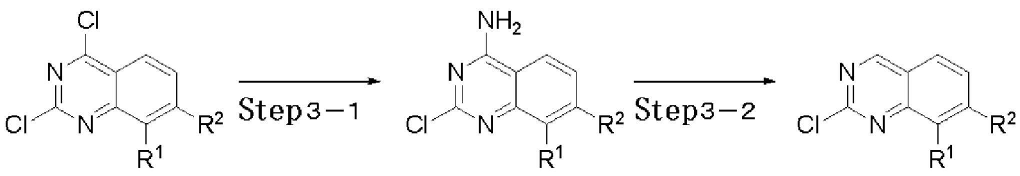


(VII) (VIII) (III)

wherein $R^1$ and $R^2$ are as defined above.
This step can be carried out by a known method [see, for example, U.S. Patent Application Publication No. 2004/0209904] or a method analogous thereto. Specifically, the step can be carried out in the following manner.

Step 3-1

A compound (VIII) can be obtained by aminating with an ammonia in a solvent in a position-selective manner.
The solvent is not specifically limited as long as it is inert to the reaction and, for example, THF can be preferably used.
It is possible to use, as an ammonia source, a commercially available ammonia solution, and preferably a 7N methanol solution. The reaction is completed at a temperature of 0°C to room temperature within 3 to 48 hours.

Step 3-2

The compound (III) can be obtained by reacting the compound (VIII) with a nitrite ester in a solvent.
The solvent is not specifically limited as long as it is inert to the reaction and, for example, THF can be preferably used.
It is possible to preferably use, as the nitrite ester, tert-butyl nitrite or isoamyl nitrite.
The reaction is completed at a temperature of 60 to 100°C within 4 to 24 hours.
The compound represented by the formula (I) of the present invention can also be produced, for example, in accordance with Scheme 4:

[Scheme 4]

[Chemical Formula 8]

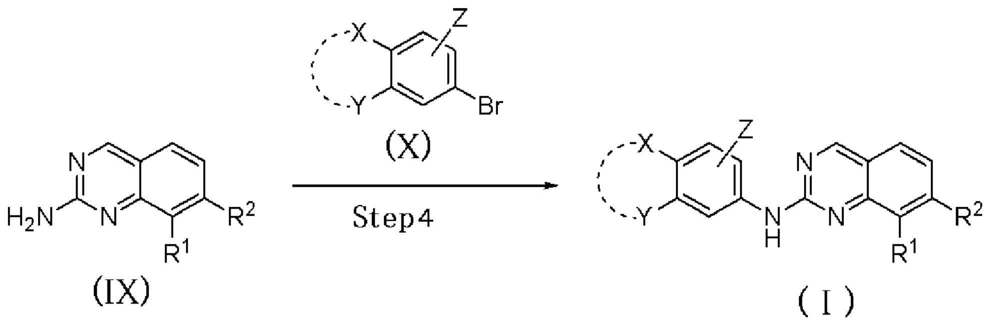


wherein $R^1$, $R^2$, X, Y and Z are as defined above.
The compound (I) can be obtained by reacting a compound (IX) and 1 to 5 mol equivalents, preferably 1 to 1.5 mol equivalents of a compound (X) with heating in a solvent in the presence of a base catalyst and a palladium catalyst, if necessary.
The solvent is not specifically limited as long as it is inert to the reaction and, for example, it is possible to use NMP, DMF and 1,4-dioxane, and preferably 1,4-dioxane.
As the base, sodium carbonate, potassium carbonate and cesium carbonate can be used.
It is possible to use, as the palladium catalyst, a palladium source and a phosphine ligand which are commonly used, and preferably tris(dibenzylideneacetone)dipalladium as the palladium source and Xantphos as the ligand.
The reaction can be carried out by heating at a temperature of 100 to 150°C for 3 to 24 hours.
The compound (X) can be obtained as a commercially available product (for example, a product manufactured by SIGMA-ALDRICH Co.) or obtained by a known method or a method analogous thereto.
The compound (IX), which is a raw material of Scheme 4, can be produced, for example, by the method shown in Scheme 5:

[Scheme 5]

[Chemical Formula 9]

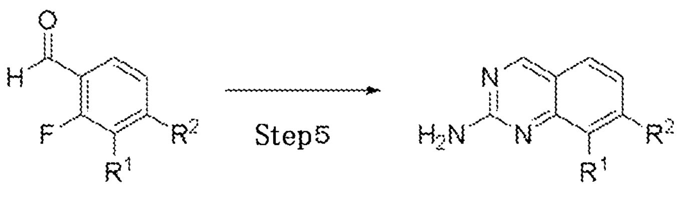


(XI) (IX)

wherein $R^1$ and $R^2$ are as defined above.

This step can be carried out by a known method [see, for example, J. Org. Chem. 2006, 71, 3959] or a method analogous thereto.

The compound (IX), which is a starting material of Scheme 4, can be obtained by reacting a compound (XI) with guanidine with heating in a solvent in the presence of a base catalyst, if necessary.

The compound (XI) can be obtained as a commercially available product (for example, a product manufactured by SIGMA-ALDRICH Co.) or obtained by a known method or a method analogous thereto.

The solvent is not specifically limited as long as it is inert to the reaction and, for example, it is possible to use NMP, DMF and 1,4-dioxane, and preferably NMP.

Examples of the base include triethylamine, tributylamine and DIEA.

The reaction is completed at a temperature of 150 to 160°C within 1 to 3 hours.

Among the compound (I), a compound (Ib) in which $R^1$ represents a lower alkyl grounp which may be substituted with a halogen atom, $R^2$ is $NR^3R^4$ in which $R^3$ and $R^4$ each independently represents a hydrogen atom, a substituted or unsubstituted lower alkyl group, or $R^3$ and $R^4$ may be combined to form a 5- or 6-membrebed ring forming a bicyclic fused ring, wherein the 5- to 6-membered ring may optionally have a substituent, provided that when $R^3$ and $R^4$ are combined to form a fused ring, a saturated or unsaturated, bicyclic alicyclic or heterocyclic fused ring can be formed can be prepared by the following method.

[Scheme 6]

[Chemical Formula 10]

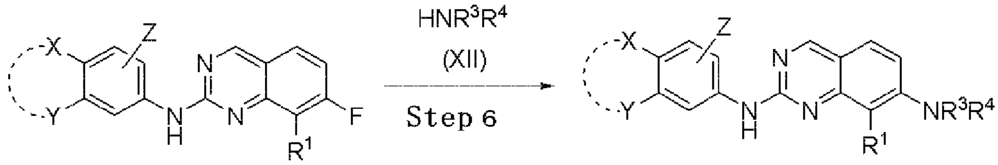


(Ia) (Ib)

wherein $R^1$, $R^3$, $R^4$, X, Y and Z are as defined above.

The compound (Ib) can be prepared by reacting a compound (Ia) and 1 to 5 mol equivalents, preferably 2 to 3 mol equivalents of a compound (XII) with heating in a solvent.

The solvent is not specifically limited as long as it is inert to the reaction and, for example, it is possible to use NMP, DMF, 1,4-dioxane etc., and preferably NMP.

The reaction can be carried out by heating at a temperature of 100 to 150°C for 3 to 48 hours. Preferably, the above compound can be synthesized by reacting at a temperature of 120 to 140°C for 10 to 60 minutes using a microwave synthesis apparatus.

The compound (XII) can be obtained as a commercially available product (for example, a product manufactured by SIGMA-ALDRICH Co.) or obtained by a known method or a method analogous thereto.

The compound (Ia) can be obtained by the same reaction as in Schemes 1 and 2.

Among the compound (I), a compound (Ic) in which $R^1$ is a lower alkyl group which may be substituted with a halogen atom and $R^2$ is an unsubstituted amino group, for example, can be obtained by the method described below.

[Scheme 7]

[Chemical Formula 11]

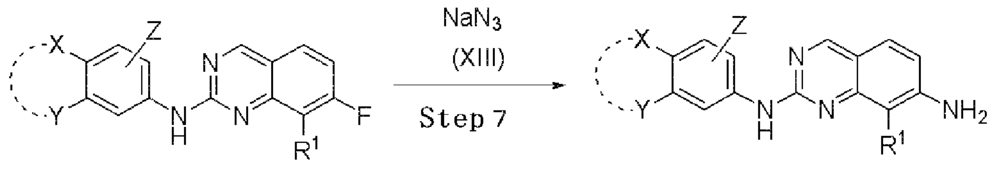


(Ia) (Ic)

wherein $R^1$, X, Y and Z are as defined above.

The compound (Ic) can be obtained by reacting a compound (Ia) and 1 to 20 mol equivalents, preferably 5 to 10 mol equivalents of sodium azide with heating in a solvent in the presence of 1 to 1.5 mol equivalents of 18-crown 6-ether.

The solvent is not specifically limited as long as it is inert to the reaction and, for example, it is possible to use NMP, DMF, 1,4-dioxane etc., and preferably DMF.

The reaction can be carried out by heating at a temperature of 100 to 150°C for 3 to 24 hours.

Among the compound (I), a compound (Id) in which $R^1$ is a lower alkyl group which may be substituted with a halogen atom and $R^2$ is $OR^5$ (in which $R^5$ represents a substituted or unsubstituted lower alkyl group), for example, can be obtained by the method described below.

[Scheme 8]

[Chemical Formula 12]

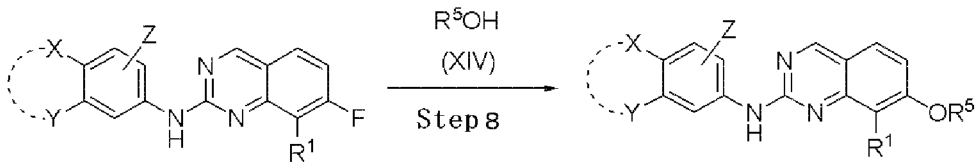


(Ia) (Id)

wherein $R^1$, $R^5$, X, Y and Z are as defined above.

The compound (Id) can be obtained by reacting a compound (Ia) and 1 to 5 mol equivalents of a compound (XIV) in a solvent in the presence of 5 to 20 mol equivalents of a base. The solvent is not specifically limited as long as it is inert to the reaction and, for example, it is possible to use NMP, DMF, 1,4-dioxane etc., and preferably DMF.

The reaction can be carried out by heating at a temperature of 70 to 150°C for 3 to 24 hours.

As the base, sodium hydride, sodium hydroxide, lithium hydroxide, potassium hydroxide etc. can be used, and sodium hydride can be preferably used.

The compound (XIV) can be obtained as a commercially available product (for example, a product manufactured by SIGMA-ALDRICH Co.) or obtained by a known method or a method analogous thereto.

Among the compound (I), a compound (If) in which $R^1$ is a lower alkyl group which may be substituted with a halogen atom and $R^2$ is a hydroxyl group, for example, can be obtained by the method described below.

[Scheme 9]

[Chemical Formula 13]

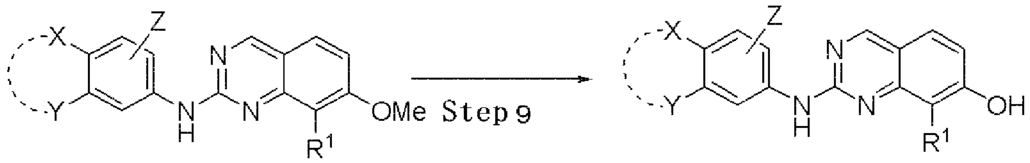


(Ie) (If)

wherein $R^1$, X, Y and Z are as defined above.

The compound (If) can be obtained by subjecting to the conditions of a demethylation reaction of arylmetylether used usually in an organic synthetic chemistry [method described in T. W. Greene, Protective Groups in Organic Synthesis 3rd Edition, John Wiley & Sons, Inc.,1999, p.249 or a method analogous thereto].

The compound (Ie) can be obtained by the same reaction as in Schemes 1 and 2.

The effect of the present invention will now be described by way of Test Example.

Bioactivity of the compounds of the present invention was measured by the following method.

Test Example 1

Measurement of Bioactivity

1. Preparation and Storage of Test Compound Solution

A test compound was dissolved in DMSO to obtain a 10 mM test compound solution, which was stored in a dark place at - 20°C until measurement. When bioactivity (kinase activity) is measured, a test compound solution is diluted with DMSO so that the concentration becomes 100 times higher than that of the above test compound solution, and then diluted 25 times (DMSO concentration is 4%) with an assay buffer described hereinafter.

2. Measurement of Kinase Activity

Products manufactured by Carna Biosciences Inc. (Kobe) used as protein kinases (recombinant human kinases) in the test are as follows:

SYK (product number: 08-176), JAK3 (product number: 08-046), FLT3 (product number: 08-154), PDGFRa (PDGFRA) (product number: 08-157), TRKA (NTRK1) (product number: 08-186), KDR (product number: 08-191), CDK2/cycA (product number: 04-103), and AurA (AURKA) (product number: 05-101).

Kinase activity was measured by a mobility shift assay (MSA) method using QuickScout Screening Assist™ MSA (commercially available kit, manufactured by Carna Biosciences Inc.)

5 μl of a test compound solution (the concentration is 4 times of the final concentration) dissolved or suspended in an assay buffer [20 mM HEPES, 0.01% Triton™ X-100, 1 mM dithiothreitol, pH 7.5] or 5 μl of a solvent (4% DMSO-assay buffer) was charged in a 384 well plate made of polypropylene (product number 781280, manufactured by Greiner Bio-One Co., Ltd.). Then, 5 μl of ATP/substrate/metal solution of QuickScout Screening Assist MSA was added. Furthermore, 10 μl of a kinase solution diluted with assay buffer was added and the reaction was initiated. However, only assay buffer (10 μl) was added to blank. The concentration of kinase and reaction conditions were controlled in accordance with the protocol of QuickScout Screening Assist MSA. Then, the reaction was terminated by adding 60 μl of termination buffer of QuickScout Screening Assist MSA and the amount of a substrate (S) and a phosphorylated substrate (P) in the reaction solution were determined in accordance with the protocol of QuickScout Screening Assist MSA using LabChip3000 (manufactured by Caliper Life Sciences Co., MA, USA), and also inhibition of the test compound at a

concentration of 0.01, 0.1 and 1 μM was measured, respectively. The amounts of S and P were expressed by the height of each separated peak. Inhibition (%) of the test compound was calculated in accordance with the following equation:

$$\text{Inhibition (\%)} = (1 - (C - A)/(B - A)) \times 100$$

where A denotes blank, B denotes a solvent and C denotes P/(P + S) of a well of the compound.

3. Inhibitory Action against Kinase

In the manner described above, an inhibitory action of the compound of the present invention against various protein kinases was studied. As a result, the compounds of the present invention exerted excellent inhibitory activity. For example, N-(benzo[d][1,3]dioxol-5-yl)-8-methylquinazoline-2-amine (a compound of Example 6) and N-(2,3-dihydrobenzo[b][1,4]dioxin-6-yl)-8-methylquinazoline-2-amine (a compound of Example 7) exerted 50% or more of inhibitory activity against Flt3 kinase at 0.1 μM, and 6-(8-methylquinazolin-2-ylamino)-2H-benzo[b][1,4]oxazin-3(4H)-one (a compound of Example 8), N-(3-(8-methylquinazolin-2-ylamino)phenyl)acetamide (a compound of Example 11) and 8-chloro-N-(1H-indazol-6-yl)quinazoline-2-amine (a compound of Example 32) exerted 50% or more of inhibitory activity at 0.01 μM.

N-(1H-indol-6-yl)-8-methylquinazoline-2-amine (a compound of Example 5) exerted 50% or more inhibitory activity against AurA kinase at 0.1 μM, N-(3,4-dimethylphenyl)-8-methylquinazoline-2-amine (a compound of Example 30) exerted 50% or more of inhibitory activity against JAK2 and JAK3 kinases at 0.1 μM, furthermore, N-(1H-indazol-6-yl)-8-methylquinazoline-2-amine (a compound of Example 1), N-(1H-benzo[d][1,2,3]triazol-5-yl)-8-methylquinazoline-2-amine (a compound of Example 2), 5-(8-methylquinazolin-2-ylamino)-2-methylphenol (a compound of Example 29), 8-methyl-N-(3-methyl-1H-indazol-6-yl)-quinazoline-2-amine (a compound of Example 34), 8-methyl-N-(4-methyl-1H-indazol-6-yl)-quinazoline-2-amine (a compound of Example 37), 7-hydroxy-N-(1H-indazol-6-yl)-8-methylquinazoline-2-amine (a compound of Example 48), 8-methyl-N-(4-amino-1H-indazol-6-yl)quinazoline-2-amine (a compound of Example 52), 2-[2-(1H-indazol-6-ylamino)-8-methylquinazolin-7-ylamino]ethanol (a compound of Example 73), 7-amino-N-(1H-indazol-6-yl)-8-methylquinazoline-2-amine (a compound of Example 74), 8-methyl-N-(4-hydroxy-1H-indazol-6-yl)quinazoline-2-amine (a compound of Example 82), {1-[2-(1H-indazol-6-ylamino)-8-methylquinazolin-7-yl]piperidin-4-yl}methanol (a compound of Example 84), 8-methyl-N-(2-methyl-4-amino-1H-benzo[d]imidazol-6-yl)quinazoline-2-amine (a compound of Example 88), {1-[2-(3-methyl-1H-indazol-6-ylamino)-8-methylquinazolin-7-yl]piperidin-4-yl}methanol (a compound of Example 94), N-(3-methyl-1H-indazol-6-yl)-7-methoxy-8-methylquinazoline-2-amine (a compound of Example 96), N-(4-methyl-1H-indazol-6-yl)-7-methoxy-8-methylquinazoline-2-amine (a compound of Example 97), 1-(2-hydroxyethyl)-3-[6-(8-methylquinazolin-2-ylamino)-1H-indazol-4-yl]urea (a compound of Example 98), 7-fluoro-N-(4-amino-1H-indazol-6-yl)-8-methylquinazoline-2-amine (a compound of Example 99), {1-[2-(4-amino-1H-indazol-6-ylamino)-8-methylquinazolin-7-yl]piperidin-4-yl}methanol (a compound of Example 101), N-(4-amino-1H-indazol-6-yl)-7-methoxy-8-methylquinazoline-2-amine (a compound of Example 102), 2-[2-(4-methyl-1H-indazol-6-ylamino)-8-methylquinazolin-7-ylamino]ethanol (a compound of Example 109), 7-fluoro-8-methyl-N-(4-amino-1H-benzo[d]imidazol-6-yl) quinazoline-2-amine (a compound of Example 123) and 2-[2-(4-amino-1H-benzo[d]imidazol-6-yl)-8-methylquinazolin-7-ylamino]ethanol (a compound of Example 124) exerted 50% or more inhibitory activity against Syk kinase at 0.1 μM.

Test Example 2

Action against IgE-Induced Degranulation

An inhibitory action against SYK was evaluated by an inhibitory action against degranulation of basophils induced by IgE. Rat basophilic cells RBL-2H3 were incubated with an anti-DNP-IgE antibody (1 μg/mL, Zymed) overnight. After washing cells twice with HBSS, an antigen DNP-BSA (1 μg/mL, LSL) was added, followed by incubation for 30 minutes. The supernatant was taken and an activity of β-hexosaminidase in the solution isolated by degranulation was measured using p-nitrophenyl-N-β-D-glucosaminide as a substrate. Using an absorbance meter (Spectra MAX Pro, Molecular Devices), each absorbance at 405 nm and 570 nm of the solution was measured and an increase in the amount was taken as an enzyme activity. 10 minutes before the addition of an antigen, a test material and a solvent (0.1% DMSO) were added to cells. Inhibition rate (%) against degranulation was calculated by the following equation.

$$\text{Inhibition rate (\%)} = (1-(A-C)/(B-C)) \times 100$$

where A denotes an increase in absorbance of a test material group, B denotes an increase in absorbance of a solvent group, and C denotes an increase in absorbance of a solvent group which is not stimulated by an antigen.

In this test, for example, N-(1H-indazol-6-yl)-8-methyl-7-(4-methylpiperazin-1-yl)quinazoline-2-amine (a compound of Example 63), 4-amino-N-ethyl-6-(8-methylquinazolin-2-ylamino)-1H-indazole-1-carboxamide (a compound of Example 79), ethyl [6-(8-methylquinazolin-2-ylamino)-1H-indazol-4-yl]methylcarbamate (a compound of Example 80), 8-methyl-N-(4-hydroxy-1H-indazol-6-yl)quinazoline-2-amine (a compound of Example 82) and 8-methyl-N-(2-methyl-4-amino-1H-benzo[d]imidazol-6-yl)quinazoline-2-amine (a compound of Example 88) exerted 50% or more inhibitory activity at 0.05 μM.

As is apparent from the test results described above, the compounds of the present invention have a Syk inhibitory action and a degranulation inhibitory action and therefore seem to be useful as pharmaceuticals for prevention or treatment of Syk-mediated diseases such as allergic diseases, autoimmune diseases, and arthritis.

The present invention will now be described in more detail by way of Examples and Reference Examples, but the present invention is not limited to these Examples.

The compounds were identified by hydrogen nuclear magnetic resonance spectra ($^1$H-NMR) and mass spectra (MS). The hydrogen nuclear magnetic resonance spectra were measured at 600 MHz unless otherwise specified, and an exchangeable hydrogen atom cannot sometimes be measured clearly depending on the compounds and measurement conditions. Br means a wide signal (broad).

Reference Example 1

Preparation of 2-chloro-8-methylquinazoline (Raw compound (III) of Compounds (I), (II) of the present invention)

First Step

An NMP solution (30 mL) of 2-methyl-3-aminobenzoic acid (7.56 g, 50 mmol) (a kind of the compound (V) in Scheme 2) and urea (9.00 g, 150 mmol) was heated to a temperature of 180 to 190°C and then stirred for 3.5 hours. After cooling to room temperature, water (100 mL) was added, followed by stirring for 0.5 hours. The precipitate was filtered, washed and then dried to obtain 6.44 g of the following 8-methyl-2,4-quinazolinedione (VI)-A, which is a kind of the compound (VI) in Scheme 2.

[Chemical Formula 14]

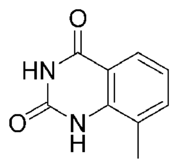

(VI)-A

$^1$H-NMR (DMSO-$d_6$) d (ppm): 2.34 (s, 3H), 7.09 (t, 1H, J = 7.8 Hz), 7.48 (d, 1H, J = 7.8 Hz), 7.77 (d, 1H, J = 7.2 Hz), 10.37 (br, 1H), 11.31 (br, 1H).

Second Step

A mixture of the product (3.11 g, 17.65 mmol) of the first step, N,N-dimethylaniline (1.8 mL) and $POCl_3$ (18 mL) was stirred at 115°C for 3.5 hours. After cooling to room temperature, the reaction mixture was added to ice water (50 mL) and the precipitated solid was collected by filtration, washed with water and then dried to obtain 3.25 g of 2,4-dichloro-8-methylquinazoline (VII)-A which is a kind of the compound (VII) in Scheme 2.

[Chemical Formula 15]

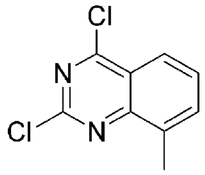

(VII)-A

$^1$H-NMR ($CDCl_3$) d (ppm): 2.75 (s, 3H), 7.61 (t, 1H, J = 7.8 Hz), 7.82 (d, 1H, J = 7.8 Hz), 8.10 (d, 1H, J = 7.8 Hz).

Third Step

To a two-phase solution of a $CH_2Cl_2$ solution (25 mL) of the product (3.25 g, 15.26 mmol) of the second step and an aqueous 9% ammonia-containing saturated brine solution (25 mL), a zinc powder (3.25 g) was added, followed by reflux for 5 hours. The reaction mixture was filtered through cerite and then the $CH_2Cl_2$ layer was distilled off under reduced pressure. To the resulting aqueous layer residue, ethyl acetate was added and the organic layer was separated. The organic layer was washed with 1N hydrochloric acid and then dried over anhydrous magnesium sulfate. The solvent was distilled off under reduced pressure and the residue was purified by silica gel chromatography (ethyl acetate:hexane = 1:5 to 2:7) to obtain 1.71 g of 2-chloro-8-methylquinazoline (III)-A, which is a kind of the compound (III) in Schemes 1 and 2, as a pale yellow powder.

[Chemical Formula 16]

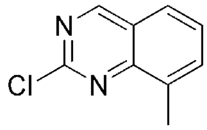

(III)-A

$^1$H-NMR ($CDCl_3$) d (ppm): 2.75 (s, 3H), 7.57 (t, 1H, J = 7.8 Hz), 7.75-7.80 (m, 2H), 9.25 (s, 1H).

Example 1

Preparation of N-(1H-indazol-6-yl)-8-methylquinazoline-2-amine

Specifically, a mixed solution of 2-chloro-8-methylquinazoline (compound (III)-A) (71 mg, 0.4 mmol) of Reference Example 1, 6-aminoindazole (67 mg, 0.5 mmol), a drop of concentrated hydrochloric acid and n-butanol (1.5 mL) was reacted for 15 minutes using a microwave synthesis apparatus (manufactured by CEM Co., 120°C, 100 W). The reaction solution was air-cooled to room temperature, and then the precipitated solid was collected by filtration and washed with cold 2-propanol. The resulting solid was purified by silica gel chromatography (ethyl acetate:n-hexane = 3:2) to obtain 22 mg of the following compound (I)-A, which is a kind of the compound (I) of the present invention, as a colorless powder.

[Chemical Formula 17]

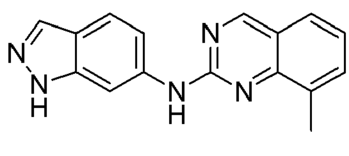

(I)-A

$^{1}H$-NMR (DMSO-$d_6$) d (ppm): 2.72 (s, 3H), 7.32 (t, 1H, J = 7.8 Hz), 7.44 (d, 1H, J = 9.0 Hz), 7.65 (d, 1H, J = 9.0 Hz), 7.73 (d, 1H, J = 7.8 Hz), 7.78 (d, 1H, J = 7.8 Hz), 7.93 (s, 1H), 8.75 (s, 1H), 9.30 (s, 1H), 10.06 (s, 1H), 12.92 (s, 1H); MALDI TOF-MS (m/z): 276 $[M+H]^+$, 298 $[M+Na]^+$.

Example 2

Preparation of N-(1H-benzo[d][1,2,3]triazol-5-yl)-8-methylquinazoline-2-amine

In accordance with Scheme 1 described above, the compound (I) of the present invention was prepared.

Specifically, a mixed solution of 2-chloro-8-methylquinazoline (compound (III)-A) (71 mg, 0.4 mmol) of Reference Example 1, 5-aminobenztriazole (67 mg, 0.5 mmol), a drop of concentrated hydrochloric acid and n-butanol (1.5 mL) was reacted for 15 minutes using a microwave synthesis apparatus (manufactured by CEM Co., 120°C, 100 W). The reaction mixture was diluted with ethyl acetate and washed with water, and then the organic layer was dried over anhydrous magnesium sulfate. The solvent was distilled off under reduced pressure, and then the residue was purified by silica gel chromatography (ethyl acetate:n-hexane = 2:1) to obtain 15 mg of the following compound (I)-B, which is a kind of the compound (I) of the present invention, as a colorless powder.

[Chemical Formula 18]

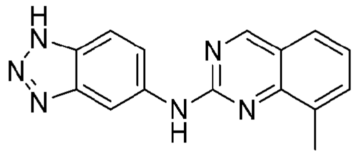

(I)-B

$^{1}H$-NMR (DMSO-$d_6$) d (ppm): 2.73 (s, 3H), 7.34 (dd, 1H, J = 7.8, 7.2 Hz), 7.6-7.7 (m, 1H), 7.75 (d, 1H, J = 7.2 Hz), 7.80 (d, 1H, J = 7.8 Hz), 7.8-7.95 (m, 1H), 9.00 (s, 1H), 9.33 (s, 1H), 10.25 (s, 1H); MALDI TOF-MS (m/z): 277 $[M+H]^+$.

Example 3

Preparation of N-(1H-benzo[d]imidazol-6-yl)-8-methylquinazoline-2-amine

In accordance with Scheme 1 described above, the compound (I) of the present invention was prepared.

Specifically, the crude product obtained by reacting and treating in the same manner as in Example 2, except for using 2-chloro-8-methylquinazoline (compound (III)-A) (71 mg, 0.4 mmol) of Reference Example 1 and 5-aminobenzimidazole (67 mg, 0.5 mmol), was purified by preparative thin-layer chromatography (chloroform:methanol = 10:1) to obtain 20 mg of the following compound (I)-C which is a kind of the compound (I) of the present invention.

[Chemical Formula 19]

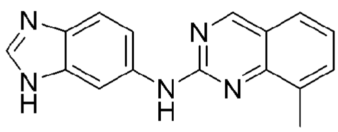

(I)-C

$^{1}H$-NMR (DMSO-$d_6$) d (ppm): 2.68 (s, 3H), 7.27 (dd, 1H, J = 7.8, 7.2 Hz), 7.53 (d, 1H, J = 8.4 Hz), 7.57 (d, 1H, J = 8.4 Hz),7.69 (d, 1H, J = 7.2 Hz), 7.74 (d, 1H, J = 7.8 Hz), 8.14 (s, 1H), 8.65 (s, 1H), 9.26 (s, 1H), 9.88 (s, 1H), 12.48 (br, 1H); MALDI TOF-MS (m/z): 276 $[M+H]^+$.

Example 4

Preparation of N-(2,3-dihydro-1H-inden-5-yl)-8-methylquinazoline-2-amine

In accordance with Scheme 1 described above, the compound (I) of the present invention was prepared.

Specifically, 60 mg of the following compound (I)-D, which is a kind of the compound (I) of the present invention, was obtained by reacting and treating in the same manner as in Example 2, except for using 2-chloro-8-methylquinazoline (compound (III)-A) (71 mg, 0.4 mmol) of Reference Example 1 and 5-aminoindan (67 mg, 0.5 mmol).

[Chemical Formula 20]

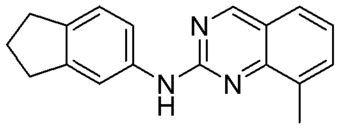

(I)-D

$^1$H-NMR (DMSO-$d_6$) d (ppm): 2.0-2.1 (m, 2H), 2.60 (s, 3H), 2.83 (t, 2H, J = 7.2 Hz), 2.88 (t, 2H, J = 7.2 Hz), 7.17 (d, 1H, J = 7.8 Hz), 7.26 (dd, 1H, J = 7.8, 7.2 Hz), 7.67 (d, 1H, J = 7.2 Hz), 7.73 (d, 1H, J = 7.8 Hz), 7.75 (d, 1H, J = 7.8 Hz), 7.99 (s, 1H), 9.23 (s, 1H), 9.74(s, 1H); MALDI TOF-MS (m/z): 276 $[M+H]^+$.

Example 5

Preparation of N-(1H-indol-6-yl)-8-methylquinazoline-2-amine

In accordance with Scheme 1 described above, the compound (I) of the present invention was prepared.

Specifically, the crude product obtained by reacting and treating in the same manner as in Example 2, except for using 2-chloro-8-methylquinazoline (compound (III)-A) (71 mg, 0.4 mmol) of Reference Example 1 and 6-aminoindole (66 mg, 0.5 mmol), was purified by preparative thin-layer chromatography (chloroform:methanol = 10:1) to obtain 10 mg of the following compound (I)-E, which is a kind of the compound (I) of the present invention.

[Chemical Formula 21]

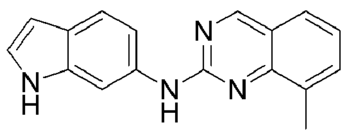

(I)-E

$^1$H-NMR (DMSO-$d_6$) d (ppm): 2.68 (s, 3H), 6.35 (s, 1H), 7.22 (s, 1H), 7.25 (dd, 1H, J = 7.8, 7.2 Hz), 7.40 (d, 1H, J = 8.4 Hz), 7.44 (d, 1H, J = 8.4 Hz), 7.67 (d, 1H, J = 6.6 Hz), 7.72 (d, 1H, J = 7.8 Hz), 8.50 (s, 1H), 9.23 (s, 1H), 9.74 (s, 1H), 11.02 (s, 1H); MALDI TOF-MS (m/z): 275 $[M+H]^+$.

Example 6

Preparation of N-(benzo[d][1,3]dioxol-5-yl)-8-methylquinazoline-2-amine

In accordance with Scheme 1 described above, the compound (I) of the present invention was prepared.

Specifically, 26 mg of the following compound (I)-F, which is a kind of the compound (I) of the present invention,

was obtained by reacting and treating in the same manner as in Example 2, except for using 2-chloro-8-methylquinazoline (compound (III)-A) (71 mg, 0.4 mmol) of Reference Example 1 and 3,4-methylenedioxyaniline (69 mg, 0.5 mmol).

[Chemical Formula 22]

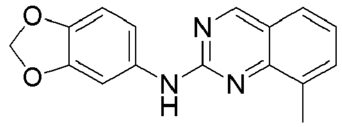

(I)-F

$^1$H-NMR (DMSO-$d_6$) d (ppm) : 2.59 (s, 3H), 5.98 (s, 2H), 6.89 (d, 1H, J = 8.4 Hz), 7.26 (dd, 1H, J = 7.8, 7.2 Hz), 7.38 (dd, 1H, J = 8.4, 1.8 Hz), 7.67 (d, 1H, J = 7.2 Hz), 7.73 (d, 1H, J = 7.8 Hz), 7.90 (s, 1H), 9.23 (s, 1H), 9.79 (s, 1H); MALDI TOF-MS (m/z): 280 $[M+H]^+$.

Example 7

Preparation of N-(2,3-dihydrobenzo[b][1,4]dioxin-6-yl)-8-methylquinazoline-2-amine

In accordance with Scheme 1 described above, the compound (I) of the present invention was prepared.

Specifically, a mixed solution of 2-chloro-8-methylquinazoline (compound (III)-A) (71 mg, 0.4 mmol) of Reference Example 1, 3,4-ethylenedioxyaniline (91 mg, 0.5 mmol), a drop of concentrated hydrochloric acid and n-butanol (1.5 mL) was reacted for 15 minutes using a microwave synthesis apparatus (manufactured by CEM Co., 120°C, 100 W). The reaction solution was air-cooled to room temperature and then the precipitated solid was collected by filtration and washed with cold 2-propanol to obtain 119 mg of the following compound (I)-G, which is a kind of the compound (I) of the present invention.

[Chemical Formula 23]

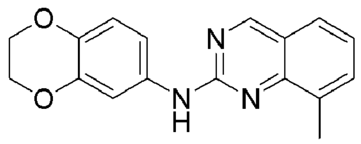

(I)-G

$^1$H-NMR (DMSO-$d_6$) d (ppm) : 2.59 (s, 3H), 4.21 (d, 2H, J = 2.4 Hz), 4.25 (d, 2H, J = 2.4Hz), 6.81 (d, 1H, J = 9.0 Hz), 7.26 (dd, 1H, J = 7.8, 7.2 Hz), 7.32 (dd, 1H, J = 9.0, 2.4 Hz), 7.67 (d, 1H, J = 7.2 Hz), 7.73 (d, 1H, J = 7.8 Hz), 7.86 (s, 1H), 9.23 (s, 1H), 9.75 (s, 1H); MALDI TOF-MS (m/z): 294 $[M+H]^+$.

Example 8

Preparation of 6-(8-methylquinazolin-2-ylamino)-2H-benzo[b][1,4]oxazin-3(4H)-one

In accordance with Scheme 1 described above, the compound (I) of the present invention was prepared.

Specifically, 61.0 mg of the following compound (I)-H, which is a kind of the compound (I) of the present invention, was obtained as a colorless powder by reacting and treating in the same manner as in Example 1, except for using 2-chloro-8-methylquinazoline (compound (III)-A) (71 mg, 0.4 mmol) of Reference Example 1 and 6-amino-2H-benzo[b][1,4]oxazin-3(4H)-one (82 mg, 0.5 mmol).

[Chemical Formula 24]

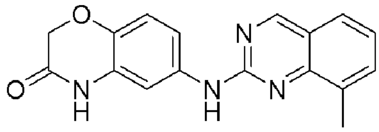

(I)-H

$^1$H-NMR (DMSO-$d_6$) d (ppm): 2.62 (s, 3H), 4.52 (s, 2H), 6.92 (d, 1H, J = 8.4 Hz), 7.26 (dd, 1H, J = 7.8, 7.2 Hz), 7.45 (d, 1H, J = 8.4 Hz), 7.67 (d, 1H, J = 7.2 Hz), 7.7-7.8 (m, 2H), 9.23 (s, 1H), 9.79 (s, 1H), 10.83 (s, 1H); MALDI TOF-MS (m/z): 307 $[M+H]^+$.

Example 9

Preparation of 6-(8-methylquinazolin-2-ylamino)-2-methyl-2H-benzo[b][1,4]oxazin-3(4H)-one

In accordance with Scheme 1 described above, the compound (I) of the present invention was prepared.

Specifically, 115 mg of the following compound (I)-I, which is a kind of the compound (I) of the present invention, was obtained by reacting and treating in the same manner as in Example 7, except for using 2-chloro-8-methylquinazoline (compound (III)-A) (71 mg, 0.4 mmol) of Reference Example 1 and 6-amino-2-methyl-2H-benzo[b][1,4]oxazin-3(4H)-one (71 mg, 0.4 mol).

[Chemical Formula 25]

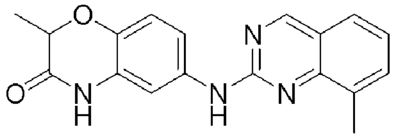

(I)-I

$^1$H-NMR (DMSO-$d_6$) d (ppm): 1.42 (d, 3H, J = 6.6 Hz), 2.60 (s, 3H), 4.65 (q, 1H, J = 6.6 Hz),6.85 (d, 1H, J = 9.0 Hz), 7.28 (dd, 1H, J = 7.8, 7.2 Hz), 7.60 (d, 1H, J = 8.4 Hz), 7.68 (d, 1H, J = 7.2 Hz), 7.74 (d, 1H, J = 7.8 Hz), 7.80 (s, 1H), 9.25 (s, 1H), 9.85 (s, 1H), 10.53 (s, 1H); MALDI TOF-MS (m/z): 321 $[M+H]^+$.

Example 10

Preparation of N-(benzo[d]thiazol-6-yl)-8-methylquinazoline-2-amine

In accordance with Scheme 1 described above, the compound (I) of the present invention was prepared.

Specifically, 36 mg of the following compound (I)-J, which is a kind of the compound (I) of the present invention, was obtained by reacting and treating in the same manner as in Example 2, except for using 2-chloro-8-methylquinazoline (compound (III)-A) (71 mg, 0.4 mmol) of Reference Example 1 and 6-aminobenzothiazole (75 mg, 0.5 mmol).

[Chemical Formula 26]

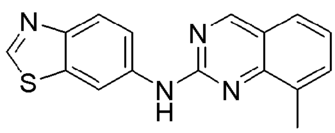

(I)-J

$^1$H-NMR (DMSO-$d_6$) d (ppm): 2.67 (s, 3H), 7.32 (dd, 1H, J = 7.8, 7.2 Hz), 7.73 (d, 1H, J = 7.2 Hz), 7.79 (d, 1H, J = 7.8 Hz), 7.96 (d, 1H, J = 9.0 Hz), 8.03 (d, 1H, J = 9.0 Hz), 9.11 (s, 1H), 9.22 (s, 1H), 9.32 (s, 1H), 10.22 (s, 1H); MALDI TOF-MS (m/z) : 293 $[M+H]^+$.

Example 11

Preparation of N-(3-(8-methylquinazolin-2-ylamino)phenyl)acetamide

In accordance with Scheme 1 described above, the compound (II) of the present invention was prepared.

Specifically, a mixed solution of 2-chloro-8-methylquinazoline (compound (III)-A) (89 mg, 0.5 mmol) of Reference Example 1, 3-aminoacetoanilide (90 mg, 0.6 mmol), a drop of concentrated hydrochloric acid and n-butanol (2.0 mL) was reacted for 15 minutes using a microwave synthesis apparatus (manufactured by CEM Co., 120°C, 100 W). The reaction mixture was concentrated under reduced pressure and then the resulting residue was purified by silica gel chromatography (ethyl acetate:n-hexane = 2:1) to obtain 106 mg of the following compound (II)-A, which is a kind of the compound (II) of the present invention.

[Chemical Formula 27]

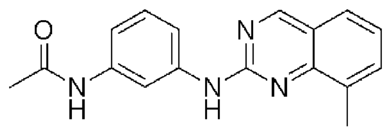

(II)-A

$^1$H-NMR (DMSO-$d_6$) d (ppm): 2.05 (s, 3H), 2.62 (s, 3H), 7.14 (d, 1H, J = 7.8 Hz), 7.23 (dd, 1H, J = 8.4, 7.8 Hz), 7.28 (dd, 1H, J = 7.8, 7.2 Hz), 7.67 (d, 1H, J = 7.2 Hz), 7.74 (d, 1H, J = 7.8 Hz), 7.77 (d, 1H, J = 8.4 Hz), 8.21 (s, 1H), 9.26 (s, 1H), 9.83 (s, 2H); MALDI TOF-MS (m/z): 293 $[M+H]^+$.

Example 12

Preparation of N1-(8-methylquinazolin-2-yl)benzene-1,3-diamine hydrochloride

The compound (100 mg, 0.34 mmol) of Example 11 was dissolved in ethanol (5.0 mL) and concentrated hydrochloric acid (4.0 mL) was added, followed by heating to 100°C and further stirring for 3.5 hours. The reaction solution was cooled to room temperature and then the precipitated solid was collected by filtration to obtain 12 mg of the following compound (II)-B, which is a kind of the compound (II) of the present invention.

[Chemical Formula 28]

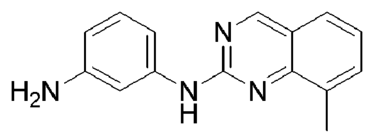

(II)-B

$^1$H-NMR (DMSO-$d_6$) d (ppm): 2.67 (s, 3H), 7.08 (d, 1H, J = 7.8 Hz), 7.34 (t, 1H, J = 7.8 Hz), 7.45 (dd, 1H, J = 7.8, 6.6 Hz), 7.73 (d, 1H, J = 6.6 Hz), 7.79 (d, 1H, J = 7.8 Hz), 7.99 (d, 1H, J = 7.8 Hz), 8.23 (s, 1H), 9.33 (s, 1H), 10.1-10.7 (m, 3H); MALDI TOF-MS (m/z): 251 $[M+H]^+$.

Example 13

Preparation of 3-(8-methylquinazolin-2-yl-amino)phenol

In accordance with Scheme 1 described above, the compound (II) of the present invention was prepared.

Specifically, a mixed solution of 2-chloro-8-methylquinazoline (compound (III)-A) (47 mg, 0.263 mmol) of Reference Example 1, 3-aminophenol (29 mg, 0.263 mmol), a drop of concentrated hydrochloric acid and n-butanol (2.0 mL) was reacted for 25 minutes using a microwave synthesis apparatus (manufactured by CEM Co., 135°C, 100 W). The precipitate was removed by filtration and the filtrate was concentrated under reduced pressure, and then the resulting residue was purified by silica gel chromatography (chloroform:methanol = 10:1) to obtain 8 mg of the following compound (II)-C, which is a kind of the compound (II) of the present invention.

[Chemical Formula 29]

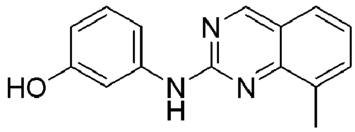

(II)-C

$^1$H-NMR (DMSO-$d_6$) d (ppm): 2.48 (s, 3H), 6.39 (dd, 1H, J = 8.4, 1.8 Hz), 7.07 (dd, 1H, J = 8.4, 7.8 Hz), 7.26 (dd, 1H, J = 7.8, 7.2 Hz), 7.45 (d, 1H, J = 7.8 Hz), 7.53 (d, 1H, J = 1.8 Hz), 7.66 (d, 1H, J = 7.2 Hz), 7.72 (d, 1H, J = 7.8 Hz), 9.21 (s, 1H), 9.23 (s, 1H), 9.71(s, 1H); MALDI TOF-MS (m/z): 252 $[M+H]^+$.

Example 14

Preparation of N-(2-methoxyphenyl)-8-methylquinazoline-2-amine

In accordance with Scheme 1 described above, the compound (II) of the present invention was prepared.

Specifically, a mixed solution of 2-chloro-8-methylquinazoline (compound (III)-A) (89 mg, 0.5 mmol) of Reference Example 1, 2-aminoanisole (76 mg, 0.6 mmol), a drop of concentrated hydrochloric acid and n-butanol (2.0 mL) was reacted for 15 minutes using a microwave synthesis apparatus (manufactured by CEM Co., 120°C, 100 W). The reaction mixture was diluted with ethyl acetate and washed with water and saturated brine, and then the organic layer was dried over anhydrous magnesium sulfate. The solvent was distilled off under reduced pressure and then the residue was purified by silica gel chromatography (ethyl acetate:n-hexane = 3:7) to obtain 43 mg of the following compound (II)-D, which is a kind of the compound (II) of the present invention.

[Chemical Formula 30]

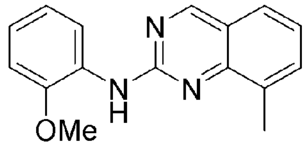

(II)-D

$^1$H-NMR (DMSO-$d_6$) d (ppm): 2.62 (s, 3H), 3.92 (s, 3H), 6.95-7.05 (m, 2H), 7.05-7.15 (m, 1H), 7.31 (dd, 1H, J = 7.9, 7.0 Hz), 7.71 (d, 1H, J = 7.0 Hz), 7.77 (d, 1H, J = 7.9 Hz), 8.18 (s, 1H), 8.65-8.75 (m, 1H), 9.28 (s, 1H); MALDI TOF-MS (m/z): 266 $[M+H]^+$.

Example 15

Preparation of 2-(8-methylquinazolin-2-ylamino)phenol

To a $CH_2Cl_2$ solution (15 mL) of the compound (35 mg, 0.13 mmol) of Example 14, a 1M $CH_2Cl_2$ solution (0.9 mL) of borane tribromide was added dropwise under ice cooling, followed by stirring at room temperature for 3 hours. The reaction solution was diluted with $CH_2Cl_2$ (30 mL) and an aqueous sodium thiosulfate solution was added while ice cooling. The organic layer was separated and dried over anhydrous magnesium sulfate, and then the solvent was distilled off under reduced pressure. The residue was purified by silica gel chromatography (chloroform:methanol = 80:1) to obtain 17 mg of the following compound (II)-E, which is a kind of the compound (II) of the present invention.

[Chemical Formula 31]

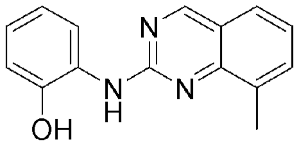

(II)-E

$^1$H-NMR (DMSO-$d_6$) d (ppm): 2.58 (s, 3H), 6.8-7.0 (m, 3H), 7.31 (dd, 1H, J = 7.9, 7.1 Hz), 7.7 (d, 1H, J = 7.1 Hz), 7.77 (d, 1H, J = 7.9 Hz), 8.36 (d, 1H, J = 7.7 Hz), 8.50 (s, 1H), 9.28 (s, 1H), 10.29 (s, 1H); MALDI TOF-MS (m/z): 252 $[M+H]^+$.

Example 16

Preparation of N-(4-methoxyphenyl)-8-methylquinazoline-2-amine

In accordance with Scheme 1 described above, the compound (II) of the present invention was prepared.

Specifically, 74 mg of the following compound (II)-F, which is a kind of the compound (II) of the present invention, was obtained by reacting and treating in the same manner as in Example 7, except for using 2-chloro-8-methylquinazoline (compound (III)-A) (89 mg, 0.5 mmol) of Reference Example 1 and 4-aminoanisole (76 mg, 0.6 mmol).

[Chemical Formula 32]

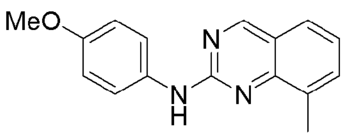

(II)-F

$^1$H-NMR (DMSO-$d_6$) d (ppm) : 2.60 (s, 3H), 3.75 (s, 3H), 6.94 (d, 2H, J = 9.0 Hz), 7.26 (dd, 1H, J = 8.4, 7.2 Hz), 7.67 (d, 1H, J = 7.2 Hz), 7.73 (d, 1H, J = 8.4 Hz), 7.94 (d, 2H, J = 9.0 Hz), 9.23 (s, 1H), 9.76 (s, 1H); MALDI TOF-MS (m/z): 266 $[M+H]^+$.

Example 17

Preparation of 2-(8-methylquinazolin-2-ylamino)phenol

Specifically, 11 mg of the following compound (II)-G, which is a kind of the compound (II) of the present invention, was obtained by reacting and treating in the same manner as in Example 15, except for using the compound (53 mg, 0.2 mmol) of Example 16.

[Chemical Formula 33]

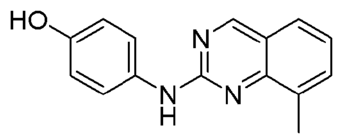

(II)-G

$^1$H-NMR (DMSO-$d_6$) d (ppm): 2.57 (s, 3H), 6.74 (d, 2H, J = 8.7 Hz), 7.22 (dd, 1H, J = 8.6, 7.9 Hz), 7.64 (d, 1H, J = 8.6 Hz), 7.69 (d, 1H, J = 7.9 Hz), 7.80 (d, 2H, J = 8.9 Hz), 9.03 (s, 1H), 9.19 (s, 1H), 9.57 (s, 1H); MALDI TOF-MS (m/z): 252 $[M+H]^+$.

Example 18

Preparation of N-(3-(trifluoromethyl)phenyl)-8-methylquinazoline-2-amine

In accordance with Scheme 1 described above, the compound (II) of the present invention was prepared.
Specifically, 20 mg of the following compound (II)-H, which is a kind of the compound (II) of the present invention, was obtained by reacting and treating in the same manner as in Example 7, except for using 2-chloro-8-methylquinazoline (compound (III)-A) (89 mg, 0.5 mmol) of Reference Example 1 and 3-trifluoromethylaniline (97 mg, 0.6 mmol).

[Chemical Formula 34]

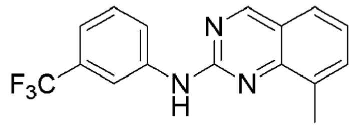

(II)-H

$^1$H-NMR (DMSO-$d_6$) d (ppm): 2.63 (s, 3H), 7.31 (d, 1H, J = 7.8 Hz), 7.35 (dd, 1H, J = 7.8, 7.2 Hz), 7.56 (dd, 1H, J = 8.4, 7.2 Hz), 7.74 (d, 1H, J = 7.2 Hz), 7.80 (d, 1H, J = 8.4 Hz), 8.01 (d, 1H, J = 7.2 Hz), 8.94 (s, 1H), 9.34 (s, 1H), 10.29 (s, 1H); MALDI TOF-MS (m/z): 304 $[M+H]^+$.

Example 19

Preparation of N-(3-bromophenyl)-8-methylquinazoline-2-amine

In accordance with Scheme 1 described above, the compound (II) of the present invention was prepared.
Specifically, 127 mg of the following compound (II)-I, which is a kind of the compound (II) of the present invention, was obtained by reacting and treating in the same manner as in Example 7, except for using 2-chloro-8-methylquinazoline (compound (III)-A) (89 mg, 0.5 mmol) of Reference Example 1 and 3-bromoaniline (102 mg, 0.6 mmol).

[Chemical Formula 35]

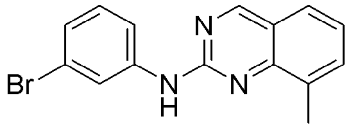

(II)-I

$^1$H-NMR (DMSO-$d_6$) d (ppm): 2.64 (s, 3H), 7.15 (d, 1H, J = 8.4 Hz), 7.29 (dd, 1H, J = 8.4, 7.8 Hz), 7.34 (dd, 1H, J = 7.8, 7.2 Hz), 7.73 (d, 1H, J = 7.2 Hz), 7.79 (d, 1H, J = 8.4 Hz), 7.86 (d, 1H, J = 7.8 Hz), 8.63 (s, 1H), 9.32 (s, 1H), 10.12 (s, 1H); MALDI TOF-MS (m/z): 315 $[M+H]^+$.

Example 20

Preparation of N-(3-chlorophenyl)-8-methylquinazoline-2-amine

In accordance with Scheme 1 described above, the compound (II) of the present invention was prepared.

Specifically, 34 mg of the following compound (II)-J, which is a kind of the compound (II) of the present invention, was obtained by reacting and treating in the same manner as in Example 7, except for using 2-chloro-8-methylquinazoline (71 mg, 0.4 mmol) (compound (III)-A) and 3-chloroaniline (64 mg, 0.5 mmol).

[Chemical Formula 36]

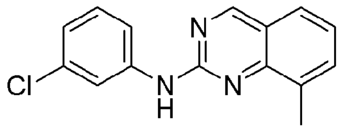

(II)-J

$^1$H-NMR (DMSO-$d_6$) d (ppm) : 2.64 (s, 3H), 7.02 (d, 1H, J = 8.4 Hz), 7.35 (dd, 2H, J = 8.4, 7.8 Hz), 7.73 (d, 1H, J = 7.8 Hz), 7.79 (d, 1H, J = 8.4 Hz), 7.84 (d, 1H, J = 7.8 Hz), 8.45 (s, 1H), 9.32 (s, 1H), 10.13 (s, 1H); MALDI TOF-MS (m/z): 270 $[M+H]^+$.

Example 21

Preparation of N-(3-fluorophenyl)-8-methylquinazoline-2-amine

In accordance with Scheme 1 described above, the compound (II) of the present invention was prepared.

Specifically, 26 mg of the following compound (II)-K, which is a kind of the compound (II) of the present invention, was obtained by reacting and treating in the same manner as in Example 7, except for using 2-chloro-8-methylquinazoline (compound (III)-A) (71 mg, 0.4 mmol) of Reference Example 1 and 3-fluoroaniline (56 mg, 0.5 mmol).

[Chemical Formula 37]

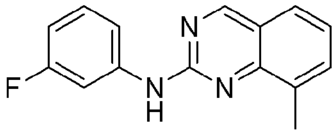

(II)-K

$^1$H-NMR (DMSO-$d_6$) d (ppm): 2.64 (s, 3H), 6.78 (dd, 1H, J = 8.4, 6.6 Hz), 7.3-7.4 (m, 2H), 7.68 (d, 1H, J = 8.4 Hz), 7.73 (d, 1H, J = 7.2 Hz), 7.79 (d, 1H, J = 7.8 Hz), 8.21 (d, 1H, J = 12.6 Hz), 9.31 (s, 1H), 10.14 (s, 1H); MALDI TOF-MS (m/z): 254 $[M+H]^+$.

Example 22

Preparation of 3-(8-methylquinazolin-2-ylamino)benzoic acid

First Step

Methyl 3-(8-methylquinazolin-2-ylamino)benzoate

In accordance with Scheme 1 described above, the compound (II) of the present invention was prepared.

Specifically, 111 mg of methyl 3-(8-methylquinazolin-2-ylamino)benzoate was obtained by reacting and treating in the same manner as in Example 7, except for using 2-chloro-8-methylquinazoline (compound (III)-A) (89 mg, 0.5 mmol) of Reference Example 1 and methyl 3-aminobenzoate (91 mg, 0.6 mol).

[Chemical Formula 38]

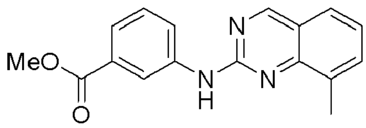

$^1$H-NMR (DMSO-$d_6$) d (ppm): 2.66 (s, 3H), 3.88 (s, 3H), 7.33 (dd, 1H, J = 7.8, 7.2 Hz), 7.47 (dd, 1H, J = 7.8, 7.2), 7.58 (d, 1H, J = 7.2 Hz), 7.72 (d, 1H, J = 7.2 Hz), 7.78 (d, 1H, J = 7.8 Hz), 8.06 (s, 1H), 9.15 (s, 1H), 9.31 (s, 1H), 10.15 (s, 1H).

Second Step

To a THF/methanol 1:1 mixed solution (4 mL) of the product (63 mg, 0.21 mmol) of the first step, an aqueous 1N sodium hydroxide solution (1 mL) was added, and then the mixture was reacted for 30 minutes using a microwave synthesis apparatus (manufactured by CEM Co., 50°C, 10 W). The reaction solution was diluted with water (15 mL) while ice cooling and then acidified by adding concentrated hydrochloric acid. The precipitated solid was collected by filtration and then washed twice with water (15 mL) to obtain 59 mg of 3-(8-methylquinazolin-2-ylamino)benzoic acid as the compound (II)-L, which is a kind of the compound (II) of the present invention.

[Chemical Formula 39]

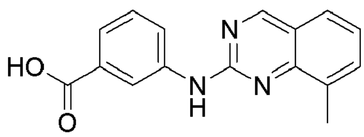

(II)-L

$^1$H-NMR (DMSO-$d_6$) d (ppm): 2.66 (s, 3H), 7.32 (d, 1H, J = 8.4, 7.8 Hz), 7.45 (dd, 1H, J = 7.8, 7.2 Hz), 7.57 (d, 1H, J = 7.2 Hz), 7.71 (d, 1H, J = 8.4 Hz), 7.78 (d, 1H, J = 8.4 Hz), 8.15 (d, 1H, J = 7.8 Hz), 8.94 (s, 1H), 9.30 (s, 1H), 10.09 (s, 1H), 12.80 (br, 1H); MALDI TOF-MS (m/z): 280 $[M+H]^+$, 302 $[M+Na]^+$.

Example 23

Preparation of 3-(8-methylquinazolin-2-ylamino)benzoic acid amide

The compound (59 mg, 0.21 mmol) of Example 22 was dissolved in an N,N-dimethylformamide solution (6 mL) and then WSC (61 mg, 0.32 mmol) and HOBt (49 mg, 0.32 mmol) were added under ice cooling. After about 2 minutes, an aqueous 25% ammonia solution (1 mL) was added, followed by stirring at room temperature overnight. After the reaction solution was diluted with ethyl acetate and washed in turn with water and saturated brine, the organic layer was dried over anhydrous magnesium sulfate and the solvent was distilled off under reduced pressure. The resulting residue

was purified by silica gel column chromatography (chloroform:methanol = 30:1) to obtain 4.0 mg of the following compound (II)-M, which is a kind of the compound (II) of the present invention.

[Chemical Formula 40]

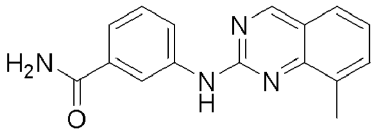

(II)-M

$^1$H-NMR (DMSO-$d_6$) d (ppm): 2.65 (s, 3H), 7.2-7.35 (m, 2H), 7.35-7.5 (m, 2H), 7.65-7.72 (m, 1H), 7.72-7.8 (m, 1H), 7.8-7.9 (m, 1H), 8.05-8.15 (m, 1H), 8.67 (s, 1H), 9.30 (s, 1H), 9.99 (s, 1H); MALDI TOF-MS (m/z): 279 $[M+H]^+$.

Example 24

Preparation of 3-(8-methylquinazolin-2-ylamino)benzonitrile

In accordance with Scheme 1 described above, the compound (II) of the present invention was prepared.
Specifically, 71 mg of the following compound (II)-N, which is a kind of the compound (II) of the present invention, was obtained by reacting and treating in the same manner as in Example 7, except for using 2-chloro-8-methylquinazoline (compound (III)-A) (71 mg, 0.5 mmol) of Reference Example 1 and 3-aminobenzonitrile (59 mg, 0.6 mmol).

[Chemical Formula 41]

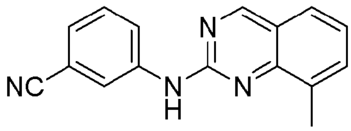

(II)-N

$^1$H-NMR (DMSO-$d_6$) d (ppm): 2.65 (s, 3H), 7.36 (dd, 1H, J = 7.8, 7.2 Hz), 7.42 (d, 1H, J = 8.4 Hz), 7.55 (dd, 1H, J = 8.4, 7.8 Hz), 7.75 (d, 1H, J = 7.2 Hz), 7.81 (d, 1H, J = 7.8 Hz), 8.19 (d, 1H, J = 7.8 Hz), 8.71 (s, 1H), 9.35 (s, 1H), 10.30 (s, 1H); MALDI TOF-MS (m/z): 261 $[M+H]^+$.

Example 25

Preparation of N-(4-(8-methylquinazolin-2-ylamino)phenyl)acetamide

In accordance with Scheme 1 described above, the compound (II) of the present invention was prepared.
Specifically, 99 mg of the following compound (II)-O, which is a kind of the compound (II) of the present invention, was obtained by reacting and treating in the same manner as in Example 7, except for using 2-chloro-8-methylquinazoline (compound (III)-A) (71 mg, 0.4 mmol) of Reference Example 1 and 4-aminoacetoanilide (75 mg, 0.5 mmol).

[Chemical Formula 42]

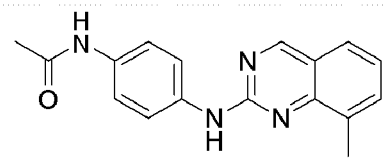

(II)-O

$^1$H-NMR (DMSO-$d_6$) d (ppm): 2.03 (s, 3H), 2.61 (s, 3H), 7.27 (dd, 1H, J = 8.4, 7.2 Hz), 7.53 (d, 2H, J = 7.8 Hz), 7.68 (d, 1H, J = 7.2 Hz), 7.73 (d, 1H, J = 8.4 Hz), 7.94 (d, 2H, J = 7.8 Hz), 9.25 (s, 1H), 9.84 (s, 2H); MALDI TOF-MS (m/z): 293 $[M+H]^+$.

Example 26

Preparation of 5-(8-methylquinazolin-2-ylamino)-2-methoxyphenol

In accordance with Scheme 1 described above, the compound (II) of the present invention was prepared.
Specifically, 38 mg of the following compound (II)-P, which is a kind of the compound (II) of the present invention, was obtained by reacting and treating in the same manner as in Example 2, except for using 2-chloro-8-methylquinazoline (compound (III)-A) (89 mg, 0.5 mmol) of Reference Example 1 and 5-amino-2-methoxyphenol (83 mg, 0.6 mmol).

[Chemical Formula 43]

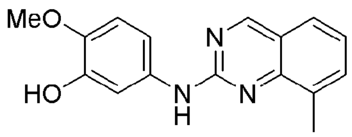

(II)-P

$^1$H-NMR (DMSO-$d_6$) d (ppm) : 2.60 (s, 3H), 3.75 (s, 3H), 6.89 (d, 1H, J = 9.0 Hz), 7.24 (dd, 1H, J = 8.4, 7.2 Hz), 7.4-7.55 (m, 2H), 7.65 (d, 1H, J = 7.2 Hz), 7.71 (d, 1H, J = 7.8 Hz), 8.82 (s, 1H), 9.20 (s, 1H), 9.58 (s, 1H); MALDI TOF-MS (m/z): 282 $[M+H]^+$.

Example 27

Preparation of N-(4-chloro-3-methoxyphenyl)-8-methylquinazoline-2-amine

In accordance with Scheme 1 described above, the compound (II) of the present invention was prepared.
Specifically, 127 mg of the following compound (II)-Q, which is a kind of the compound (II) of the present invention, was obtained by reacting and treating in the same manner as in Example 7, except for using 2-chloro-8-methylquinazoline (compound (III)-A) (89 mg, 0.5 mmol) of Reference Example 1 and 4-chloro-3-methoxyaniline (95 mg, 0.6 mmol).

[Chemical Formula 44]

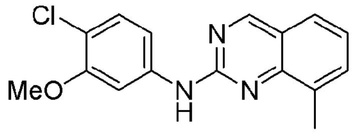

(II)-Q

$^1$H-NMR (DMSO-$d_6$) d (ppm): 2.65 (s, 3H), 3.94 (s, 3H), 7.25-7.4 (m, 3H), 7.71 (d, 1H, J = 6.6 Hz), 7.78 (d, 1H, J = 7.8 Hz), 8.36 (s, 1H), 9.30 (s, 1H), 10.06 (s, 1H); MALDI TOF-MS (m/z): 300 $[M+H]^+$.

Example 28

Preparation of 5-(8-methylquinazolin-2-ylamino)-2-chlorophenol

Specifically, 15 mg of the following compound (II)-R, which is a kind of the compound (II) of the present invention, was obtained by reacting and treating in the same manner as in Example 15, except for using the compound (60 mg, 0.2 mmol) of Example 27.

[Chemical Formula 45]

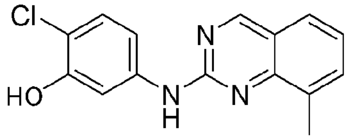

(II)-R

$^1$H-NMR (DMSO-$d_6$) d (ppm): 2.63 (s, 3H), 7.24 (d, 1H, J = 8.4 Hz), 7.30 (dd, 1H, J = 8.4, 6.6 Hz), 7.51 (d, 1H, J = 8.4 Hz), 7.69 (d, 1H, J = 6.6 Hz), 7.71 (s, 1H), 7.75 (d, 1H, J = 8.4 Hz), 9.27 (s, 1H), 9.88 (s, 1H), 9.98 (s, 1H); MALDI TOF-MS (m/z): 286 $[M+H]^+$.

Example 29

Preparation of 5-(8-methylquinazolin-2-ylamino)-2-methylphenol

In accordance with Scheme 1 described above, the compound (II) of the present invention was prepared.

Specifically, 30 mg of the following compound (II)-S, which is a kind of the compound (II) of the present invention, was obtained by reacting and treating in the same manner as in Example 2, except for using 2-chloro-8-methylquinazoline (compound (III)-A) (71 mg, 0.4 mmol) of Reference Example 1 and 5-amino-2-methylphenol (62 mg, 0.5 mmol).

[Chemical Formula 46]

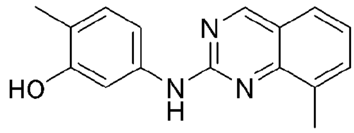

(II)-S

$^1$H-NMR (DMSO-$d_6$) d (ppm): 2.09 (s, 3H), 2.61 (s, 3H), 6.98 (d, 1H, J = 8.4 Hz), 7.25 (dd, 1H, J = 7.8, 7.2 Hz), 7.35-7.5 (m, 2H), 7.66 (d, 1H, J = 7.2 Hz), 7.71 (d, 1H, J = 7.8 Hz); 9.11 (s, 1H), 9.22 (s, 1H), 9.63 (s, 1H); MALDI TOF-MS (m/z): 266 $[M+H]^+$.

Example 30

Preparation of N-(3,4-dimethylphenyl)-8-methylquinazoline-2-amine

In accordance with Scheme 1 described above, the compound (II) of the present invention was prepared.

Specifically, 80 mg of the following compound (II)-T, which is a kind of the compound (II) of the present invention, was obtained by reacting and treating in the same manner as in Example 7, except for using 2-chloro-8-methylquinazoline (compound (III)-A) (89 mg, 0.5 mmol) of Reference Example 1 and 3,4-dimethoxyaniline (92 mg, 0.6 mmol).

[Chemical Formula 47]

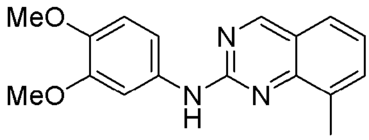

(II)-T

$^1$H-NMR (DMSO-$d_6$) d (ppm) :2. 62 (s, 3H), 3.73 (s, 3H), 3.83 (s, 3H), 6.93 (d, 1H, J = 8.4 Hz), 7.26 (dd, 1H, J = 8.4, 7.2 Hz), 7.37 (dd, 1H, J = 8.4, 1.8 Hz), 7.67 (d, 1H, J = 7.2), 7.73 (d, 1H, J = 8.4 Hz), 8.04 (s, 1H), 9.23 (s, 1H), 9.73 (s, 1H); MALDI TOF-MS (m/z): 296 $[M+H]^+$.

Reference Example 2

Preparation of 2,8-dichloroquinazoline (Raw compound (III) of Compounds (I), (II) of the present invention)

In accordance with Schemes 2 and 3 described above, 2,8-dichloroquinazoline was prepared in the following manner.

First Step: Step 2-1 of Scheme 2

An NMP solution (5 mL) of 2-amino-3-chlorobenzoic acid (1.0 g, 5.83 mmol) and urea (1.05 mg, 17.5 mmol) was heated to a temperature of 180 to 190°C and then stirred for 4 hours. After cooling to room temperature, water (15 mL) was added, followed by stirring for 0.5 hour. The precipitate was filtered, washed and then dried to obtain 819 mg of the following 8-chloro-2,4-quinazolinedione (VI)-B, which is a kind of the compound (VI) in Scheme 2.

[Chemical Formula 48]

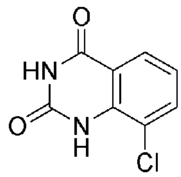

(VI)-B

$^1$H-NMR (DMSO-$d_6$) d (ppm) : 11.50 (s, 1H), 10.66 (s, 1H), 7.87 (d, 1H, J = 8.4 Hz), 7.76 (d, 1H, J = 7.8 Hz), 7.17 (t, 1H, J = 8.4 Hz).

Second Step: Step 2-2 of Scheme 2

A mixture of the product (VI)-B (700 mg, 3.56 mmol) of the first step, N,N-dimethylaniline (0.1 mL) and $POCl_3$ (7 mL) was stirred at 115°C for 4 hours. After cooling to room temperature, the reaction mixture was added to ice water (20 mL). The precipitated solid was collected by filtration, washed with water and then dried to obtain 414 mg of 2,4,8-trichloroquinazoline (VII)-B, which is a kind of the compound (VII) in Scheme 2.

[Chemical Formula 49]

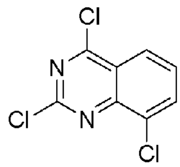

(VII)-B

$^1$H-NMR ($CD_3OD$) d (ppm): 8.31 (d, 1H, J = 8.1 Hz) 8.21 (d, 1H, J = 8.7 Hz), 7.78 (t, 1H, J = 8.1 Hz).

Third Step: Step 3-1 of Scheme 3

A THF solution (2 mL) of the product (VII)-B (151 mg, 0.65 mmol) of the second step and 28% ammonia water (1.5 mL) was stirred at room temperature overnight. The solvent was concentrated under reduced pressure and water (5 mL) was added to the resulting residue. The precipitated solid was collected by filtration, washed with water and then dried to obtain 122 mg of 2,8-dichloroquinazoline-4-amine (VIII)-B, which is a kind of the compound (VIII) in Scheme 3.

[Chemical Formula 50]

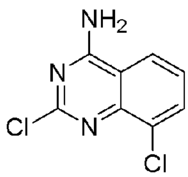

(VIII)-B

$^1$H-NMR ($CD_3OD$) d (ppm): 8.05 (d, 1H, J = 8.4 Hz), 7.90(d, 1H, J = 7.5 Hz), 7.44 (t, 1H, J = 8.0 Hz).

Fourth Step

A THF solution (2 mL) of the product (VIII)-B (87 mg, 0.41 mmol) of the third step and isoamyl nitrite (0.11 mL, 0.81 mmol) was heated to 60°C and then stirred for 4 hours. After cooling to room temperature, the solution was diluted with water and then extracted with ethyl acetate. The resulting organic layer was dried over anhydrous magnesium sulfate and then the solvent was distilled off under reduced pressure. The residue was purified by silica gel chromatography (ethyl acetate:hexane = 3:1) to obtain 60 mg of 2,8-dichloroquinazoline (III)-B.

[Chemical Formula 51]

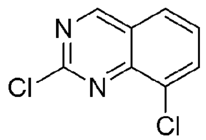

(III)-B

$^1$H-NMR ($CD_3OD$) d (ppm): 9.49 (s, 1H), 8.13 (m, 2H), 7.73 (t, 1H, J = 7.8 Hz).

Example 31

Preparation of N-(3-(8-chloroquinazolin-2-ylamino)phenyl)acetamide

In accordance with Scheme 1 described above, the compound (II) of the present invention (corresponding to the compound (I) in Scheme 1) was prepared.

Specifically, an n-butanol (2 mL) solution of 2,8-dichloroquinazoline (compound (III)-B) (70 mg, 0.35 mmol) of Reference Example 2 and 3-aminoacetoanilide (66 mg, 0.35 mmol) was stirred at 110°C for 3 hours. The solvent was distilled off under reduced pressure and then the residue was purified by silica gel chromatography ($CH_2Cl_2$:ethyl acetate = 1:1) to obtain 35 mg of the following compound (II)-U, which is a kind of the compound (II) of the present invention, as a yellow powder.

[Chemical Formula 52]

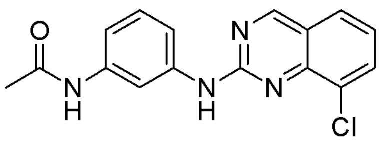

(II)-U

$^1$H-NMR (DMSO-$d_6$) d (ppm): 10.14 (s, 1H), 9.85 (s, 1H), 9.34 (s, 1H), 8.14(s, 1H), 7.97 (d, 1H, J = 6.6 Hz), 7.90 (m, 2H), 7.35 (t, 1H, J = 7.8 Hz), 7.23 (m, 2H), 2.03 (s, 3H); ESI-MS (m/z): 313 $[M+H]^+$.

Example 32

Preparation of 8-chloro-N-(1H-indazol-6-yl)quinazoline-2-amine

In accordance with Scheme 1 described above, the compound (I) of the present invention was prepared.

Specifically, 39 mg of the following compound (I)-K, which is a kind of the compound (I) of the present invention, was obtained as a yellow powder by reacting and treating in the same manner as in Example 31, except for using 2,8-dichloroquinazoline (compound (III)-B) (47 mg, 0.24 mmol) of Reference Example 2 and 6-aminoindazole (31 mg, 0.24 mmol).

[Chemical Formula 53]

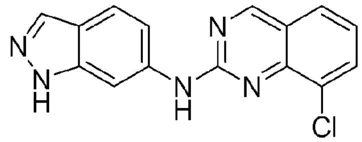

(I)-K

$^1$H-NMR (DMSO-$d_6$) d (ppm): 12.97 (s, 1H), 10.35 (s, 1H), 9.39 (s, 1H), 8.95 (s, 1H), 8.02 (d, 1H, J = 7.8 Hz), 7.92 (m, 2H), 7.65 (d, 1H, J = 9.0 Hz), 7.40 (m, 2H); ESI-MS (m/z): 296 $[M+H]^+$.

Reference Example 3

Preparation of 2-chloro-7-fluoro-8-methylquinazoline

First Step

2-amino-4-fluoro-3-methylbenzoic acid [which can be synthesized, for example, by the method described in J. Med. Chem. 1991, 34, 217] (11.4 g, 67.45 mmol) and an NMP solution (24 mL) of urea (12.14 g, 20.23 mmol) were stirred at 180°C for 4 hours. After cooling to room temperature, the reaction mixture was diluted with water (60 mL) and the precipitated solid was collected by filtration. The solid was washed in turn with water and ethyl acetate and then dried to obtain 10.0 g of 7-fluoro-8-methyl-2,4-quinazolinedione.

[Chemical Formula 54]

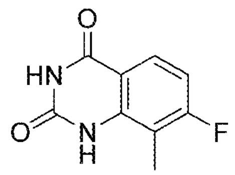

$^1$H-NMR (400 MHz, DMSO-$d_6$) d (ppm): 2.22 (s, 3H), 7.01 (t, 1H, J = 9.0 Hz), 7.81 (dd, 1H, J = 8.4, 6.8 Hz), 10.61 (br, 1H), 11.38 (br, 1H); ESI-MS (m/z): 195 $[M+H]^+$.

Second Step

A mixture of the product (1.5 g) of the first step, N,N-dimethylaniline (5 mL) and phosphoryl chloride (25 mL) was refluxed for 12 hours. After cooling to room temperature, the solvent was distilled off under reduced pressure. The residue was added to ice water, followed by extraction with ethyl acetate. The resulting organic layer was dried over an anhydrous sodium sulfate and the solvent was distilled off under reduced pressure, and then the residue was purified by silica gel chromatography (ethyl acetate:hexane= 1:9) to obtain 0.55 g of 2,4-dichloro-7-fluoro-8-methylquinazoline.

[Chemical Formula 55]

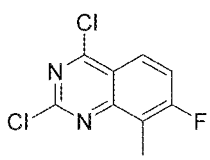

$^1H$ -NMR (400 MHz, $CDCl_3$) d (ppm) : 2.62 (s, 3H), 7.44 (t, 1H, J = 8.8 Hz), 8.12 (dd, 1H, J = 5.8, 9.0 Hz); ESI-MS (m/z): 230 $[M+H]^+$.

Third Step

To a two-phase solution of a dichloromethane solution (21 mL) of the product (0.50 g, 2.17 mmol) of the second step and an aqueous 9% ammonia-containing saturated brine solution (14 mL), a zinc powder (0.92g ) was added, followed by reflux for 6 hours. The reaction mixture was filtered through cerite, and the filtrate was washed in turn with dilute hydrochloric acid (10%) and a saturated brine solution and then dried over anhydrous sodium sulfate. The solvent was distilled off under reduced pressure and the residue was purified by silica gel chromatography (ethyl acetate:hexane = 1:4) to obtain 0.187 g of 2-chloro-7-fluoro-8-methylquinazoline as a colorless powder.

[Chemical Formula 56]

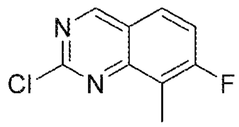

$^1H$-NMR (400 MHz, $CDCl_3$) d (ppm): 2.62 (s, 3H), 7.41 (t, 1H, J = 8.9 Hz), 8.06 (dd, 1H, J = 5.5, 8.6 Hz), 9.21 (s, 1H); ESI-MS (m/z): 197 $[M+H]^+$.

Reference Example 4

Preparation of 2-chloro-7-methoxy-8-methylquinazoline

First Step

To an ethanol solution (50 mL) 2-amino-4-methoxy-3-methylbenzoic acid [which can be synthesized, for example, by the method described in J. Med. Chem. 1991, 34, 217 or WO2007014927] (2.3 g, 12.70 mmol), thionyl chloride (10 mL) was added while cooling to 0°C under a nitrogen gas flow, and then reaction mixture was refluxed for 24 hours. After cooling to room temperature, the solvent was distilled off under reduced pressure and an aqueous saturated sodium hydrogen carbonate solution (15 mL) was added to the resulting residue, followed by extraction with ethyl acetate. The resulting organic layer was dried over anhydrous sodium sulfate and the solvent was distilled off under reduced pressure, and then the residue was purified by silica gel chromatography (ethyl acetate:hexane= 1:9 to 3:17) to obtain 1.01 g of ethyl 2-amino-4-methoxy-3-methylbenzoate.

[Chemical Formula 57]

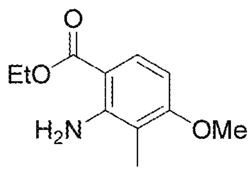

ESI-MS (m/z): 210 $[M+H]^+$.

Second Step

A mixture of the product (1.00 g, 4.78 mmol) of the first step and urea (2.29 g, 38.27 mmol) was stirred in a sealed tube at 220°C for 2 hours. After cooling to room temperature, the reaction mixture was suspended in ethyl acetate and the solid was collected by filtration. The solid was washed with water and then dried to obtain 2.01 g of a mixture of urea and 7-methoxy-8-methyl-2,4-quinazolinedione

[Chemical Formula 58]

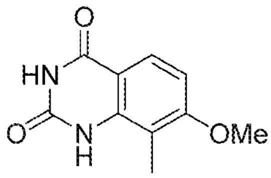

$^1$H-NMR (400 MHz, DMSO-$d_6$) d (ppm): 2.14 (s, 3H), 3.87 (s, 3H), 6.90 (d, 1H, J = 8.0 Hz), 7.79 (d, 1H, J = 8.8 Hz), 10.14 (br, 1H), 11.05 (br, 1H); ESI-MS (m/z): 207 $[M+H]^+$.

Third Step

A mixture of the product (2.0 g) of the second step, N,N-dimethylaniline (5 mL) and phosphoryl chloride (50 mL) was refluxed for 7 hours. After cooling to room temperature, the solvent was distilled off under reduced pressure and the residue was added to ice water, followed by extraction with ethyl acetate. The resulting organic layer was dried over anhydrous sodium sulfate and the solvent was distilled off under reduced pressure, and then the residue was purified by silica gel chromatography (ethyl acetate:hexane= 1:9) to obtain 0.95 g of 2,4-dichloro-7-methoxy-8-methyl-quinazoline.

[Chemical Formula 59]

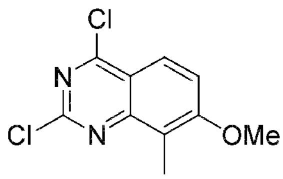

$^1$H-NMR (400 MHz, $CDCl_3$) d (ppm) : 2.54 (s, 3H), 4.04 (s, 3H), 7.38 (d, 1H, J = 9.2 Hz), 8.12 (d, 1H, J = 10.1 Hz); ESI-MS (m/z): 243, 245 $[M+H]^+$.

Fourth Step

To a two-phase solution of a dichloromethane solution (10 mL) of the product (0.20 g, 0.82 mmol) of the third step and an aqueous 9% ammonia-containing saturated brine solution (10 mL), a zinc powder (0.215 g) was added, followed by reflux for 4 hours. The reaction mixture was filtered through cerite, and the filtrate was washed in turn with dilute hydrochloric acid (10%) and a saturated brine solution and then dried over anhydrous sodium sulfate. The solvent was distilled off under reduced pressure and the residue was purified by silica gel chromatography (ethyl acetate:hexane = 1:4) to obtain 0.104 g of 2-chloro-7-methoxy-8-methylquinazoline as a colorless powder.

[Chemical Formula 60]

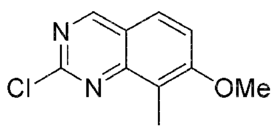

$^1$H-NMR (400 MHz, $CDCl_3$) d (ppm): 2.55 (s, 3H), 4.03 (s, 3H), 7.37 (d, 1H, J = 9.2 Hz), 7.80 (d, 1H, J = 9.0 Hz), 9.09 (s, 1H); ESI-MS (m/z): 209 $[M+H]^+$.

Reference Example 5

Preparation of 2-chloro-8-ethylquinazoline

First Step

An NMP solution (3 mL) of a mixture of 2-amino-3-ethylbenzoic acid (382 mg, 2.31 mmol) and urea (417 mg, 6.94 mmol) was stirred at 180°C for 5 hours. To the reaction mixture, ice water was added and the precipitated solid was collected by filtration to obtain 382 mg of a mixture of 8-ethyl-2,4-quinazolinedione.

[Chemical Formula 61]

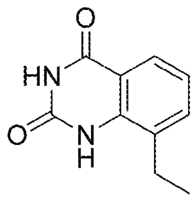

Second Step

A mixture of the product (313 mg, 1.65 mmol) of the first step and phosphoryl chloride (3 mL) was stirred at 115°C for 4 hours. The solvent was distilled off under reduced pressure and the residue was purified by silica gel chromatography to obtain 221 mg of 2,4-dichloro-8-ethylquinazoline.

[Chemical Formula 62]

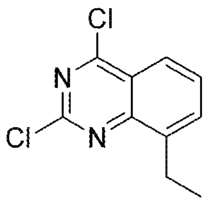

Third Step

A THF solution (2 mL) of the product (221 mg, 0.97 mmol) of the second step and 28% ammonia water (2 mL) was stirred at room temperature overnight. The solvent was distilled off under reduced pressure and the residue was purified by silica gel chromatography to obtain 183 mg of 2-chloro-8-ethylquinazoline-4-amine.

[Chemical Formula 63]

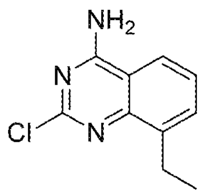

Fourth Step

A THF solution (5 mL) of the product (183 mg, 0.88 mmol) of the third step and isoamyl nitrite (0.24 mL, 1.76

mmol) was heated to 60°C and then stirred for 5 hours. The solution was cooled to room temperature, diluted with water and then extracted with ethyl acetate. The solvent was distilled off under reduced pressure and the residue was purified by silica gel chromatography to obtain 61 mg of 2-chloro-8-ethylquinazoline.

[Chemical Formula 64]

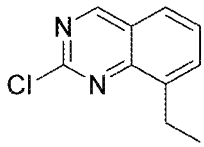

Example 33

Preparation of N-(1H-indazol-6-yl)-8-ethylquinazoline-2-amine

[Chemical Formula 65]

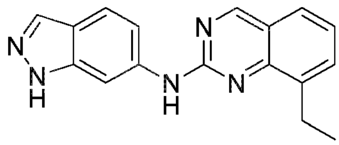

11 mg of the titled compound was obtained by reacting and treating 2-chloro-8-ethylquinazoline (see Reference Example 5) (61 mg, 0.317 mmol) and 6-aminoindazole (42 mg, 0.317 mmol) in the same manner as in Example 31. $^1$H-NMR (300 MHz, DMSO-$d_6$) d (ppm): 1.39 (t, 3H, J = 7.2 Hz), 3.1-3.3 (m, 2H), 7.28 (t, 1H, J = 7.5 Hz),7.41 (d, 1H, J = 9.0 Hz), 7.63 (d, 1H, J = 8.4 Hz), 7.70 (d, 1H, J = 6.9 Hz),7.76 (d, 1H, J =7.5 Hz), 7.93 (s, 1H), 8.73 (s, 1H), 9.29 (s, 1H), 10.05 (s, 1H), 12.95 (br, 1H)

Example 34

Preparation of 8-methyl-N-(3-methyl-1H-indazol-6-yl)-quinazoline-2-amine

[Chemical Formula 66]

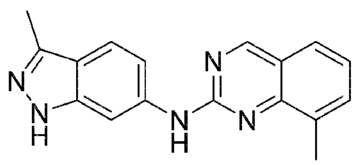

First Step

[Chemical Formula 67]

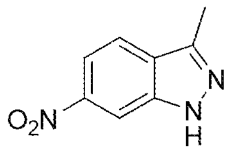

To a glacial acetic acid solution (300 mL) of 2-ethyl-5-nitroaniline (15.8 g, 95 mmol), a glacial acetic acid solution (40 mL) of tert-butyl nitrite (9.8 g, 95 mmol) was gradually added over 15 minutes, After stirring for 30 minutes, acetic acid was distilled off under reduced pressure to obtain an orange solid. The resulting solid was dissolved in ethyl acetate and then washed three times with 100 mL of a saturated sodium hydrogen carbonate solution. The organic layer was dried over anhydrous magnesium sulfate and the solvent was concentrated under reduced pressure to obtain 11.7 g of 6-nitro-3-methylindazole.
$^1$H-NMR ($CDCl_3$) d (ppm): 2.65 (s, 3H), 7.79 (d, 1H, J = 8.4 Hz), 8.03 (d, 1H, J = 8.4 Hz), 8.40 (s, 1H), 10.30 (br, 1H).

Second Step

[Chemical Formula 68]

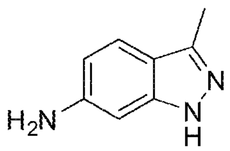

To an ethyl acetate solution (50 mL) of the product (1.17 g, 6.60 mmol) of the first step, 10% Pd-C (0.46 g) was added, followed by stirring under a hydrogen gas flow at room temperature for 10 hours. The insoluble substances were filtered through cerite and the filtrate was concentrated under reduced pressure. The residue was purified by silica gel chromatography (ethyl acetate:n-hexane = 3:1) to obtain 0.57 g of 6-amino-3-methylindazole.
$^1$H-NMR ($CDCl_3$) d: 2.50 (s, 3H), 6.4-6.65 (m, 2H), 7.43 (d, 1H, J = 8.4 Hz), 9.35 (br, 1H).

Third Step

A mixed solution of 2-chloro-8-methylquinazoline (see Reference Example 1) (89 mg, 0.5 mmol), the product (88 mg, 0.6 mmol) of the second step and n-butanol (2.0 mL) was reacted for 120 minutes using a microwave synthesizer (manufactured by CEM Co., 120°C, 100W). The reaction solution was air-cooled to room temperature, and the precipitated solid was collected by filtration and then washed with cold 2-propanol to obtain 75 mg of 8-methyl-N-(3-methyl-1H-indazol-6-yl)-quinazoline-2-amine as a yellow solid.
$^1$H-NMR (DMSO-$d_6$) d: 2.45 (s, 3H), 2.71 (s, 3H), 7.31 (t, 1H, J = 7.2 Hz), 7.41 (d, 1H, J = 8.4 Hz), 7.58 (d, 1H, J = 8.4 Hz), 7.72 (d, 1H, J = 7.2 Hz), 7.77 (d, 1H, J = 8.4 Hz), 8.65 (s, 1H), 9.29 (s, 1H), 10.02 (s, 1H), 12.47 (br, 1H); ESI-MS (m/z) : 290 $[M+H]^+$.

Example 35

Preparation of N-(indoline-6-yl)-8-methylquinazoline-2-amine

[Chemical Formula 69]

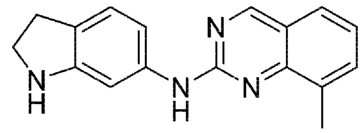

A mixed solution of 2-chloro-8-methylquinazoline (see Reference Example 1) (89 mg, 0.5 mmol), tert-butyl 6-aminoindoline-1-carboxylate (117 mg, 0.5 mmol) and n-butanol (2.0 mL) was reacted for one hour using a microwave synthesizer (manufactured by CEM Co., 120°C, 100W). The reaction mixture was diluted with ethyl acetate, washed in turn with an aqueous saturated sodium hydrogen carbonate solution and water, and then dried over anhydrous magnesium sulfate. The solvent was distilled off under reduced pressure and the resulting residue was purified by silica gel chromatography (ethyl acetate:n-hexane = 1:3 to 1:1) to obtain 8.0 mg of the titled compound.
$^1$H-NMR (DMSO-$d_6$) d: 2.60 (s, 3H), 2.86 (t, 2H, J = 8.4 Hz), 3.41 (t, 2H, J = 8.4 Hz), 5.49 (s, 1H), 6.95 (d, 1H, J = 7.8 Hz), 7.20 (dd, 1H, J = 7.8, 1.2 Hz), 7.24 (dd, 1H, J = 7.8, 7.2 Hz), 7.36 (dd, 1H, J = 1.2 Hz), 7.65 (d, 1H, J = 7.2 Hz), 7.71 (d, 1H, J = 7.2 Hz), 7.71 (d, 1H, J = 7.8 Hz), 9.20 (s, 1H), 9.55 (s, 1H); MALDI TOF-MS (m/z): 282 $[M+H]^+$.

Example 36

Preparation of 8-methyl-N-(7-methyl-1H-indazol-6-yl)-quinazoline-2-amine

[Chemical Formula 70]

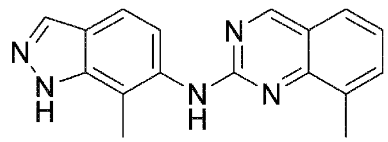

60 mg of the titled compound was obtained by reacting and treating 2-chloro-8-methylquinazoline (see Reference Example 1) (89 mg, 0.5 mmol) and 6-amino-7-methylindazole [which can be synthesized, for example, by the method described in WO9823609] (88 mg, 0.6 mmol) in the same manner as in Example 35.
$^1$H-NMR (DMSO-$d_6$) d: 2.42 (s, 3H), 2.44 (s, 3H), 7.20 (dd, 1H, J = 7.8, 7.2 Hz), 7.44 (d, 1H, J = 8.4 Hz), 7.54 (d, 1H, J = 8.4 Hz), 7.59 (d, 1H, J = 7.2 Hz), 7.69 (d, 1H, J = 7.8 Hz), 8.01 (s, 1H), 9.14 (s, 1H), 9.19 (s, 1H); ESI-MS (m/z): 290 $[M+H]^+$.

Example 37

Preparation of 8-methyl-N-(4-methyl-1H-indazol-6-yl)-quinazoline-2-amine

[Chemical Formula 71]

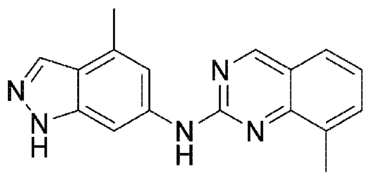

First Step

[Chemical Formula 72]

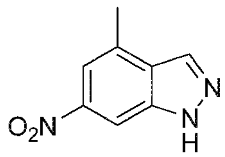

To a glacial acetic acid solution (120 mL) of 2,3-dimethyl-5-nitroaniline [which can be synthesized, for example, by the method described in WO2005117819] (1.0 g, 6.02 mmol), an aqueous solution (3 mL) of sodium nitrite (0.42 g, 6.14 mmol) was added under ice cooling. The mixed solution was stirred under ice cooling for one hour, and then stirred at room temperature for 2 days. Acetic acid was distilled off under reduced pressure and water was added to the resulting residue, followed by stirring under ice cooling for 30 minutes. The precipitate was collected by filtration to obtain 1.14 g of 6-nitro-4-methylindazole.
$^{1}$H-NMR (500 MHz, DMSO-$d_6$) d: 2.67 (s, 3H), 7.75 (s, 1H), 8.27 (s, 1H), 8.37 (s, 1H).

Second Step

[Chemical Formula 73]

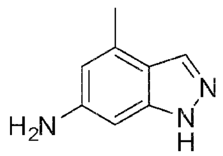

To an aqueous 80% ethanol solution (75 mL) of the product (0.80 g, 4.52 mmol) of the first step, ammonium chloride (0.12 g, 2.26 mmol) and iron (2.56 g, 45.2 mmol) were added, followed by reflux for one hour. The reaction mixture was cooled to room temperature and insoluble substances were filtered through cerite, followed by washing in turn with ethanol and ethyl acetate. The filtrate was concentrated under reduced pressure and water was added to the resulting residue, and then the precipitated solid was collected by filtration to obtain 0.43 g of 6-amino-4-methylindazole.
$^{1}$H-NMR (500 MHz, DMSO-$d_6$) d: 2.37 (s, 3H), 5.09 (s, 2H), 6.25 (s, 1H), 6.31 (s, 1H), 7.74 (s, 1H), 12.19 (s, 1H).

Third Step

50 mg of 8-methyl-N-(4-methyl-1H-indazol-6-yl)-quinazoline-2-amine was obtained as a yellow solid by reacting and treating 2-chloro-8-methylquinazoline (see Reference Example 1) (89 mg, 0.5 mmol) and the product (77 mg, 0.53 mmol) of the second step in the same manner as in Example 34.
$^1$H-NMR (500 MHz, DMSO-$d_6$) d: 2.52 (s, 3H), 2.71 (s, 3H), 7.2-7.4 (m, 2H), 7.72 (d, 1H, J = 7.1 Hz), 7.77 (d, 1H, J = 7.6 Hz), 7.97 (s, 1H), 8.52 (s, 1H), 9.29 (s, 1H), 9.96 (s, 1H), 12.88 (s, 1H); ESI-MS (m/z): 290 $[M+H]^+$.

Example 38

Preparation of 8-methyl-N-(5-methyl-1H-indazol-6-yl)-quinazoline-2-amine

[Chemical Formula 74]

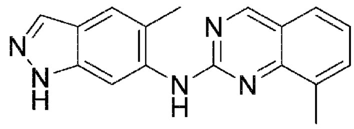

22 mg of the titled compound was obtained by reacting and treating 2-chloro-8-methylquinazoline (see Reference Example 1) (89 mg, 0.5 mmol) and 6-amino-5-methylindazole [which can be synthesized, for example, by the method described in WO2001017982] (74 mg, 0.5 mmol) in the same manner as in Example 14.
$^1$H-NMR (500 MHz, DMSO-$d_6$) d: 2.42 (s, 3H), 2.57 (s, 3H), 7.28 (dd, 1H, J = 7.9, 7.2 Hz), 7.57 (s, 1H), 7.68 (d, 1H, J = 7.9 Hz), 7.76 (d, 1H, J = 7.1 Hz), 7.92 (s, 1H), 8.41 (s, 1H), 8.72 (s, 1H), 9.27 (s, 1H), 12.87 (s, 1H); ESI-MS (m/z): 290 $[M+H]^+$.

Example 39

Preparation of (6-(8-methylquinazolin-2-ylamino)-1H-indazol-3-yl)methanol

[Chemical Formula 75]

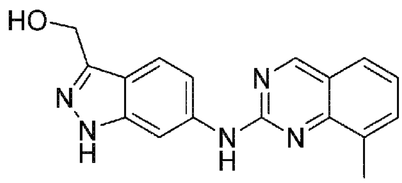

To an aqueous 80% dioxane solution (2 mL) of 2-chloro-8-methylquinazoline (see Reference Example 1) (36 mg, 0.2 mmol) and (6-aminoindazol-3-yl)methanol [which can be synthesized, for example, by the method described in U.S. Patent Application Publication No. 2006/194801] (40 mg, 0.25 mmol), a drop of concentrated hydrochloric acid was added and then the reaction was conducted for 30 minute using a microwave synthesizer (manufactured by Biotage, Ltd., 120°C). The reaction mixture was diluted with ethyl acetate, washed in turn with an aqueous saturated sodium hydrogen carbonate solution and water, and then dried over anhydrous magnesium sulfate. The solvent was distilled off under reduced pressure the resulting residue was purified by silica gel chromatography (ethyl acetate:n-hexane = 1:4 to 4:1) to obtain 10 mg of the titled compound.
$^1$H-NMR (500 MHz, DMSO-$d_6$) d: 2.71(s, 3H), 4.75 (d, 2H, J = 3.5 Hz), 5.1-5.4 (m, 1H), 7.31 (dd, 1H, J = 7.7, 7.4 Hz), 7.42 (dd, 1H, J = 8.8, 1.8 Hz), 7.65-7.75 (m, 2H), 7.77 (d, 1H, J = 8.8 Hz), 8.69 (s, 1H), 9.58 (s, 1H), 10.05 (s, 1H), 12.65 (s, 1H); ESI-MS (m/z): 306 $[M+H]^+$, .304 $[M-H]^-$.

Example 40

Preparation of N-[3-(butoxymethyl)-1H-indazol-6-yl]-8-methylquinazoline-2-amine

[Chemical Formula 76]

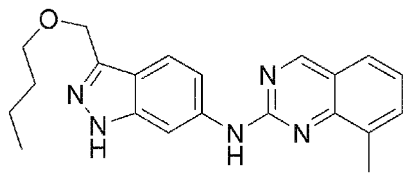

38 mg of the titled compound was obtained by reacting and treating 2-chloro-8-methylquinazoline (see Reference Example 1) (179 mg, 1.0 mmol) and (6-aminoindazol-3-yl)methanol [which can be synthesized, for example, by the method described in U.S. Patent Application Publication No. 2006/194801] (269 mg, 1.65 mmol) in the same manner as in Example 1.
$^1$H-NMR (500 MHz, DMSO-$d_6$) d: 0.85 (t, 3H, J = 7.4 Hz), 1.32 (q, 2H, J = 7.4 Hz), 1.4-1.6 (m, 2H), 2.72 (s, 3H), 3.46 (t, 2H, J = 6.5 Hz), 4.72 (s, 2H), 7.32 (t, 1H, J = 7.5 Hz), 7.43 (d, 1H, J = 8.8 Hz), 7.65 (d, 1H, J = 8.8 Hz), 7.73 (d, 1H, J = 7.0 Hz), 7.77 (d, 1H, J = 7.9 Hz), 8.71 (s, 1H), 9.30 (s, 1H), 10.07 (s, 1H) 12.80 (s, 1H); ESI-MS (m/z): 362 $[M+H]^+$, . 360 $[M-H]^-$.

Example 41

Preparation of [6-(8-methylquinazolin-2-ylamino)-1H-indazol-4-yl]methanol

[Chemical Formula 77]

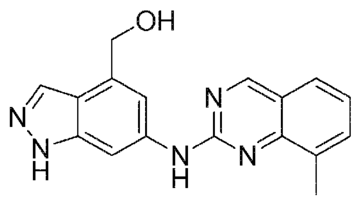

First Step

[Chemical Formula 78]

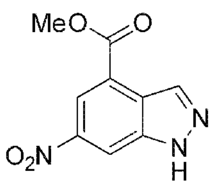

To a glacial acetic acid solution (450 mL) of methyl 3-amino-2-methyl-5-nitrobenzoate [which can be synthesized, for example, by the method described in JP04295441] (3.99 g, 19.0 mmol), an aqueous solution (8 mL) of sodium nitrite (1.35 g, 19.6 mmol) was added under ice cooling. After stirring under ice cooling for one hour, the solution was further stirred at room temperature for 3 days. Acetic acid was distilled off under reduced pressure and water was added to the resulting residue, followed by stirring under ice cooling for 30 minutes. The precipitate was collected by filtration to obtain 3.99 g of methyl 6-nitro-1H-indazole-4-carboxylate. $^1$H-NMR (-NMR (500 MHz, DMSO-$d_6$) d: 4.02 (s, 3H), 8.48 (d, 1H, J = 1.6 Hz), 8.62 (d, 1H, J = 0.8 Hz), 8.76 (br, 1H).

Second Step

[Chemical Formula 79]

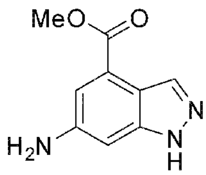

To an aqueous 80% ethanol solution (250 mL) of the product (3.00 g, 13.56 mmol) of the first step, ammonium chloride (0.363 g, 6.78 mmol) and iron (7.57 g, 136 mmol) were added, followed by reflux for one hour. After cooling the reaction mixture to room temperature, insoluble substances were filtered through cerite and then washed in turn with ethanol and ethyl acetate. The filtrate was concentrated under reduced pressure and water was added to the resulting residue, and then the precipitated solid was collected by filtration to obtain 2.32 g of methyl 6-amino-1H-indazole-4-carboxylate.
$^1$H-NMR (500 MHz, DMSO-$d_6$) d: 3.89 (s, 3H), 5.52 (s, 2H), 6.77 (dd, 1H, J = 1.8, 1.0 Hz), 7.26 (d, 1H, J = 1.8 Hz), 8.07 (s, 1H), 12.59 (s, 1H).

Third Step

[Chemical Formula 80]

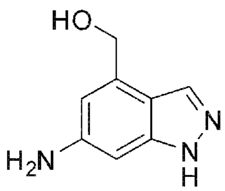

To a THF solution (200 mL) of the product (2.28 g, 11.9 mmol) of the second step, a THF solution (1 M, 36 mL) solution of lithium aluminum hydride was added under ice cooling, followed by stirring at room temperature for 20 hours. To the reaction mixture, water (2 mL), an aqueous 15% sodium hydroxide solution (2 mL) and water (5 mL) were sequentially added under ice cooling thereby terminating the reaction. After insoluble substances were filtered through cerite, the filtrate was concentrated under reduced pressure to obtain 1.34 g of (6-amino-1H-indazol-4-yl)methanol.
$^1$H-NMR (500 MHz, DMSO-$d_6$) d: 4.63 (d, 2H, J = 5.6 Hz), 5.0-5.25 (m, 3H), 6.35 (dd, 1H, J = 1.2, 0.4 Hz), 6.46 (dd, 1H, J = 1.6, 0.8 Hz), 7.76 (s, 1H), 12.21 (br, 1H).

Fourth Step

To an aqueous 90% dioxane aqueous solution (17 mL) of 2-chloro-8-methylquinazoline (see Reference Example 1) (357 mg, 2.0 mmol) and the product (408 mg, 2.5 mmol) of the third step, 50 μL of concentrated hydrochloric acid was added and the reaction was conducted for 30 minutes using a microwave synthesizer (manufactured by Biotage, Ltd., 120°C). The reaction mixture was diluted with ethyl acetate, washed with water, and then dried over anhydrous sodium sulfate. The solvent was distilled off under reduced pressure to obtain 120 mg of [6-(8-methylquinazolin-2-ylamino)-1H-indazol-4-yl]methanol.
$^1$H-NMR (500 MHz, DMSO-$d_6$) d: 2.72 (s, 3H), 4.79 (d, 2H, J = 5.1 Hz), 5.30 (t, 1H, J = 5.5 Hz), 7.31 (t, 1H, J = 7.5 Hz), 7.51 (s, 1H), 7.72 (d, 1H, J = 7.0 Hz), 7.77 (d, 1H, J = 8.0 Hz), 8.01 (s, 1H), 8.58 (s, 1H), 9.29 (s, 1H), 10.03 (s, 1H), 12.90 (s, 1H); ESI-MS (m/z): 306 $[M+H]^+$.

Example 42

Preparation of 8-methyl-N-[4-(trifluoromethyl)-1H-indazol-6-yl]-quinazoline-2-amine

[Chemical Formula 81]

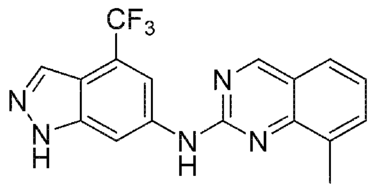

First Step

[Chemical Formula 82]

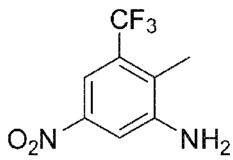

To concentrated sulfuric acid (30 mL), 2-methyl-3-trifluoromethylaniline (5.0 g, 28.5 mmol) was gradually added under ice cooling and fuming nitric acid (1.35 mL, 32.6 mmol) was added dropwise so that the inner temperature does not become higher than 15°C, followed by stirring for one hour while maintaining the liquid temperature at 15 to 20°C. The reaction mixture was gradually added to ice water and then extracted with ethyl acetate. The organic layer was washed with water and dried over anhydrous sodium sulfate, and then the solvent was distilled off under reduced pressure. The resulting residue was purified by silica gel chromatography (ethyl acetate:n-hexane = 1:10 to 1:1) to obtain 3.80 g of 2-methyl-5-nitro-3-trifluoromethylaniline.
$^1$H-NMR (400 MHz, DMSO-$d_6$) d: 2.21 (d, 3H, J = 1.2 Hz), 6.10 (br, 2H), 7.56 (d, 1H, J = 2.4 Hz), 7.74 (d, 1H, J = 2.4 Hz).

Second Step

[Chemical Formula 83]

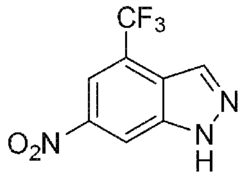

To a glacial acetic acid solution (450 mL) of the product (2.93 g, 13.31 mmol) of the first step, an aqueous solution (8 mL) of sodium nitrite (0.95 g, 13.77 mmol) was added under ice cooling. The mixed solution was stirred under ice cooling for one hour and further stirred at room temperature for one day. Acetic acid was distilled off under reduced pressure and water was added to the resulting residue, followed by stirring under ice cooling for 30 minutes. The precipitate was collected by filtration to obtain 2.90 g of 6-nitro-4-trifluoromethyl-1H-indazole.
$^1$H-NMR (400 MHz, DMSO-$d_6$) d: 8.25 (dd, 1H, J = 1.6, 0.4 Hz), 8.47 (d, 1H, J = 0.8 Hz), 8.06 (s, 1H), 14.38 (br, 1H).

Third Step

[Chemical Formula 84]

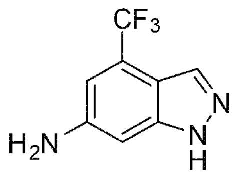

To an ethyl acetate solution (120 mL) of the product (0.93 g, 4.00 mmol) of the second step, 10% Pd-C (0.2 g) was added, followed by stirring under a hydrogen gas flow at room temperature for 6 hours. Insoluble substances were filtered through cerite and the filtrate was concentrated under reduced pressure. The residue was purified by silica gel chromatography (ethyl acetate:n-hexane = 1:4 to 1:1) to obtain 0.46 g of 6-amino-4-trifluoromethylindazole.
$^1$H-NMR (400 MHz, DMSO-$d_6$) d: 5.66 (br, 2H), 6.74 (s, 1H), 6.90 (t, 1H, J = 0.8 Hz), 7.81 (dd, 1H, J = 2.4, 1.6 Hz), 12.74 (br, 1H); ESI-MS (m/z): 202 $[M+H]^+$.

Fourth Step

50 mg of 8-methyl-N-[4-(trifluoromethyl)-1H-indazol-6-yl]-quinazoline-2-amine was obtained by reacting and treating 2-chloro-8-methylquinazoline (see Reference Example 1) (89 mg, 0.5 mmol) and the product (106 mg, 0.53 mmol) of the third step in the same manner as in Example 34.
$^1$H-NMR (500 MHz, DMSO-$d_6$) d: 2.71 (s, 3H), 7.36 (dd, 1H, J = 7.9, 7.2 Hz), 7.76 (dt, 1H, J = 7.1, 1.3 Hz), 8.06 (t, 1H, J = 1.3 Hz), 8.28 (s, 1H), 8.77 (s, 1H), 9.35 (s, 1H), 10.38 (s, 1H), 13.46 (br, 1H); ESI-MS (m/z): 344 $[M+H]^+$, 342 $[M-H]^-$.

Example 43

Preparation of methyl 6-(8-methylquinazolin-2-ylamino)-1H-indazole-4-carboxylate

[Chemical Formula 85]

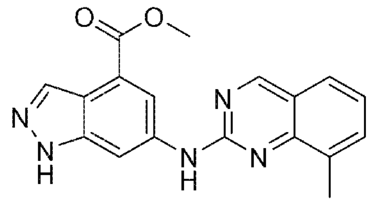

130 mg of the titled compound was obtained by reacting and treating 2-chloro-8-methylquinazoline (see Reference Example 1) (89 mg, 0.5 mmol) and methyl 6-amino-1H-indazole-4-carboxylate (see Example 41) (96 mg, 0.5 mmol) in the same manner as in Example 34.
$^1$H-NMR (500 MHz, DMSO-$d_6$) d: 2.73 (s, 3H), 3.96 (s, 3H), 7.34 (dd, 1H, J = 7.7, 7.4 Hz), 7.74 (d, 1H, J = 7.0 Hz), 7.80 (d, 1H, J = 8.0 Hz), 8.29 (d, 1H, J = 1.0 Hz), 8.57 (d, 1H, J = 1.8 Hz), 8.80 (d, 1H, J = 1.0 Hz), 9.33 (s, 1H), 10.31 (s, 1H); ESI-MS (m/z): 334 $[M+H]^+$.

Example 44

Preparation of 7-fluoro-N-(1H-indazol-6-yl)-8-methylquinazoline-2-amine

[Chemical Formula 86]

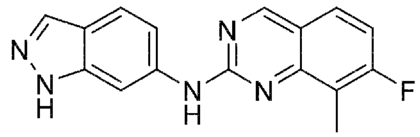

An n-butanol (1.3 mL) solution of 2-chloro-7-fluoro-8-methylquinazoline (see Reference Example 3) (50 mg, 0.255 mmol) and 6-aminoindazole (36 mg, 0.267 mmol) was stirred at 120°C for 3 hour. The reaction solution was air-cooled to room temperature and diluted with ethyl acetate, and then the precipitated solid was collected by filtration to obtain 45 mg of the titled compound.
$^1$H-NMR (400 MHz, DMSO-$d_6$) d (ppm): 2.60 (s, 3H), 7.28 (t, 1H, J = 9.1 Hz), 7.43 (d, 1H, J = 8.7 Hz), 7.65 (d, 1H, J = 8.7 Hz), 7.87 (t, 1H, J = 6.8 Hz), 7.95 (s, 1H), 8.70 (s, 1H), 9.30 (s, 1H), 10.19 (s, 1H), 12.99 (br, 1H); ESI-MS (m/z): 294 $[M+H]^+$.

Example 45

Preparation of N-(1H-indazol-6-yl)-7-methoxy-8-methylquinazoline-2-amine

[Chemical Formula 87]

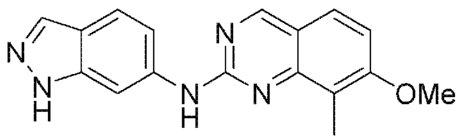

83 mg of the titled compound was obtained by reacting and treating 2-chloro-7-methoxy-8-methylquinazoline (see Reference Example 4) (100 mg, 0.48 mmol) and 6-aminoindazole (64 mg, 0.48 mmol) in the same manner as in Example 44. $^1$H-NMR (400 MHz, DMSO-$d_6$) d (ppm): 2.50 (s, 3H), 3.99 (s, 3H), 7.29 (d, 1H, J = 8.4 Hz), 7.42 (d, 1H, J = 8.0 Hz), 7.66 (d, 1H, J = 8.2 Hz), 7.85 (d, 1H, J = 8.2 Hz), 8.67 (s, 1H), 9.20 (s,1H), 10.13 (br, 1H), 12.91 (br, 1H); ESI-MS (m/z): 306 $[M+H]^+$.

Example 46

Preparation of N-{3-[(dimethylamino)methyl]-1H-indazol-6-yl}-8-methylquinazoline-2-amine

[Chemical Formula 88]

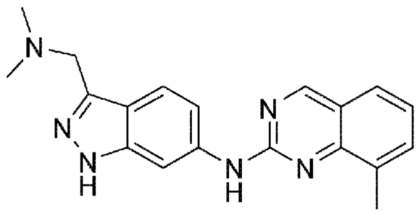

First Step

[Chemical Formula 89]

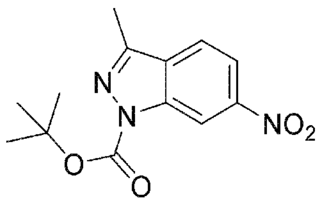

To a dichloromethane solution (10 mL) of 3-methyl-6-nitro-1H-indazole (see the first step of Example 34) (985 mg, 5.56 mol) , di-tert-butyl dicarbonate (1.58 g, 7.23 mmol), triethylamine (1.16 mL, 8.34 mmol) and a catalytic amount of N,N-dimethyl-4-aminopyridine (10 mg) were added, followed by stirring at room temperature for 3 hours. The reaction mixture was diluted with water and then extracted with dichloromethane. The solvent was distilled off under reduced pressure and the residue was purified by silica gel chromatography to obtain 1.23 g of tert-butyl 3-methyl-6-nitro-1H-indazole-1-carboxylate.

Second Step

[Chemical Formula 90]

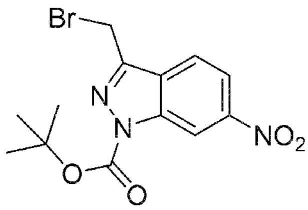

To a carbon tetrachloride solution (30 mL) of the product (1.0 g, 3.6 mmol) of the first step, N-bromosuccinimide (705 mg, 3.96 mmol) and azobisisobutyronitrile (0.02 mg) were added, followed by stirring at 85°C for 6 hours. The reaction mixture was cooled to room temperature and then filtered through cerite. The solvent was distilled off under reduced pressure and the resulting residue was purified by silica gel chromatography to obtain 611 mg of tert-butyl 3-(bromomethyl)-6-nitro-1H-indazole-1-carboxylate.

Third Step

[Chemical Formula 91]

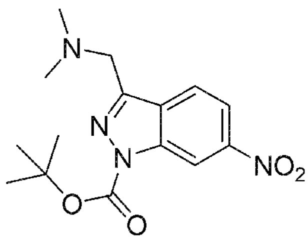

To a THF solution (3 mL) of the product (200 mg, 0.56 mmol) of the second step, a THF solution (2 M, 0.42 mL, 0.84 mmol) of dimethylamine was added, followed by stirring at room temperature for 3 hours. The solvent was distilled off under reduced pressure and the resulting residue was purified by silica gel chromatography to obtain 163 mg of tert-butyl 3-[(dimethylamino)methyl)]-6-nitro-1H-indazole-1-carboxylate.

Fourth Step

[Chemical Formula 92]

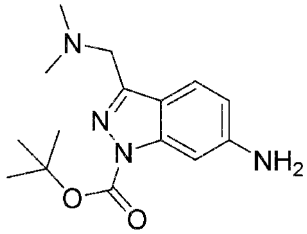

To a methanol solution (5 mL) of the product (163 mg, 0.51 mmol) of the third step, 10% palladium carbon (20 mg) was added, followed by stirring under a hydrogen gas flow at room temperature for 3 hours. The reaction mixture was filtered through cerite and the solvent was distilled off under reduced pressure to obtain 138 mg of tert-butyl 3-[(dimethylamino)methyl)]-6-amino-1H-indazole-1-carboxylate.

Fifth Step

An n-butanol (3 mL) solution of 2-chloro-8-methylquinazoline (see Reference Example 1) (85 mg, 0.48 mmol) and the product (138 mg, 0.48 mmol) of the fourth step was stirred at 120°C for 3 hours. The reaction solution was air-cooled to room temperature and the solvent was distilled off under reduced pressure, and then the residue was purified by preparative HPLC to obtain 5 mg of N-{3-[(dimethylamino)methyl]-1H-indazol-6-yl}-8-methylquinazoline-2-amine.
$^1$H-NMR (300 MHz, $CD_3OD$) d (ppm): 2.78 (s, 3H), 2.91 (s, 6H), 4.60 (s, 2H), 7.31 (t, 1H, J = 7.2 Hz), 7.40 (dd, 1H, J = 8.7, 1.8 Hz), 7.6-7.8 (m, 2H), 7.78 (d, 1H, J = 9.0 Hz), 8.91 (d, 1H, J = 1.5 Hz), 9.17 (s, 1H); ESI-MS /z): 333 $[M+H]^+$.

Example 47

Preparation of 8-methyl-N-(3-amino-1H-indazol-6-yl)-quinazoline-2-amine

[Chemical Formula 93]

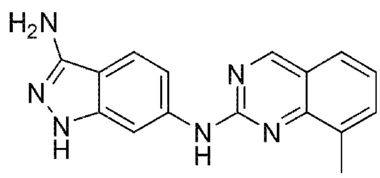

First Step

[Chemical Formula 94]

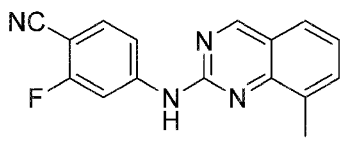

47 mg of 2-fluoro-4-(8-methylquinazolin-2-ylamino)benzonitrile was obtained by reacting and treating 2-chloro-8-methylquinazoline (see Reference Example 1) (131 mg, 0.74 mmol) and 4-amino-2-fluorobenzonitrile (100 mg, 0.74 mmol) in the same manner as in Example 31.

Second Step

To an n-butanol (2 mL) solution of the product (47 mg, 0.17 mmol) of the first step, hydrazine monohydrate (0.5 mL) was added, followed by stirring at 110°C for 5 hours. The reaction solution was air-cooled to room temperature and the solvent was distilled off under reduced pressure, and then the residue was purified by preparative HPLC to obtain 18 mg of 8-methyl-N-(3-amino-1H-indazol-6-yl)-quinazoline-2-amine. $^1$H-NMR (300 MHz, $CD_3OD$) d (ppm): 2.66 (s, 3H), 5.16 (s, 2H), 7.2-7.4 (m, 2H), 7.53 (d, 1H, J = 8.7 Hz), 7.6 (d, 1H, J = 7.2 Hz), 7.74 (d, 1H, J = 7.5 Hz), 8.40 (s, 1H), 9.26 (s, 1H), 9.90 (s, 1H), 11.25 (s, 1H); ESI-MS (m/z): 291 $[M+H]^+$.

Example 48

Preparation of 7-hydroxy-N-(1H-indazol-6-yl)-8-methylquinazoline-2-amine

[Chemical Formula 95]

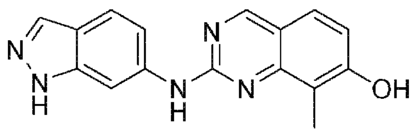

To a chloroform solution (10 mL) of the compound (50 mg, 0.16 mmol) of Example 45, borane tribromide (98%, 0.5 mL) was added, followed by reflux under a nitrogen gas flow overnight. The solvent was distilled off under reduced pressure and the residue was purified by preparative HPLC to obtain 18 mg of the titled compound.
$^1$H-NMR (400 MHz, DMSO-$d_6$) d (ppm): 2.54 (s, 3H), 7.01 (d, 1H, J = 8.7 Hz), 7.41 (m, 1H), 7.62 (d, 1H, J = 9.0 Hz), 7.92 (s, 1H), 8.72 (s, 1H), 9.05 (s, 1H), 9.87 (s, 1H), 10.35 (br, 1H), 12.92 (br, 1H); ESI-MS (m/z): 292 $[M+H]^+$.

Example 49

Preparation of 6-(8-methylquinazolin-2-ylamino)-1H-indazole-4-carboxylic acid

[Chemical Formula 96]

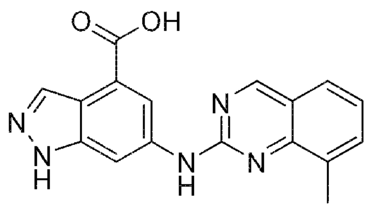

The compound (70 mg, 0.21 mmol) of Example 43 was dissolved in a 1:1 mixed solution (13 mL) of tetrahydrofuran and methanol and an aqueous 2N sodium hydroxide solution (1.0 mL) was added, and then the reaction was conducted for one hour using a microwave synthesizer (manufactured by Biotage, Ltd., 70°C). The reaction mixture was acidified by adding 2N hydrochloric acid and then extracted with ethyl acetate. The organic layer was washed with a saturated brine solution and dried over anhydrous sodium sulfate, and then the solvent was distilled off under reduced pressure. The residue was purified by silica gel chromatography (chloroform:methanol = 6:1) to obtain 15 mg of the titled compound.
$^1$H-NMR (400 MHz, DMSO-$d_6$) d: 2.72 (s, 3H), 7.32 (dd, 1H, J = 7.9, 7.2 Hz), 7.73 (dd, 1H, J = 7.1, 0.9 Hz), 7.78 (d, 1H, J = 7.8 Hz), 8.25 (s, 1H), 8.31 (s, 1H), 8.85 (s, 1H), 9.31 (d, 1H, J = 1.0 Hz), 10.21 (s, 1H), 13.11 (s, 1H); ESI-MS (m/z): 320 $[M+H]^+$, 318 $[M-H]^-$.

Example 50

Preparation of 6-(8-methylquinazolin-2-ylamino)-1H-indazole-4-carboxamide

[Chemical Formula 97]

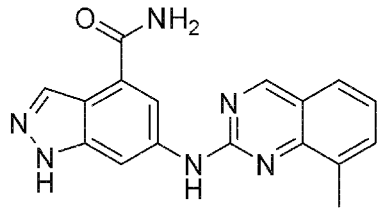

The compound (250 mg, 0.78 mmol) of Example 49 and HATU (1-[Bis-(dimethylamino)methyliumyl]-1H-1,2,3-triazolo[4,5-b]pyridine-3-oxide hexafluorophosphate) (SIGMA-ALDRICH) (445 mg, 1.17 mmol) were dissolved in DMF (10 mL), and ammonium chloride (168 mg, 3.13 mmol) and N,N-diisopropylethylamine (202 mg, 1.57 mmol) were added under ice cooling. After stirring the reaction solution at room temperature for 18 hours, the reaction mixture was diluted with ethyl acetate. The organic layer was separated, washed in turn with an aqueous 0.5 N sodium hydroxide solution and water and then dried over anhydrous sodium sulfate. The solvent was distilled off under reduced pressure, and the resulting solid was collected by filtration and then washed with cold ethyl acetate to obtain 53 mg of the titled compound. $^1$H-NMR (400 MHz, DMSO-$d_6$) d: 2.71 (s, 3H), 7.33 (t, 1H, J = 7.6 Hz), 7.46 (br, 1H), 7.6-7.85 (m, 3H), 8.00 (d, 1H, J = 1.6 Hz), 8.17 (s, 1H), 8.70 (s, 1H), 9.32 (s, 1H), 10.13 (s, 1H), 13.10 (s, 1H); ESI-MS (m/z): 319 $[M+H]^+$, 317 $[M-H]^-$.

Example 51

Preparation of ethyl 6-(8-methylquinazolin-2-ylamino)-1H-indazole-4-carbamate

[Chemical Formula 98]

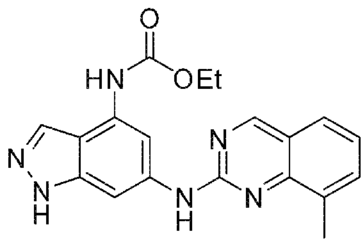

First Step

[Chemical Formula 99]

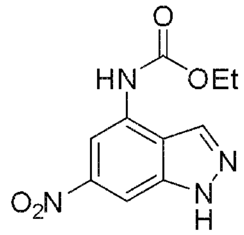

Methyl 6-nitro-1H-indazole-4-carboxylate (see Example 41) (3.02 g, 13.65 mmol) was dissolved in a THF/methanol 1:1 mixed solution (200 mL) and an aqueous 2N sodium hydroxide solution (35 mL) was added, followed by stirring at 70°C for one hour. The reaction mixture was acidified by adding 2N hydrochloric acid and then extracted with ethyl acetate. The organic layer was washed with a saturated brine solution and dried over anhydrous sodium sulfate, and then solvent was distilled off under reduced pressure to obtain 2.86 g of 6-nitro-1H-indazole-4-carboxylic acid. To an anhydrous toluene solution (250 mL) of this product, triethylamine (1.73 g, 17.06 mmol) was added under a nitrogen gas flow, and then diphenylphosphoric acid azide (4.70 g, 17.06 mmol) was added. After the reaction mixture was refluxed for one hour, ethanol (3.14 g, 68.3 mmol) was added, followed by reflux for 2 hours. After cooling to room temperature, the reaction mixture was diluted with ethyl acetate, washed in turn with an aqueous 1 N sodium hydroxide solution and water and then dried over anhydrous sodium sulfate. The solvent was distilled off under reduced pressure to obtain 3.42 g of ethyl 6-nitro-1H-indazole-4-carbamate.
$^1$H-NMR (400 MHz, DMSO-$d_6$) d: 1.32 (t, 3H, J = 7.2 Hz), 4.25 (q, 2H, J = 7.2 Hz), 8.09 (s, 1H), 8.48 (d, 1H, J = 1.6 Hz), 8.64 (s, 1H), 10.40 (s, 1H), 13.75 (br, 1H).

Second Step

[Chemical Formula 100]

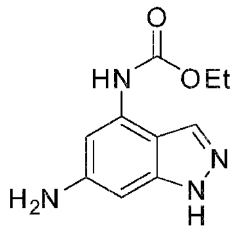

To an aqueous 80% ethanol solution (125 mL) of the product (1.30 g, 5.20 mmol) of the first step, ammonium chloride (0.139 g, 2.60 mmol) and iron (2.90 g, 52.0 mmol) were added, followed by reflux for one hour. After cooling reaction mixture to room temperature, insoluble substances were filtered through cerite and then washed in turn with ethanol and ethyl acetate. The filtrate was concentrated under reduced pressure and the resulting residue was dissolved in ethyl acetate, followed by washing with water and further drying over anhydrous sodium sulfate. The solvent was distilled off under reduced pressure and the precipitated solid was collected by filtration to obtain 0.72 g of ethyl 6-amino-1H-indazole-4-carbamate.
$^1$H-NMR (400 MHz, DMSO-$d_6$) d: 1.28 (t, 3H, J = 7.2 Hz), 4.16 (q, 2H, J = 7.2 Hz), 5.26 (br, 2H), 6.19 (s, 1H), 6.96 (s, 1H), 8.04 (s, 1H), 9.52 (s, 1H), 12.19 (br, 1H); ESI-MS (m/z): 221 $[M+H]^+$.

Third Step

40 mg of ethyl 6-(8-methylquinazolin-2-ylamino)-1H-indazole-4-carbamate was obtained by reacting and treating 2-chloro-8-methylquinazoline (see Reference Example 1) (50 mg, 0.28 mmol) and the product (62 mg, 0.28 mmol)

of the second step in the same manner as in Example 34.
$^1$H-NMR (400 MHz, DMSO-$d_6$) d: 1.31 (t, 3H, J = 7.1 Hz), 2.70 (s, 3H), 4.20 (q, 2H, J = 7.1 Hz), 7.31 (dd, 1H, J = 8.0, 6.6 Hz), 7.71 (d, 1H, J = 6.8 Hz), 7.76 (d, 1H, J = 8.0 Hz), 7.91 (s, 1H), 8.21 (d, 1H, J = 0.8 Hz), 8.35 (s, 1H), 9.28 (s, 1H), 9.75 (s, 1H), 10.01 (s, 1H), 12.88 (br, 1H); ESI-MS (m/z): 363 $[M+H]^+$.

Example 52

Preparation of 8-methyl-N-(4-amino-1H-indazol-6-yl)quinazoline-2-amine

[Chemical Formula 101]

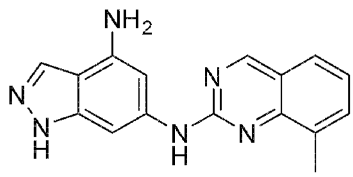

The compound (18 mg, 0.05 mmol) of Example 51 was dissolved in a 1:1 mixed solution (10 mL) of dioxane and methanol and an aqueous 2N sodium hydroxide solution (1.5 mL) was added, and then the reaction was conducted for one hour using a microwave synthesizer (manufactured by Biotage, Ltd., 120°C). The reaction mixture was extracted with ethyl acetate, and the organic layer was washed with water and then dried over anhydrous sodium sulfate. The solvent was distilled off under reduced pressure to obtain 15 mg of the titled compound.
$^1$H-NMR (400 MHz, DMSO-$d_6$) d: 2.69 (s, 3H), 5.61 (s, 2H), 6.51 (d, 1H, J = 1.6 Hz), 7.28 (dd, 1H, J = 8.0, 7.2 Hz), 7.70 (d, 1H, J = 7.2 Hz), 7.74 (d, 1H, J = 8.0 Hz), 7.91 (s, 1H), 7.98 (s, 1H), 9.25 (s, 1H), 9.69 (s, 1H), 12.50 (s, 1H); ESI-MS (m/z): 291 $[M+H]^+$.

Example 53

Preparation of 6-(8-methylquinazolin-2-ylamino)-1H-indazole-3-carboxylic acid

[Chemical Formula 102]

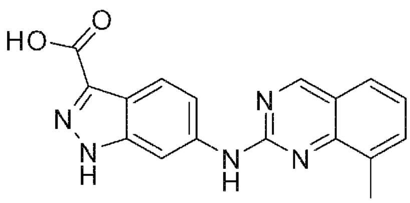

First Step

[Chemical Formula 103]

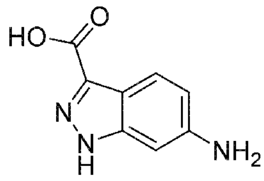

To a methanol solution (5 mL) of 6-nitro-1H-indazole-3-carboxylic acid (173 mg, 0.84 mmol), 10% palladium carbon (30 mg) was added, followed by stirring under a hydrogen gas flow at room temperature for 4 hours. The reaction mixture was filtered through cerite and the solvent was distilled off under reduced pressure to obtain 112 mg of 6-amino-1H-indazole-3-carboxylic acid.

Second Step

An n-butanol (3 mL) solution of 2-chloro-8-methylquinazoline (see Reference Example 1) (113 mg, 0.63 mmol) and the product (112 mg, 0.63 mmol) of the first step was stirred at 120°C for 3 hours. After air-cooling the reaction solution to room temperature, the solvent was distilled off under reduced pressure and the residue was purified by preparative HPLC to obtain 6 mg of 6-(8-methylquinazolin-2-ylamino)-1H-indazole-3-carboxylic acid.
$^1$H-NMR (300 MHz, DMSO-$d_6$) d (ppm): 2.6-2.8 (m, 3H), 7.2-7.4 (m, 1H), 7.5-8.0 (m, 4H), 8.8-8.9 (m, 1H), 9.2-9.4 (m, 1H), 10.1-10.2 (m, 1H); ESI-MS (m/z): 320 $[M+H]^+$.

Example 54

Preparation of N-[2-(dimethylamino)ethyl]-6-(8-methylquinazolin-2-ylamino)-1H-indazole-3-carboxamide

[Chemical Formula 104]

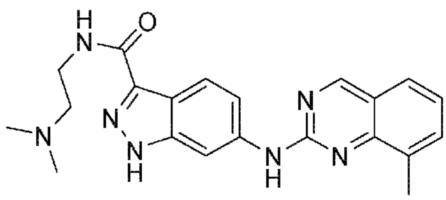

First Step

[Chemical Formula 105]

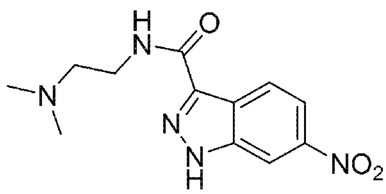

To a DMF solution (2 mL) of 6-nitro-1H-indazole-3-carboxylic acid (100 mg, 0.48 mmol), 2-dimethylaminoethylamine (0.06 mL, 0.58 mmol), triethylamine (0.1 mL, 0.73 mmol) and PyBOP™ (SIGMA-ALDRICH) (377 mg, 0.73 mmol) were added, followed by stirring at room temperature for 5 hours. The reaction mixture was diluted with water and extracted with ethyl acetate, and then the organic layer was concentrated under reduced pressure. The resulting residue was purified by silica gel chromatography to obtain 116 mg of N-[2-(dimethylamino)ethyl]-6-nitro-1H-indazole-3-carboxamide.

Second Step

To a methanol solution (5 mL) of the product (116 mg, 0.42 mmol) of the first step, 10% palladium carbon (20 mg) was added, followed by stirring under a hydrogen gas flow at room temperature for 2 hours. The reaction mixture was filtered through cerite and the solvent was distilled off under reduced pressure to obtain 86 mg of 6-amino-N-[2-(dimethylamino)ethyl]-1H-indazole-3-carboxamide.

Third Step

An n-butanol (3 mL) solution of 2-chloro-8-methylquinazoline (see Reference Example 1) (62 mg, 0.35 mmol) and the product (86 mg, 0.35 mmol) of the second step was stirred at 120°C for 3 hours. After air-cooling the reaction solution to room temperature, the solvent was distilled off under reduced pressure and the residue was purified by preparative HPLC to obtain 6 mg of N-[2-(dimethylamino)ethyl]-6-(8-methylquinazolin-2-ylamino)-1H-indazole-3-carboxamide.
$^1$H-NMR (300 MHz, $CD_3OD$) d (ppm): 2.7-3.0 (m, 9H), 3.2-3.4 (m, 2H), 3.7-3.9 (m, 2H), 7.2-7.5 (m, 2H), 7.6-7.8 (m, 2H), 8.0-8.2 (m, 1H), 8.8-9.0 (m, 1H), 9.1-9.2 (m, 1H); ESI-MS (m/z): 390 $[M+H]^+$.

Example 55

Preparation of 6-(8-methylquinazolin-2-ylamino)-N-(2-morpholinoethyl)-1H-indazole-3-carboxamide

[Chemical Formula 106]

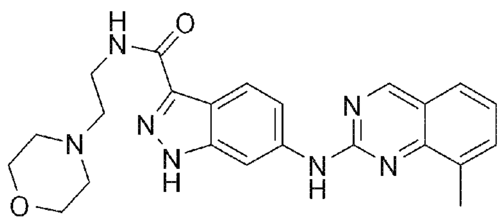

4 mg of the titled compound was obtained by reacting and treating 6-nitro-1H-indazole-3-carboxylic acid (100 mg, 0.48 mmol) and N-(2-aminoethyl)morpholine (0.08 mL, 0.58 mmol) in the same manner as in Example 54.
$^1$H-NMR (300 MHz, DMSO-$d_6$) d (ppm): 2.3-2.8 (m, 7H), 3.1-3.6 (m, 8H), 7.2-7.4 (m, 1H), 7.4-7.6 (m, 1H), 7.6-7.8 (m, 2H), 7.9-8.2 (m, 2H), 8.8-8.9 (m, 1H), 9.2-9.4 (m, 1H), 10.1-10.2 (m, 1H), 13.3-13.4 (m, 1H); ESI-MS (m/z): 432 $[M+H]^+$.

Example 56

Preparation of N-(1H-indazol-6-yl)-7-[2-(pyrrolidin-1-yl)ethylamino]-8-methylquinazoline-2-amine

[Chemical Formula 107]

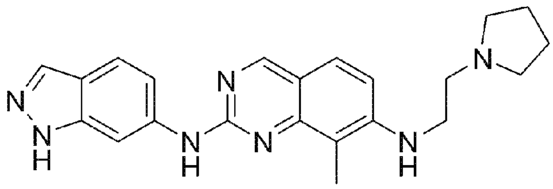

An NMP solution (0.3 mL) of the compound (50 mg, 0.17 mmol) of Example 44 and 1-(2-aminoethyl)pyrrolidine (39 mg, 0.34 mmol) was stirred at 130°C for 2 days. The reaction mixture was purified by preparative HPLC to obtain 5 mg of the titled compound.
$^1$H-NMR (400 MHz, DMSO-$d_6$) d (ppm): 1.7-1.9 (m, 2H), 1.9-2.1 (m, 2H), 2.3-2.6 (m, 7H), 3.0-3.5 (m, 4H), 6.12 (br, 1H), 6.99 (d, 1H, J = 8.8Hz), 7.40 (dd, 1H, J = 8.0, 2.0 Hz), 7.62 (d, 1H, J = 8.8 Hz), 7.66 (d, 1H, J = 8.8 Hz), 7.93 (d, 1H, J = 0.8 Hz), 8.68 (s, 1H), 8.95 (s, 1H), 9.74 (s, 1H), 12.91 (br, 1H); ESI-MS (m/z): 388 $[M+H]^+$.

Example 57

Preparation of N-(1H-indazol-6-yl)-7-(2-morpholinoethylamino)-8-methylquinazoline-2-amine

[Chemical Formula 108]

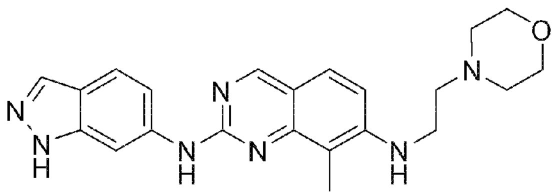

55 mg of the titled compound was obtained by reacting and treating the compound (100 mg, 0.341 mmol) of Example 44 and N-(2-aminoethyl)morpholine (88 mg, 0.682 mmol) in the same manner as in Example 56.
$^1$H-NMR (400 MHz, DMSO-$d_6$) d (ppm): 2.50 (s, 3H), 3.2-4.1 (m, 12H), 6.15 (br, 1H), 6.99 (d, 1H, J = 8.8 Hz), 7.40 (d, 1H, J = 8.7 Hz), 7.62 (d, 1H, J = 8.6 Hz), 7.67 (d, 1H, J = 8.7 Hz), 7.93 (s, 1H), 8.67 (s, 1H), 8.95 (s, 1H), 9.76 (br, 1H); ESI-MS (m/z): 404 $[M+H]^+$.

Example 58

Preparation of N-(1H-indazol-6-yl)-7-(3-morpholinopropylamino)-8-methylquinazoline-2-amine

[Chemical Formula 109]

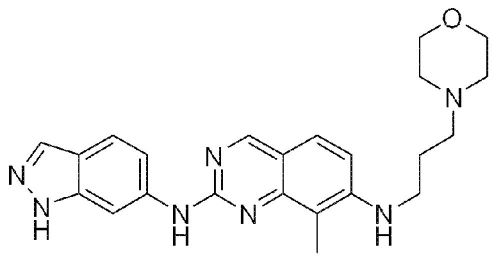

30 mg of the titled compound was obtained by reacting and treating the compound (40 mg, 0.136 mmol) of Example 44 and N-(3-aminopropyl)morpholine (59 mg, 0.408 mmol) in the same manner as in Example 56.
$^1$H-NMR (400 MHz, DMSO-$d_6$) d (ppm): 1.97 (m, 2H), 2.50 (s, 3H), 3.08-3.65 (m, 10H), 3.98 (d, 1H, J = 12.4), 6.10 (br, 1H), 6.95 (d, 1H, J = 8.8 Hz), 7.39 (d, 1H, J = 8.7 Hz), 7.62 (t, 2H, J = 8.2 Hz), 7.92 (s, 1H), 8.67 (s, 1H), 8.90 (s, 1H), 9.65 (br, 1H), 9.70 (s, 1H); ESI-MS (m/z): 418 $[M+H]^+$.

Example 59

Preparation of 6-(8-methylquinazolin-2-ylamino)-1H-indazole-3-carbonitrile

[Chemical Formula 110]

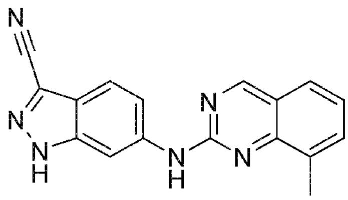

31 mg of the titled compound was obtained by reacting and treating 6-nitro-1H-indazole-3-carbonitrile (211 mg, 1.12 mmol) in the same manner as in Example 53.
$^1$H-NMR (300 MHz, DMSO-$d_6$) d (ppm): 2.6-2.8 (m, 3H), 7.2-7.8 (m, 5H), 8.8-9.0 (m, 1H), 9.2-9.4 (m, 1H), 10.2-10.4 (m, 1H); ESI-MS (m/z): 301 $[M+H]^+$.

Example 60

Preparation of 8-methyl-N-{3-[(2-morpholinoethylamino)methyl]-1H-indazol-6-yl}-quinazoline-2-amine

[Chemical Formula 111]

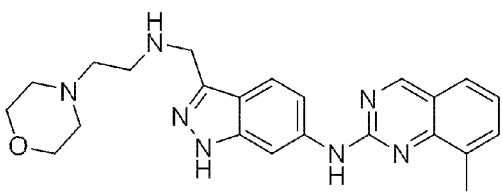

4 mg of the titled compound was obtained by reacting and treating tert-butyl 3-(bromomethyl)-6-nitro-1H-indazole-1-carboxylate (see the second step of Example 46) (200 mg, 0.56 mmol) and N-(2-aminoethyl)morpholine (0.11 mL, 0.84 mmol) in the same manner as in Example 46.
$^1$H-NMR (300 MHz, $CD_3OD$) d (ppm): 2.4-2.6 (m, 3H), 3.1-3.2 (m, 4H), 3.8-3.9 (m, 6H), 4.1-4.3 (m, 2H), 5.2-5.4 (m, 2H), 6.5-6.7 (m, 1H), 7.1-7.3 (m, 1H), 7.4-7.7 (m, 3H), 9.0-9.2 (m, 1H); ESI-MS (m/z): 418 $[M+H]^+$.

Example 61

Preparation of 8-methyl-N-{3-[(2-(dimethylamino)ethylamino)methyl]-1H-indazol-6-yl}-quinazoline-2-amine

[Chemical Formula 112]

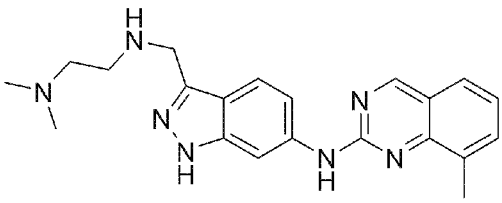

4 mg of the titled compound was obtained by reacting and treating tert-butyl 3-(bromomethyl)-6-nitro-1H-indazole-1-carboxylate (see the second step of Example 46) (200 mg, 0.56 mmol) and 2-dimethylaminoethylamine (0.092 mL, 0.84 mmol) in the same manner as in Example 46.
$^1$H-NMR (300 MHz, $CD_3OD$) d (ppm): 2.6-2.8 (m, 9H), 2.9-3.2 (m, 4H), 4.2-4.4 (m, 2H), 7.2-7.4 (m, 2H), 7.6-7.8 (m, 3H), 8.8-9.0 (m, 1H), 9.1-9.2 (m, 1H); ESI-MS (m/z): 376 $[M+H]^+$.

Example 62

Preparation of N-(1H-indazol-6-yl)-8-methyl-7-(2-morpholinoethoxy)quinazoline-2-amine

[Chemical Formula 113]

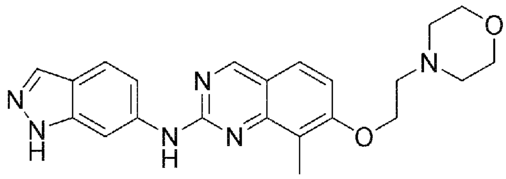

To a DMF solution (0.7 mL) of 4-(2-hydroxyethyl)morpholine (53 mg, 0.408 mmol), sodium hydride (60% w/w in oil, 98 mg, 2.44 mmol) was added, followed by stirring at 0°C for 30 minutes. To the reaction solution, the compound (40 mg, 0.136 mmol) of Example 44 was added, followed by stirring at 100°C for 24 hours. To the reaction mixture, water (1 mL) was added dropwise under ice cooling, followed by extraction with ethyl acetate. The resulting organic layer was washed with water and then dried over anhydrous sodium sulfate. The solvent was distilled off under reduced pressure and the residue was purified by preparative HPLC to obtain 9.1 mg of the titled compound.
$^1$H-NMR (400 MHz, DMSO-$d_6$) d (ppm): 2.58 (s, 3H), 3.44-3.69 (m, 8H), 4.02 (m, 2H), 4.57 (m, 2H), 7.30 (d, 1H, J = 8.9

Hz), 7.45 (d, 1H, J = 8.7 Hz) 7.64 (d, 1H, J = 8.7 Hz), 7.85 (d, 1H, J = 8.8 Hz), 7.94 (s, 1H), 8.68(s, 1H), 9.20 (s, 1H), 10.00 (s, 1H), 12.92 (br, 1H); ESI-MS (m/z): 405 $[M+H]^+$.

Example 63

Preparation of N-(1H-indazol-6-yl)-8-methyl-7-(4-methylpiperazin-1-yl)quinazoline-2-amine

[Chemical Formula 114]

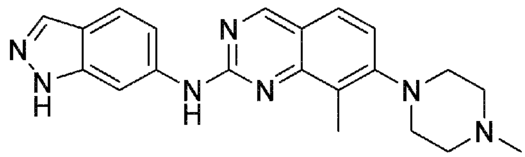

8 mg of the titled compound was obtained by reacting and treating the compound (40 mg, 0.136 mmol) of Example 44 and N-methylpiperazine (41 mg, 0.408 mmol) in the same manner as in Example 56.
$^1$H-NMR (400 MHz, DMSO-$d_6$) d (ppm): 2.63 (s, 3H), 2.92 (s, 3H), 3.0-3.6 (m, 8H), 7.24 (d, 1H, J = 8.7 Hz), 7.46 (d, 1H, J = 8.6 Hz), 7.64 (d, 1H, J = 8.7 Hz), 7.79 (d, 1H, J = 8.6 Hz), 7.94 (s, 1H), 9.21 (s, 1H), 9.62 (br, 1H), 10.03 (s, 1H); ESI-MS (m/z): 374 $[M+H]^+$.

Example 64

Preparation of N-(1H-indazol-6-yl)-8-methyl-7-[2-(pyrrolidin-1-yl)ethoxy]quinazoline-2-amine

[Chemical Formula 115]

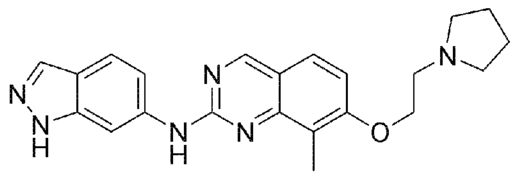

12.5 mg of the titled compound was obtained by reacting and treating 1-(2-hydroxyethyl)pyrrolidine (59 mg, 0.512 mmol) and the compound (50 mg, 0.17 mmol) of Example 44 in the same manner as in Example 62.
$^1$H-NMR (400 MHz, DMSO-$d_6$) d (ppm): 1.9-2.0 (m, 2H), 2.0-2.1 (m, 2H), 2.59 (s, 3H), 2.7-3.3 (m, 4H), 3.7-3.8 (m, 2H), 4.5-4.6 (m, 2H), 7.30 (d, 1H, J = 8.8 Hz), 7.45 (d, 1H, J = 8.8 Hz), 7.64 (d, 1H, J = 8.8 Hz), 7.85 (d, 1H, J = 8.8 Hz), 7.94 (s, 1H, J = 8.8 Hz), 8.69 (s, 1H), 9.20 (s, 1H), 10.01 (s,1H), 12.92 (br, 1H); ESI-MS (m/z): 389 $[M+H]^+$.

Example 65

Preparation of 7-[2-(dimethylamino)ethoxy]-N-(1H-indazol-6-yl)-8-methylquinazoline-2-amine

[Chemical Formula 116]

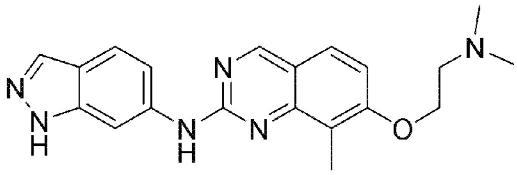

15.1 mg of the titled compound was obtained by reacting and treating N,N-dimethylethanolamine (46 mg, 0.512 mmol) and the compound (50 mg, 0.17 mmol) of Example 44 in the same manner as in Example 62.
$^1$H-NMR (400 MHz, DMSO-$d_6$) d (ppm): 2.55 (s, 3H), 2.91 (s, 6H), 3.5-3.6 (m, 2H), 4.5-4.51 (m, 2H), 7.26 (d, 1H, J = 8.9 Hz), 7.42 (d, 1H, J = 8.7 Hz) 7.61 (d, 1H, J = 8.6 Hz), 7.82 (d, 1H, J = 8.8 Hz), 7.90 (s, 1H), 8.65 (s, 1H), 9.17(s, 1H), 9.97(s, 1H), 12.89 (br, 1H); ESI-MS (m/z): 363 $[M+H]^+$.

Example 66

Preparation of 7-(2-ethoxyethoxy)-N-(1H-indazol-6-yl)-8-methylquinazoline-2-amine

[Chemical Formula 117]

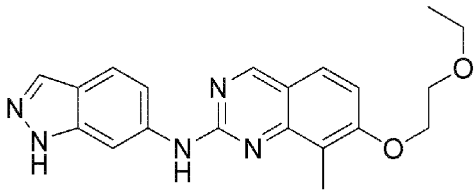

12.2 mg of the titled compound was obtained by reacting and treating 2-ethoxyethanol (46 mg, 0.512 mmol) and the compound (50 mg, 0.17 mmol) of Example 44 in the same manner as in Example 62.
$^1$H-NMR (400 MHz, DMSO-$d_6$) d (ppm): 1.12 (t, 3H, J = 7.0 Hz), 2.51 (s, 3H), 3.54 (q, 2H, J = 7.0 Hz), 3.75 (t, 2H, J = 4.4 Hz), 4.28 (t, 1H, J = 4.4 Hz), 7.23 (d, 1H, J = 9.0 Hz), 7.39 (d, 1H, J = 8.7 Hz), 7.60 (d, 1H, J = 8.7 Hz), 7.76 (d, 1H, J = 8.8 Hz), 7.89(s, 1H), 8.67(s, 1H), 9.13(s, 1H), 9.92 (s, 1H), 12.88 (br, 1H); ESI-MS (m/z): 364 $[M+H]^+$.

Example 67

Preparation of 7-(2-methoxyethylamino)-N-(1H-indazol-6-yl)-8-methylquinazoline-2-amine

[Chemical Formula 118]

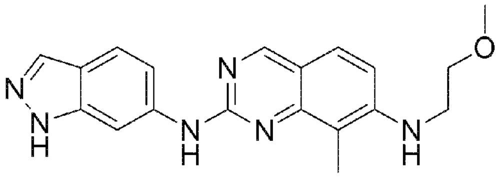

7.7 mg of the titled compound was obtained by reacting and treating the compound (100 mg, 0.341 mmol) of Example 44 and 2-methoxyethylamine (76 mg, 1.02 mmol) in the same manner as in Example 56.
$^1$H-NMR (400 MHz, DMSO-$d_6$) d (ppm): 2.54 (s, 3H), 3.2-3.6 (m, 4H), 3.30 (s, 3H), 6.97 (d, 1H, J = 9.0 Hz), 7.36 (d, 1H,

J = 8.7 Hz), 7.6-7.7 (m, 2H), 7.92 (s, 1H), 8.65 (br, 1H), 8.89 (s, 1H), 9.75 (br, 1H) 12.93 (br, 1H); ESI-MS (m/z): 349 $[M+H]^+$.

Example 68

Preparation of N-(1H-indazol-6-yl)-8-methyl-7-(pyrrolidin-1-yl)quinazoline-2-amine

[Chemical Formula 119]

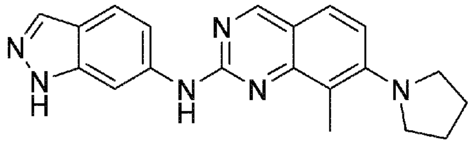

45 mg of the titled compound was obtained by reacting and treating the compound (40 mg, 0.136 mmol) of Example 44 and pyrrolidine (29 mg, 0.409 mmol) in the same manner as in Example 56.
$^1$H-NMR (400 MHz, DMSO-$d_6$) d (ppm): 1.9-2.0 (m, 4H), 2.65 (s, 3H), 3.3-3.5 (m, 4H), 7.06 (d, 1H, J = 8.8 Hz)), 7.41 (d, 1H, J = 8.5 Hz)), 7.5-7.7 (m, 2H), 7.91 (s, 1H), 8.69 (s, 1H), 8.97 (s, 1H), 9.76 (s, 1H), 12.88 (br, 1H); ESI-MS (m/z): 345 $[M+H]^+$.

Example 69

Preparation of [6-(7-methoxy-8-methylquinazolin-2-ylamino)-1H-benzo[d]imidazol-4-yl]methanol

[Chemical Formula 120]

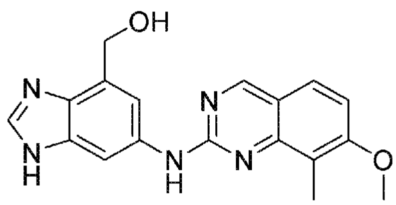

First Step

[Chemical Formula 121]

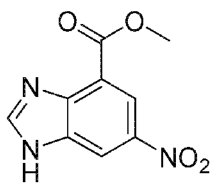

To a methanol solution (32 mL) of 6-nitrobenzo[d]imidazole-4-carboxylic acid [which can be synthesized, for example, by the method described in J. Org. Chem. 1960, 25, 942] (3.0 g, 14.5 mmol), thionyl chloride (1.58 mL) was added under a nitrogen gas flow while cooling to 0°C, and then the reaction mixture was refluxed for 24 hours. After cooling to room temperature, the solvent was distilled off under reduced pressure and water (5 mL) was added to the resulting residue, followed by extraction with ethyl acetate. The resulting organic layer was washed with an aqueous saturated sodium hydrogen carbonate solution and dried over anhydrous sodium sulfate. After the solvent was distilled off under reduced pressure, the residue was purified by silica gel chromatography (methanol:dichloromethane = 1:9) to obtain 1.7 g of 6-nitrobenzo[d]imidazole-4-methyl carboxylate.
$^1$H-NMR (400 MHz, DMSO-$d_6$) d (ppm): 4.00 (s, 3H), 8.6-8.7 (m, 2H), 8.82 (s, 1H), 13.24 (br, 1H); ESI-MS (m/z): 222 $[M+H]^+$.

Second Step

[Chemical Formula 122]

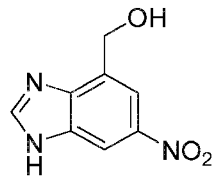

To a THF solution (5 mL) of the product (140 mg, 0.63 mmol) of the first step, a THF solution (5 mL) of lithium aluminum hydride (28.8 mg, 0.757 mmol) was added under a nitrogen gas flow while cooling to 0°C, followed by stirring at room temperature for 12 hours. An aqueous saturated ammonium chloride solution (0.5 mL) was gradually added to the reaction mixture under ice cooling, followed by extraction with ethyl acetate. The resulting organic layer was washed with a saturated brine solution and dried over anhydrous sodium sulfate. The solvent was distilled off under reduced pressure and the residue was purified by silica gel chromatography (methanol:dichloromethane = 1:9) to obtain 110 mg of 6-nitrobenzo[d]imidazole-4-methanol.
$^1$H-NMR (400 MHz, DMSO-$d_6$) d (ppm): 4.91 (d, 2H, J = 4.0 Hz), 5.66 (br, 1H), 8.14 (s, 1H), 8.41 (s, 1H), 8.52 (s, 1H); ESI-MS (m/z): 194 $[M+H]^+$.

Third Step

[Chemical Formula 123]

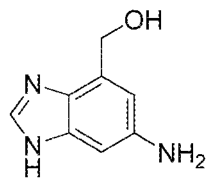

To a methanol solution (10 mL) of the product (205 mg, 1.06 mmol) of the second step, 10% palladium carbon (20 mg) was added, followed by stirring under a hydrogen gas flow at room temperature for 2 hours. The reaction mixture was filtered through cerite and the solvent was distilled off under reduced pressure, and then the residue was purified by silica gel chromatography (methanol:dichloromethane =1:9) to obtain 110 mg of 6-aminobenzo[d]imidazole-4-methanol.
$^1$H-NMR (400 MHz, DMSO-$d_6$) d (ppm): 4.6-5.2 (m, 5H), 6.48 (s, 1H), 6.59 (s, 1H), 7.78 (s, 1H), 11.79 (br, 1H); ESI-MS (m/z): 164 $[M+H]^+$.

Fourth Step

An n-butanol (1.3 mL) solution of 2-chloro-7-methoxy-8-methylquinazoline (see Reference Example 4) (104 mg, 0.5 mmol) and the product (82 mg, 0.5 mmol) of the third step was stirred at 120°C for 3 hours. The reaction solution was air-cooled to room temperature and diluted with acetone. The precipitated solid was collected by filtration and then washed with acetone to obtain 95 mg of [6-(7-methoxy-8-methylquinazolin-2-ylamino)-1H-benzo[d]imidazol-4-yl] methanol. $^1$H-NMR (400 MHz, $CD_3OD$) d (ppm): 2.59 (s, 3H), 4.01 (s, 3H), 4.9-5.1 (m, 2H), 6.9-7.0 (m, 1H), 7.2-7.3 (m, 1H), 7.7-7.8 (m, 2H), 9.0-9.1 (m, 2H), 9.20 (s, 1H); ESI-MS (m/z): 336 $[M+H]^+$.

Example 70

Preparation of [6-(8-methylquinazolin-2-ylamino)-1H-benzo[d]imidazol-4-yl]methanol

[Chemical Formula 124]

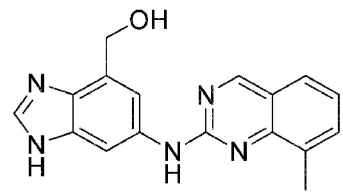

An n-butanol (1.3 mL) solution of 2-chloro-8-methylquinazoline (see Reference Example 1) (57 mg, 0.319 mmol) and 6-aminobenzo[d]imidazole-4-methanol (compound of the third step of Example 69) (52 mg, 0.319 mmol) was stirred at 120°C for 10 hours. The reaction solution was air-cooled to room temperature and diluted with acetone. The precipitated solid was collected by filtration and then washed with acetone to obtain 45 mg of the titled compound. $^1$H-NMR (400 MHz, DMSO-$d_6$) d (ppm): 2.70 (s, 3H), 4.73 (s, 1H), 4.86 (s, 2H), 5.52 (br, 1H), 6.7-6.9 (m, 1H), 7.32 (t, 1H, J = 7.4 Hz), 7.72 (d, 1H, J = 6.8 Hz), 7.78 (d, 1H, J = 7.9 Hz), 7.88 (s, 1H), 8.77 (s, 1H), 9.08 (s, 1H), 9.31 (s, 1H), 10.20 (s, 1H); ESI-MS (m/z): 306 $[M+H]^+$.

Reference Example 6

Preparation of 8-methylquinazoline-2-amine

[Chemical Formula 125]

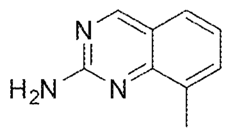

To an ethanol solution (8 mL) of 2-chloro-8-methylquinazoline (see Reference Example 1) (0.71 g, 3.97 mmol), 28% ammonia water (8 mL) was added and the reaction was conducted for 60 minutes using a microwave synthesizer (manufactured by Biotage, Ltd., 140°C). The reaction mixture was added to ice water and the precipitated solid was collected by filtration to obtain 509 mg of the titled compound.
$^1$H-NMR (400 MHz, DMSO-$d_6$) d: 2.46 (s, 3H), 6.79 (br, 1H), 7.10 (dd, 1H, J = 8.0, 6.8 Hz), 7.5-7.65 (m, 2H), 9.05 (s, 1H); ESI-MS (m/z): 160 $[M+H]^+$.

Example 71

Preparation of N-(4-methoxy-1H-indazol-6-yl)-8-methylquinazoline-2-amine

[Chemical Formula 126]

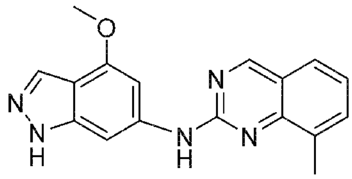

First Step

[Chemical Formula 127]

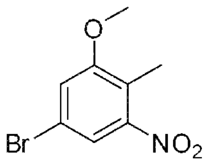

To a DMF solution (22 mL) of 5-bromo-2-methyl-3-nitrophenol [which can be synthesized, for example, by the method described in WO2005084315] (1.52 g, 6.55 mmol), sodium hydride (341 mg, 8.52 mmol) was gradually added under ice cooling, followed by stirring for 10 minutes. To the reaction solution, methyl iodide (1.40 g, 9.83 mmol) was added, followed by stirring at room temperature for 5 hours. To the reaction mixture, ice water was added, followed by extraction with ethyl acetate. The organic layer was washed in turn with water and a saturated brine solution and dried over anhydrous sodium sulfate, and then solvent was distilled off under reduced pressure. The residue was purified by silica gel chromatography (ethyl acetate:n-hexane = 1:9 to 1:6) to obtain 0.32 g of 5-bromo-1-methoxy-2-methyl-3-nitrobenzene.
$^1$H-NMR (400 MHz, $CDCl_3$) d: 2.30 (s, 3H), 3.89 (s, 3H), 7.14 (d, 1H, J = 1.6 Hz), 7.55 (d, 1H, J = 1.6 Hz).

Second Step

[Chemical Formula 128]

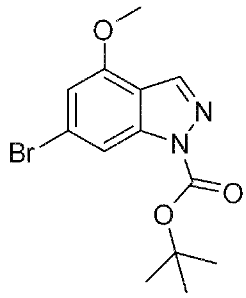

To an aqueous 80% ethanol solution (25 mL) of the product (0.30 g, 1.22 mmol) of the first step, ammonium chloride (33 mg, 0.61 mmol) and iron (0.68 g, 12.2 mmol) were added, followed by reflux for 30 minutes. The reaction mixture was filtered through cerite and washed with ethyl acetate, and then the filtrate was concentrated under reduced pressure. The residue was dissolved in ethyl acetate, washed with water and then dried over anhydrous sodium sulfate. The solvent was distilled off under reduced pressure to obtain 0.26 g of 5-bromo-3-methoxy-2-methylaniline. To a glacial acetic acid solution (20 mL) of this product, an aqueous solution (1 mL) of sodium nitrite (87 mg, 1.26 mmol) was added under ice cooling, followed by stirring at 0°C for one hour and further stirring at room temperature for 2 days. Acetic acid was distilled off under reduced pressure and the resulting residue was dissolved in ethyl acetate, washed with water and then dried over anhydrous sodium sulfate. The solvent was distilled off under reduced pressure to obtain 0.28 g of 6-bromo-4-methoxyindazole. This product was dissolved in acetonitrile (12 mL) and di-tert-butyl dicarbonate (320 mg, 1.46 mmol) and triethylamine (183 mg, 1.83 mmol) were added under ice cooling, followed by stirring at room temperature for 1.5 hours. Furthermore, di-tert-butyl dicarbonate (133 mg) and triethylamine (62 mg) were added to the reaction solution, followed by stirring at room temperature for one day. The reaction mixture was diluted with ethyl acetate, washed in turn with water and a saturated brine solution and dried over anhydrous sodium sulfate, and then the solvent was distilled off under reduced pressure. The residue was purified by silica gel chromatography (ethyl acetate: n-hexane = 1:10 to 1:8) to obtain 0.11 g of tert-butyl 6-bromo-4-methoxy-1H-indazole-1-carboxylate.
$^1$H-NMR (400 MHz, $CDCl_3$) d: 1.72 (s, 9H), 3.96 (s, 3H), 6.79 (d, 1H, J = 1.6 Hz), 7.98 (s, 1H), 8.17 (d, 1H, J = 1.6 Hz).

Third Step

[Chemical Formula 129]

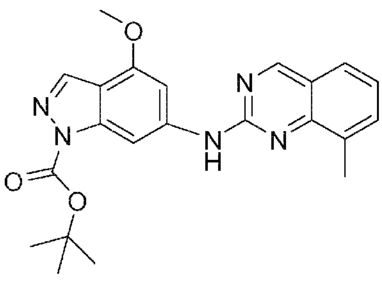

To an anhydrous dioxane solution (6 mL) of 8-methylquinazoline-2-amine (see Reference Example 6) (54 mg, 0.34 mmol) and the product (110 mg, 0.34 mmol) of the second step, Pd2 (dba) 3 (31 mg, 0.034 mmol), 4,5-bis(diphenylphosphino)-9,9-dimethylxanthen (Xantphos) (39 mg, 0.067 mmol) and cesium carbonate (219 mg, 0.67 mmol) were added, followed by reflux under a nitrogen gas flow for 10 hours. Insoluble substances were filtered through cerite and then washed with ethyl acetate. The filtrate was washed in turn with water and a saturated brine solution and dried over anhydrous sodium sulfate, and then the solvent was distilled off under reduced pressure. The residue was purified by silica gel chromatography (ethyl acetate:n-hexane = 1:10 to 1:1) to obtain 60 mg of tert-butyl-4-methoxy-6-(8-methoxyquinazolin-2-ylamino)-1H-indazole-1-carboxylate.
$^1$H-NMR (400 MHz, $CDCl_3$) d: 1.73 (s, 9H), 2.80 (s, 3H), 4.06 (s, 3H), 7.29 (dd, 1H, J = 8.0, 7.2 Hz), 7.6-7.75 (m, 4H), 8.16 (d, 1H, J = 0.8 Hz), 8.24 (br, 1H), 9.10 (s, 1H).

Fourth Step

The product (30 mg, 0.074 mmol) of third step was dissolved in dioxane (10 mL) and a 4 N hydrochloric acid dioxane solution (5 mL) was added under ice cooling, followed by stirring at room temperature for one day. The reaction solution was concentrated under reduced pressure, and the precipitated solid was collected by filtration and then washed with cold ethyl acetate. The resulting solid was purified by silica gel chromatography (chloroform:methanol = 1:0 to 50:1) to obtain 1 mg of N-(4-methoxy-1H-indazol-6-yl)-8-methylquinazoline-2-amine.
$^1$H-NMR (400 MHz, DMSO-$d_6$) d: 2.71 (s, 3H), 3.95 (s, 3H), 7.25-7.4 (m, 2H), 7.72 (d, 1H, J = 6.8 Hz), 7.77 (d, 1H, J = 8.0 Hz), 7.90 (s, 1H), 8.09 (s, 1H), 9.30 (s, 1H), 9.98 (s, 1H), 12.90 (s, 1H); ESI-MS (m/z): 306 $[M+H]^+$, 304 $[M-H]^-$.

Example 72

Preparation of 7-[2-(dimethylamino)ethylamino]-N-(1H-indazol-6-yl)-8-methylquinazoline-2-amine

[Chemical Formula 130]

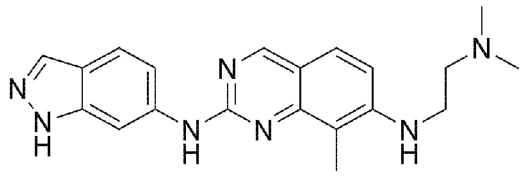

22 mg of the titled compound was obtained by reacting and treating the compound (50 mg, 0.17 mmol) of Example 44 and 2-dimethylaminoethylamine (45 mg, 0.511 mmol) in the same manner as in Example 56.
$^1$H-NMR (400 MHz, DMSO-$d_6$) d (ppm): 2.50 (s, 3H), 2.87 (s, 6H), 3.3-3.4 (m, 2H), 3.6-3.8 (m, 2H), 6.13 (br, 1H), 7.00 (d, 1H, J = 8.8 Hz), 7.38 (d, 1H, J = 8.6 Hz), 7.62 (d, 1H, J = 8.7 Hz), 7.67 (d, 1H, J = 8.7 Hz), 7.93 (s, 1H), 8.68 (s, 1H), 8.95 (s, 1H) 9.43 (br, 1H), 9.80 (s, 1H); ESI-MS (m/z): 362 $[M+H]^+$.

Example 73

Preparation of 2-[2-(1H-indazol-6-ylamino)-8-methylquinazolin-7-ylamino]ethanol

[Chemical Formula 131]

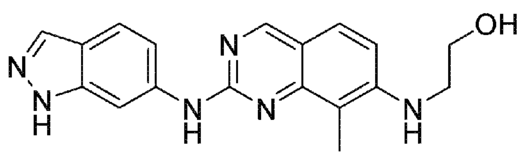

21 mg of the titled compound was obtained by reacting and treating the compound (50 mg, 0.17 mmol) of Example 44 and 2-aminoethanol (31 mg, 0.511 mmol) in the same manner as in Example 56.
$^1$H-NMR (400 MHz, DMSO-$d_6$) d (ppm): 2.32 (s, 3H), 3.3-3.4 (m, 2H), 3.6-3.7 (m, 2H), 6.98 (d, 1H, J = 9.7 Hz), 7.35 (d, 1H, J = 8.7 Hz), 7.63 (d, 2H, J = 7.9 Hz), 7.93 (s, 1H), 8.62 (br, 1H), 8.89 (s, 1H), 9.75 (bs, 1H), 12.95 (br, 1H); ESI-MS

(m/z): 335 $[M+H]^+$.

Example 74

Preparation of 7-amino-N-(1H-indazol-6-yl)-8-methylquinazoline-2-amine

[Chemical Formula 132]

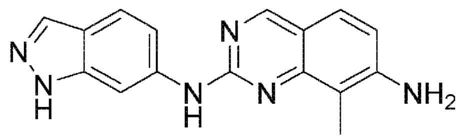

A DMF solution (2 mL) of the compound (80 mg, 0.27 mmol) of Example 44, sodium azide (17 mg, 2.70 mmol) and 18-crown 6-ether (70 mg, 0.27 mmol) was stirred at 150°C for 24 hours. The reaction mixture was purified by preparative HPLC to obtain 20 mg of the titled compound.
$^1H$-NMR (400 MHz, DMSO-$d_6$) d (ppm): 2.37 (s, 3H), 6.84 (d, 1H, J = 8.6 Hz), 7.34 (d, 1H, J = 8.7 Hz), 7.50 (d, 1H, J = 8.6 Hz), 7.63 (d, 1H, J = 8.6 Hz), 7.93 (s, 1H) 8.63 (br, 1H), 8.84(s, 1H), 9.70 (br, 1H), 12.89 (br, 1H); ESI-MS (m/z): 291 $[M+H]^+$.

Example 75

Preparation of N-(1H-indazol-6-yl)-7-[2-(piperazin-1-yl)ethylamino]-8-methylquinazoline-2-amine

[Chemical Formula 133]

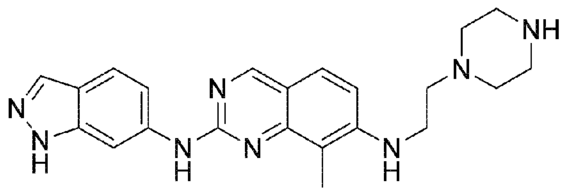

14 mg of the titled compound was obtained by reacting and treating the compound (100 mg, 0.34 mmol) of Example 44 and 1-(2-aminoethyl)piperazine (130 mg, 1.02 mmol) in the same manner as in Example 56.
$^1H$-NMR (400 MHz, DMSO-$d_6$) d (ppm): 2.45 (s, 3H), 2.7-3.6 (m, 12H), 6.95 (d, 1H, J = 8.6 Hz), 7.40 (d, 1H, J = 8.6 Hz), 7.6-7.7 (m, 2H), 7.92 (s, 1H), 8.66 (s, 1H), 8.91 (s, 1H), 9.72 (s, 1H), 12.89 (bs, 1H); ESI-MS (m/z): 403 $[M+H]^+$.

Example 76

Preparation of 7-dimethylamino-N-(1H-indazol-6-yl)-8-methylquinazoline-2-amine

[Chemical Formula 134]

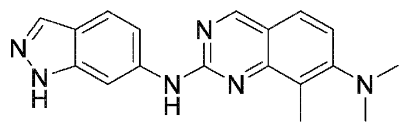

16 mg of the titled compound was obtained by reacting and treating the compound (59 mg, 0.201 mmol) of Example 44, dimethylamine hydrochloride (160 mg, 2.01 mmol) and triethylamine (203 mg, 2.01 mmol) in the same manner as in Example 56.
$^1$H-NMR (400 MHz, DMSO-$d_6$) d (ppm) : 2.62 (s, 3H), 2.87 (s, 6H), 7.22 (d, 1H, J = 8.8 Hz), 7.43 (d, 1H, J = 8.6 Hz), 7.63 (d, 1H, J = 8.7 Hz), 7.71 (d, 1H, J = 8.7 Hz), 7.92 (s, 1H), 8.72 (s, 1H), 9.11(s, 1H), 9.89(s, 1H), 12.89 (br, 1H); ESI-MS (m/z): 319 $[M+H]^+$.

Example 77

Preparation of ethyl 6-(8-methylquinazolin-2-ylamino)-1H-benzo[d]imidazol-4-ylcarbamate

[Chemical Formula 135]

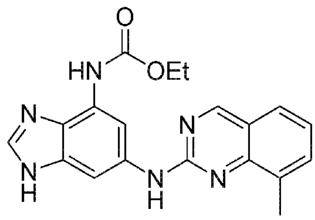

First Step

[Chemical Formula 136]

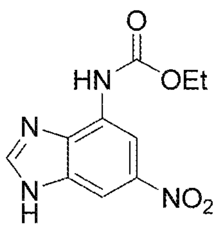

To an anhydrous ethanol solution (150 mL) of 6-nitrobenzo[d]imidazole-4-carboxylic acid [which can be synthesized, for example, by the method described in J. Org. Chem. 1960, 25, 942] (900 mg, 4.34 mmol) and triethylamine (1 mL, 5.2 mmol), diphenylphosphoric acid azide (1.4 mL, 5.0 mmol) was added, followed by reflux for 3 hours. The reaction mixture was diluted with ethyl acetate (150 mL), washed in turn with an aqueous 1 N sodium hydroxide solution

(2 × 50 mL) and water and dried over anhydrous sodium sulfate, and then the solvent was distilled off under reduced pressure to obtain 195 mg of ethyl 6-nitro-1H-benzo[d]imidazol-4-ylcarbamate.
$^1$H-NMR (400 MHz, DMSO-$d_6$) d (ppm): 1.32 (t, 3H, J = 7.0 Hz), 4.22 (q, 2H, J = 7.0 Hz), 8.23 (s, 1H), 8.50 (s, 1H), 8.53 (s, 1H), 9.69 (br, 1H); ESI-MS (m/z): 251 $[M+H]^+$.

Second Step

[Chemical Formula 137]

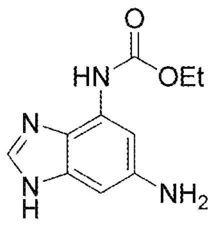

To a methanol solution (8 mL) of the product (195 mg, 0.78 mmol) of the first step, 10% palladium carbon (20 mg) was added, followed by stirring under a hydrogen gas flow at room temperature for 2 hours. The reaction mixture was filtered through cerite and the solvent was distilled off under reduced pressure to obtain 150 mg of ethyl 6-amino-1H-benzo[d]imidazol-4-ylcarbamate.
$^1$H-NMR (400 MHz, DMSO-$d_6$) d (ppm): 1.24 (t, 3H, J = 7.0 Hz), 4.14 (q, 2H, J = 7.0 Hz), 6.38 (s, 1H), 6.96 (s, 1H), 7.87 (s, 1H), 8.62 (s, 1H); ESI-MS (m/z): 221 $[M+H]^+$.

Third Step

An n-butanol (1.3 mL) solution of 2-chloro-8-methylquinazoline (see Reference Example 1) (145 mg, 0.88 mmol) and the product (150 mg, 0.68 mmol) of the second step was stirred at 120°C for 10 hours. The reaction solution was air-cooled to room temperature and diluted with ethyl acetate. The precipitated solid was collected by filtration and then washed with ethyl acetate to obtain 160 mg of ethyl 6-(8-methylquinazolin-2-ylamino)-1H-benzo[d]imidazol-4-ylcarbamate.
$^1$H-NMR (400 MHz, DMSO-$d_6$) d (ppm): 1.29 (t, 3H, J = 7.0 Hz), 2.71 (s, 3H), 4.22 (q, 2H, J = 7.0 Hz), 7.34 (t, 1H, J = 7.6 Hz), 7.74 (d, 1H, J = 7.0 Hz), 7.80 (d, 1H, J = 8.0 Hz), 8.05 (s, 1H), 8.75 (s, 1H), 9.33 (s, 1H), 9.49 (s, 1H), 10.02 (s, 1H), 10.37 (s, 1H); ESI-MS (m/z): 363 $[M+H]^+$.

Example 78

Preparation of 8-methyl-N-(4-amino-1H-benzo[d]imidazol-6-yl)quinazoline-2-amine

[Chemical Formula 138]

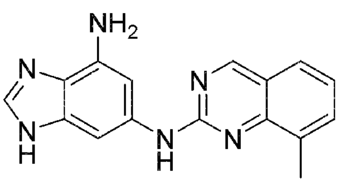

The compound (100 mg, 0.275 mmol) of Example 77 was dissolved in a 1,4-dioxane/methanol 1:1 solution (5 mL) and an aqueous 2 N sodium hydroxide solution (1 mL) was added, followed by reflux for 12 hours. The reaction mixture was extracted with ethyl acetate, and the resulting organic layer was washed with a saturated brine solution and then dried over anhydrous sodium sulfate. The solvent was distilled off under reduced pressure to obtain 65 mg of the titled compound.
$^1$H-NMR (400 MHz, DMSO-$d_6$) d (ppm): 2.66 (s, 3H), 5.14 (s, 2H), 6.75 (s, 1H), 7.24 (t, 1H, J = 7.4 Hz), 7.66 (d, 1H, J = 6.7 Hz), 7.71 (d, 1H, J = 7.9 Hz), 7.8-7.9 (m, 2H), 9.20 (s, 1H), 9.60 (s, 1H), 12.10 (br, 1H); ESI-MS (m/z): 291 $[M+H]^+$.

Example 79

Preparation of 4-amino-N-ethyl-6-(8-methylquinazolin-2-ylamino)-1H-indazole-1-carboxamide

[Chemical Formula 139]

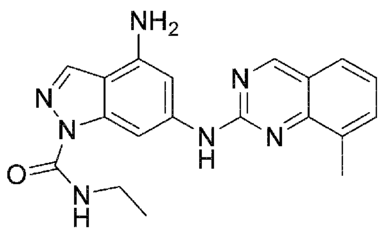

To a dimethylacetamide solution (0.5 mL) of the compound (10 mg, 0.034 mmol) of Example 52, dimethylacetamide (0.25 mL) of ethyl isocyanate (2.4 mg, 0.034 mmol) was added, followed by stirring at room temperature for one day. The reaction mixture was concentrated under reduced pressure and the resulting residue was purified by silica gel chromatography (ethyl acetate:n-hexane = 1:9 to 7:3) to obtain 5 mg of the titled compound.
$^1$H-NMR (400 MHz, DMSO-$d_6$) d: 1.18 (t, 3H, J = 7.2 Hz), 2.69 (s, 3H), 3.2-3.5 (m, 2H), 5.85 (s, 2H), 6.32 (d, 1H, J = 1.6 Hz), 7.31 (t, 1H, J = 8.0 Hz), 7.71 (d, 1H, J = 8.0 Hz), 7.76 (d, 1H, J = 8.0 Hz), 8.00 (s, 1H), 8.72 (t, 1H, 6.0 Hz), 8.92 (d, 1H, J = 1.2 Hz), 9.27 (s, 1H), 9.74 (s, 1H); ESI-MS (m/z): 362 $[M+H]^+$.

Example 80

Preparation of ethyl [6-(8-methylquinazolin-2-ylamino)-1H-indazol-4-yl]methylcarbamate

[Chemical Formula 140]

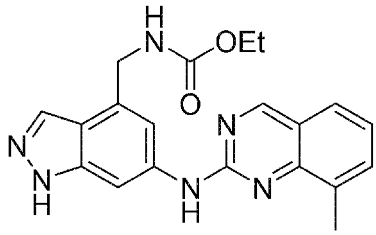

First Step

[Chemical Formula 141]

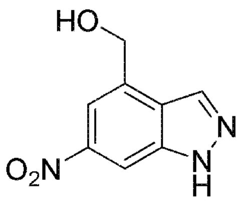

A THF suspension (20 mL) of methyl 6-nitro-1H-indazole-4-carboxylate (see Example 41) (1.19 g, 5.40 mmol) was added to a THF suspension (20 mL) of lithium aluminum hydride (205 mg, 5.40 mmol) under ice cooling, followed by stirring room temperature for 5 hours. The reaction mixture was diluted with ethyl acetate, and water and an aqueous 15% sodium hydroxide solution were sequentially added thereby terminating the reaction. The organic layer was separated, washed in turn with water and a saturated brine solution and then dried over sodium sulfate. The solvent was distilled off under reduced pressure and the resulting residue was suspended in diisopropylether, and then the solid was collected by filtration to obtain 695 mg of (6-nitro-1H-indazol-4-yl)methanol.
$^1$H-NMR (400 MHz, DMSO-$d_6$) d: 4.93 (d, 2H, J = 5.6 Hz), 5.64 (t, 1H, J = 5.7 Hz), 7.9-8.0 (m, 1H), 8.3-8.4 (m, 2H), 13.78 (br, 1H).

Second Step

[Chemical Formula 142]

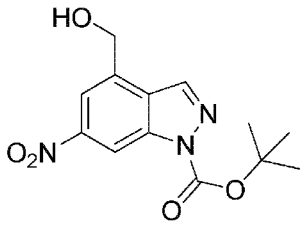

To a THF solution (5 mL) of the product (247.2 mg, 1.28 mmol) of the first step and triethylamine (168 mg, 1.66 mmol), di-tert-butyl dicarbonate (331 mg, 1.52 mmol) was added under ice cooling, followed by stirring at room temperature for 2 hours. The reaction mixture was diluted with ethyl acetate and then washed in turn with water and a saturated brine solution. The solvent was distilled off under reduced pressure and the resulting residue was purified by silica gel chromatography (chloroform:methanol = 1:0 to 25:1) to obtain 286 mg of tert-butyl 4-(hydroxymethyl)-6-nitro-1H-indazole-1-carboxylate.
$^1$H-NMR (400 MHz, $CDCl_3$) d: 1.76 (s, 9H), 2.05 (t, 1H, J = 5.7 Hz), 5.14 (d, 2H, J = 5.7 Hz), 8.1-8.2 (m, 1H), 8.46 (d, 1H, J = 1.0 Hz), 9.05 (d, 1H, J = 1.0 Hz).

Third Step

[Chemical Formula 143]

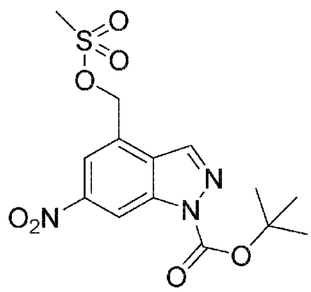

To a THF solution (5 mL) of the product (279 mg, 0.95 mmol) of the second step and triethylamine (117 mg, 1.11 mmol), methanesulfonyl chloride (331 mg, 1.515 mmol) was added under ice cooling, followed by stirring at room temperature for 40 minutes. The reaction mixture was diluted with ethyl acetate and then washed in turn with water and a saturated brine solution. The solvent was distilled off under reduced pressure and the resulting residue was purified by silica gel chromatography (chloroform:methanol = 1:0 to 25:1) to obtain 307 mg of tert-butyl 4-[(methanesulfonyloxy) methyl]-6-nitro-1H-indazole-1-carboxylate.
$^1$H-NMR (400 MHz, $CDCl_3$) d: 1.77 (s, 9H), 3.07 (s, 3H), 5.60 (s, 2H), 8.2-8.3 (m, 1H), 8.46 (d, 1H, J = 0.8 Hz), 9.18 (d, 1H, J = 1.2 Hz).

Fourth Step

[Chemical Formula 144]

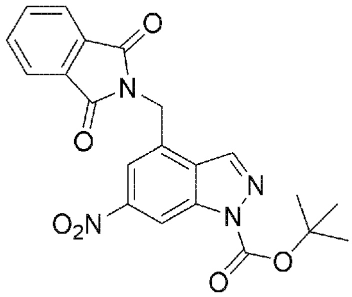

A DMF solution (1.75 mL) of the product (147 mg, 0.396 mmol) of the third step and a phthalimide potassium salt (84.3 mg, 0.455 mmol) was stirred at 60°C for one hour and half. The reaction mixture was added to water and the precipitated solid was collected by filtration to obtain 129 mg of tert-butyl 4-[(1,3-dioxoisoindolyn-2-yl)methyl]-6-nitro-1H-indazole-1-carboxylate.
$^1$H-NMR (400 MHz, $CDCl_3$) d: 1.73 (s, 9H), 5.21 (s, 2H), 7.74 (dd, 2H, J = 5,5, 3.0 Hz), 7.86 (dd, 2H, J = 2.0, 4.5 Hz), 8.29 (d, 1H, J = 2.0 Hz), 8.65 (d, 1H, J = 1.2 Hz), 9.06 (d, 1H, J = 0.8 Hz).

Fifth Step

[Chemical Formula 145]

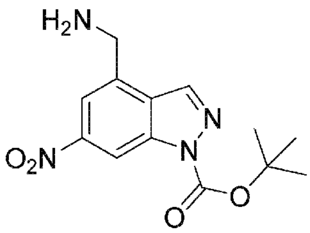

A DMF solution (0.5 mL) of the product (119 mg, 0.281 mmol) of the fourth step and hydrazine monohydrate (0.5 mL, 10.3 mmol) was stirred at 60°C for 0.5 hour. The reaction mixture was diluted with 2-butanol and then washed in turn with 2N sodium hydroxide and a saturated brine solution. The solvent was distilled off under reduced pressure to obtain 82 mg of tert-butyl 4-aminomethyl-6-nitro-1H-indazole-1-carboxylate.
$^1$H-NMR (400 MHz, DMSO-$d_6$) d: 1.6-1.8 (m, 9H), 4.12 (s, 2H), 7.9-8.0 (m, 1H), 8.3-8.4 (m, 2H), 8.94 (br, 2H).

Sixth Step

[Chemical Formula 146]

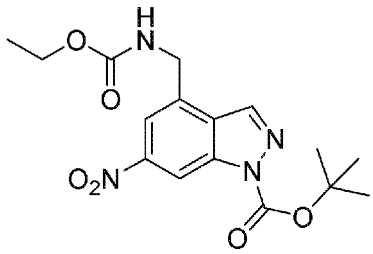

To a THF solution (3 mL) of the product (82 mg, 0.28 mmol) of the fifth step and triethylamine (85 mg, 0.837 mmol), ethyl chloroformate (56 mg, 0.52 mmol) was added under ice cooling, followed by stirring at room temperature for one hour. The reaction mixture was diluted with ethyl acetate and then washed with water and a saturated brine solution. After drying over sodium sulfate, the solvent was distilled off under reduced pressure and the resulting residue was purified by silica gel chromatography (chloroform:methanol = 1:0 to 99:1) to obtain 61 mg of tert-butyl 4-[(ethoxycarbonylamino)methyl]-6-nitro-1H-indazole-1-carboxylate.
$^1$H-NMR (400 MHz, $CDCl_3$) d: 0.7-1.0 (m, 3H), 1.2-1.4 (m, 9H), 4.21 (q, 2H, J = 7.1 Hz), 4.79 (d, 2H, J = 6.1 Hz), 5.18 (br, 1H), 7.96 (s, 1H), 8.30 (s, 1H), 8.38 (s, 1H), 10.70 (br, 1H).

Seventh Step

[Chemical Formula 147]

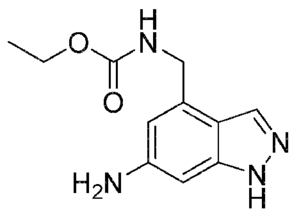

To a methanol solution (1.5 mL) of the product (0.56 g, 0.154 mmol) of the sixth step, an aqueous 70% ethanol solution (2.85 mL) of ammonium chloride (4.13 g, 0.077 mmol) and iron (86 mg, 1.54 mmol) was added, followed by reflux for 1.5 hours. After cooling the reaction mixture to room temperature, insoluble substances were filtered through cerite and washed with methanol, and then the filtrate was concentrated under reduced pressure to obtain 54.4 mg of 4-[(ethoxycarbonylamino)methyl]-6-amino-1H-indazole.
$^1$H-NMR (400 MHz, DMSO-$d_6$) d: 1.1-1.2 (m, 3H), 4.01 (q, 2H, J = 7.1 Hz), 4.32 (d, 2H, J = 6.2 Hz), 5.23 (br, 2H), 6.3-6.4 (m, 1H), 7.62 (t, 1H, J = 6.0 Hz), 7.81 (s, 1H), 12.27 (s, 1H).

Eighth Step

An n-butanol solution (1.0 mL) of 2-chloro-8-methylquinazoline (see Reference Example 1) (24 mg, 0.135 mmol) and the product (45 mg, 0.135 mmol) of the seventh step was reacted for 2 hours using a microwave synthesizer (manufactured by Biotage, Ltd., 120°C). After air-cooling the reaction solution to room temperature, the precipitated solid was collected by filtration and then washed with diisopropylether to obtain 52.6 mg of ethyl [6-(8-methylquinazolin-2-ylamino)-1H-indazol-4-yl]methylcarbamate.

$^1H$-NMR (400 MHz, DMSO-$d_6$) d (ppm): 1.1-1.3 (m, 3H), 2.71 (s, 3H), 4.04 (q, 2H, J = 7.0 Hz), 4.46 (d, 2H, J = 5.9 Hz), 7.28 (t, 1H, J = 7.6 Hz), 7.43 (s, 1H), 7.72 (d, 1H, J = 6.8 Hz), 7.77 (d, 1H, J = 7.6 Hz), 8.04 (s, 1H), 8.56 (s, 1H), 9.29 (s, 1H), 10.07 (s, 1H), 12.95 (br, 1H); ESI-MS (m/z): 377 $[M+H]^+$.

Example 81

Preparation of 2-(1H-indazol-6-yl)-8-methylquinazoline-7-acetamide

[Chemical Formula 148]

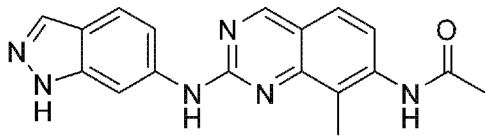

To a DMF solution (2 mL) of the compound (53 mg, 0.182 mmol) of Example 74 and triethylamine (0.030 mL, 0.219 mmol), acetic anhydride (0.021 mL, 0.219 mmol) was added, followed by stirring at room temperature for 24 hours. The solvent was distilled off under reduced pressure and the residue was purified by preparative HPLC to obtain 3 mg of the titled compound.
$^1H$-NMR (400 MHz, DMSO-$d_6$) d (ppm): 2.46 (s, 3H), 2.72 (s, 3H), 5.99 (s, 1H), 6.84 (d, 1H, J = 8.6 Hz), 7.47 (d, 1H, J = 8.7 Hz), 7.61 (d, 1H, J = 8.7 Hz), 7.73 (d, 1H, J = 8.6 Hz), 8.29 (s, 1H), 8.83 (s, 1H), 9.4-9.5 (m, 1H), 9.63 (s, 1H), 9.90 (s, 1H); ESI-MS (m/z): 333 $[M+H]^+$.

Example 82

Preparation of 8-methyl-N-(4-hydroxy-1H-indazol-6-yl)quinazoline-2-amine

[Chemical Formula 149]

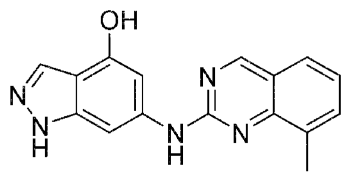

To a dichloromethane solution (30 mL) of the compound (30 mg, 0.074 mmol) of Example 71, a dichloromethane solution (1 M, 4.8 mL) of borane tribromide was added under ice cooling, followed by stirring at room temperature for 9 days. The reaction mixture was diluted with dichloromethane and an aqueous sodium thiosulfate solution was added under ice cooling, followed by extraction with 10% methanol-chloroform. The organic layer was dried over anhydrous sodium sulfate and the solvent was distilled off under reduced pressure, and then the resulting residue was purified by silica gel chromatography (chloroform:methanol = 50:1 to 10:1) to obtain 4.5 mg of the titled compound.
$^1H$-NMR (400 MHz, DMSO-$d_6$) d: 2.70 (s, 3H), 6.79 (d, 1H, J = 1.6 Hz), 7.30 (t, 1H, J = 7.6 Hz), 7.71 (d, 1H, J = 7.2 Hz), 7.76 (d, 1H, J = 7.6 Hz), 7.91 (s, 1H), 8.20 (s, 1H), 9.27 (s, 1H), 9.90 (s, 1H), 9.95 (s, 1H), 12.74 (s, 1H); ESI-MS (m/z): 292 $[M+H]^+$, 290 $[M-H]^-$.

Example 83

Preparation of N-(1H-indazol-6-yl)-8-methyl-7-morpholinoquinazoline-2-amine

[Chemical Formula 150]

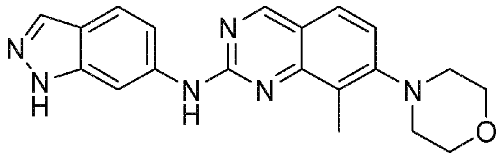

7 mg of the titled compound was obtained by reacting and treating the compound (50 mg, 0.17 mmol) of Example 44 and morpholine (45 mg, 0.51 mmol) in the same manner as in Example 56.
$^1$H-NMR (400 MHz, DMSO-$d_6$) d (ppm): 2.63 (s, 3H), 3.0-3.1 (m, 4H), 3.8-3.9 (m, 4H), 7.22 (d, 1H, J = 8.5 Hz), 7.43 (d, 1H, J = 8.8 Hz), 7.63 (d, 1H, J = 8.6 Hz), 7.76 (d, 1H, J = 8.6 Hz), 7.93 (s, 1H), 8.74 (s, 1H), 9.17 (s, 1H), 9.97(s,1H), 12.90 (br, 1H); ESI-MS (m/z): 361 $[M+H]^+$.

Example 84

Preparation of {1-[2-(1H-indazol-6-ylamino)-8-methylquinazolin-7-yl]piperidin-4-yl}methanol

[Chemical Formula 151]

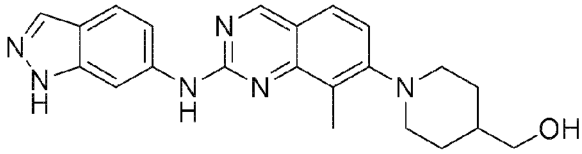

7 mg of the titled compound was obtained by reacting and treating the compound (60 mg, 0.20 mmol) of Example 44 and 4-piperidine methanol (71 mg, 0.61 mmol) in the same manner as in Example 56.
$^1$H-NMR (400 MHz, DMSO-$d_6$) d (ppm): 1.3-1.6 (m, 3H), 1.83 (d, 2H, J = 11.7 Hz), 2.60 (s, 3H), 2.79 (t, 2H, J = 11.4 Hz), 3.32 (d, 2H, J = 11.4 Hz), 3.36 (d, 2H, J = 5.3 Hz), 7.22 (d, 1H, J = 8.6 Hz), 7.41 (d, 1H, J = 8.7 Hz), 7.63 (d, 1H, J = 8.7 Hz), 7.72 (d, 1H, J = 8.6 Hz), 7.93 (s, 1H), 8.76 (s, 1H), 9.14 (s, 1H), 9.95 (s, 1H), 12.90 (br, 1H); ESI-MS (m/z): 389 $[M+H]^+$.

Example 85

Preparation of N-(1H-indazol-6-yl)-8-methyl-7-(piperidin-1-yl)quinazoline-2-amine

[Chemical Formula 152]

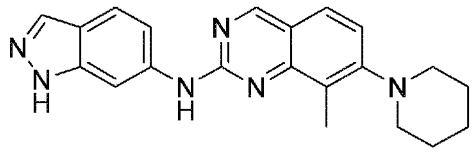

7 mg of the titled compound was obtained by reacting and treating the compound (50 mg, 0.17 mmol) of Example 44 and piperidine (44 mg, 0.51 mmol) in the same manner as in Example 56.
$^1$H-NMR (400 MHz, DMSO-$d_6$) d (ppm): 1.5-1.6 (m, 2H), 1.7-1.8 (m, 4H), 2.61 (s, 3H), 3.0-3.1 (m, 4H), 7.20 (d, 1H, J = 8.7 Hz), 7.41 (d, 1H, J = 8.4 Hz), 7.62 (d, 1H, J = 8.7 Hz), 7.72 (d, 1H, J = 8.6 Hz), 7.92 (s, 1H), 8.77(s, 1H), 9.13(s, 1H), 9.92(s, 1H), 12.88 (br, 1H); ESI-MS (m/z): 359 $[M+H]^+$.

Example 86

Preparation of [2-methyl-6-(8-methylquinazolin-2-ylamino)-1H-benzo[d]imidazol-4-yl]methanol

[Chemical Formula 153]

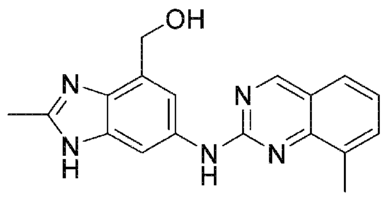

First Step

[Chemical Formula 154]

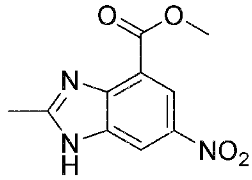

To a methanol solution (32 mL) of 2-methyl-6-nitrobenzo[d]imidazole-4-carboxylic acid [which can be synthesized, for example, by the method described in J. Org. Chem. 1960, 25, 942] (300 mg, 1.357 mmol), concentrated sulfuric acid (0.6 mL) was added, followed by reflux for 12 hours. The solvent was distilled off under reduced pressure and an aqueous 10% sodium hydrogen carbonate solution (25 mL) was added to the resulting residue, followed by extraction with ethyl acetate. The resulting organic layer was washed with water and dried over anhydrous sodium sulfate, and then the solvent was distilled off under reduced pressure. The resulting residue was suspended in diethylether and then filtered to obtain 170 mg of 2-methyl-6-nitrobenzo[d]imidazole-4-methyl carboxylate.
$^1$H-NMR (400 MHz, DMSO-$d_6$) d (ppm): 2.73 (s, 3H), 4.05 (s, 3H), 8.75 (d, 1H, J = 1.6 Hz), 8.78 (d, 1H, J = 1.6 Hz); ESI-MS (m/z): 236 $[M+H]^+$.

Second Step

[Chemical Formula 155]

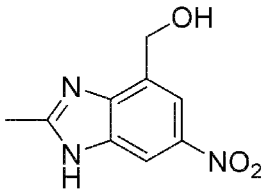

To lithium aluminum hydride (133 mg), a THF solution (8 mL) of the product (165 mg, 0.702 mmol) of the first step was added under a nitrogen gas flow while cooling to 0°C, followed by stirring at room temperature for 12 hours. The aqueous saturated ammonium chloride solution (0.5 mL) was gradually added to the reaction mixture under ice cooling, followed by extraction with ethyl acetate. The resulting organic layer was dried over anhydrous sodium sulfate and the solvent was distilled off under reduced pressure to obtain 65 mg of 2-methyl-6-nitrobenzo[d]imidazole-4-methanol.
ESI-MS (m/z): 208 $[M+H]^+$.

Third Step

[Chemical Formula 156]

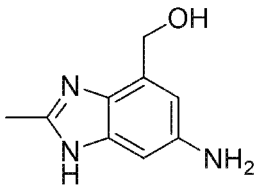

To a methanol solution (8 mL) of the product (63 mg, 0.304 mmol) of the second step, 10% palladium carbon (15 mg) was added, followed by stirring under a hydrogen gas flow at room temperature for 2 hours and the reaction mixture was filtered through cerite, and then the solvent was distilled off under reduced pressure to obtain 42 mg of 2-methyl-6-aminobenzo[d]imidazole-4-methanol.
ESI-MS (m/z): 178 $[M+H]^+$.

Fourth Step

An n-butanol (1 mL) solution of 2-chloro-8-methylquinazoline (see Reference Example 1) (52 mg, 0.294 mmol) and the product (40 mg, 0.226 mmol) of the third step was stirred at 120°C for 10 hours. The reaction solution was air-cooled to room temperature and diluted with ethyl acetate, and then the precipitated solid was collected by filtration. The resulting solid was purified by preparative HPLC to obtain 7 mg of [2-methyl-6-(8-methylquinazolin-2-ylamino)-1H-benzo [d]imidazol-4-yl]methanol.
$^1$H-NMR (400 MHz, $CD_3OD$) d (ppm): 2.68 (s, 3H), 2.75 (s, 3H), 4.96 (s, 2H), 7.30 (t, 1H, J = 7.6 Hz), 7.70 (m, 3H), 8.82 (s, 1H), 9.15 (s, 1H); ESI-MS (m/z): 320 $[M+H]^+$.

Example 87

Preparation of ethyl 2-methyl-6-(8-methylquinazolin-2-ylamino)-1H-benzo[d]imidazol-4-ylcarbamate

[Chemical Formula 157]

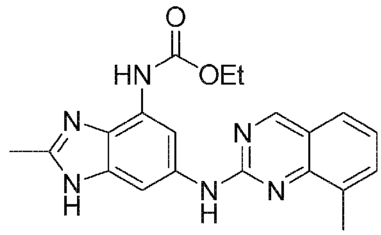

First Step

[Chemical Formula 158]

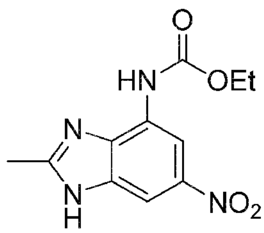

To a toluene solution (50 mL) of 2-methyl-6-nitrobenzo[d]imidazole-4-carboxylic acid [which can be synthesized, for example, by the method described in J. Org. Chem. 1960, 25, 942] (280 mg, 0.789 mmol) and triethylamine (0.22 mL, 0.98 mmol), diphenylphosphoric acid azide (0.36 mL, 0.98 mmol) was added, followed by reflux for one hour. After cooling to room temperature, ethanol (0.4 mL) was added, followed by reflux for 2 hours. The solvent was distilled off under reduced pressure and the resulting residue was dissolved in an aqueous 1N sodium hydroxide solution (30 mL), followed by extraction with ethyl acetate. The organic layer was washed with water and dried over anhydrous sodium sulfate, and then the solvent was distilled off under reduced pressure. The resulting residue was purified by silica gel chromatography (methanol:dichloromethane = 1:50) to obtain 45 mg of ethyl 2-methyl-6-nitro-1H-benzo[d]imidazol-4-ylcarbamate.
ESI-MS (m/z): 265 $[M+H]^+$.

Second Step

[Chemical Formula 159]

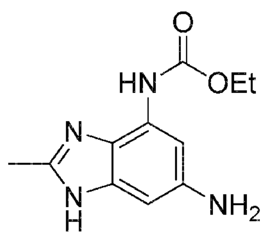

To a methanol solution (5 mL) of the product (42 mg, 0.16 mmol) of the first step, 10% palladium carbon (10 mg) was added under a hydrogen gas flow, followed by stirring at room temperature for 2 hours. The reaction mixture was filtered through cerite and the solvent was distilled off under reduced pressure to obtain 23 mg of ethyl 2-methyl-6-amino-1H-benzo[d]imidazol-4-ylcarbamate.
ESI-MS (m/z): 235 $[M+H]^+$.

Third Step

An n-butanol (1 mL) solution of 2-chloro-8-methylquinazoline (see Reference Example 1) (22 mg, 0.127 mmol) and the product (23 mg, 0.098 mmol) of the second step was stirred at 120°C for 10 hours. The reaction solution was air-cooled to room temperature and diluted with ethyl acetate. The precipitated solid was collected by filtration and then washed with ethyl acetate to obtain 20 mg of ethyl 2-methyl-6-(8-methylquinazolin-2-ylamino)-1H-benzo[d]imidazol-4-ylcarbamate.
$^1$H-NMR (400 MHz, DMSO-$d_6$) d (ppm): 1.29 (t, 3H, J = 6.8 Hz), 2.71 (s, 3H), 2.77 (s, 3H), 4.21 (q, 2H, J = 6.8 Hz), 7.34 (t, 1H, J = 7.4 Hz), 7.7-7.9 (m, 3H), 8.68 (s, 1H), 9.33 (s, 1H), 9.80 (br, 1H), 10.30 (s, 1H); ESI-MS (m/z): 377 $[M+H]^+$.

Example 88

Preparation of 8-methyl-N-(2-methyl-4-amino-1H-benzo[d]imidazol-6-yl)quinazoline-2-amine

[Chemical Formula 160]

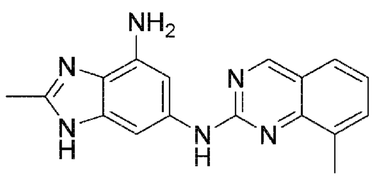

The compound (13 mg, 0.035 mmol) of Example 87 was dissolved in a 1,4-dioxane/methanol 1:1 solution (1 mL) and an aqueous 2N sodium hydroxide solution (0.3 mL) was added, followed by reflux for 12 hours. The reaction mixture was extracted with ethyl acetate, and the resulting organic layer was washed with a saturated brine solution and then dried over anhydrous sodium sulfate. The solvent was distilled off under reduced pressure to obtain 8 mg of the titled compound.
$^1$H-NMR (400 MHz, DMSO-$d_6$) d (ppm): 2.43 (s, 3H), 2.65 (s, 3H), 5.01 (br, 2H), 6.71 (s, 1H), 7.23 (t, 1H, J = 7.6 Hz), 7.65 (d, 1H, J = 6.9 Hz), 7.70 (d, 1H, J = 8.0 Hz), 7.83 (s, 1H), 9.19 (s, 1H), 9.53 (s, 1H). 11.9 (br, 1H); ESI-MS (m/z): 305 $[M+H]^+$.

Example 89

Preparation of 7-methylamino-N-(1H-indazol-6-yl)-8-methylquinazoline-2-amine

[Chemical Formula 161]

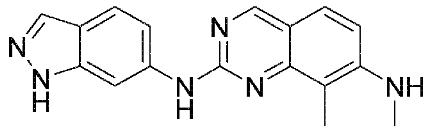

28 mg of the titled compound was obtained by reacting and treating the compound (60 mg, 0.205 mmol) of Example 44, methylamine hydrochloride (138 mg, 2.05 mmol) and triethylamine (0.28 mL, 2.05 mmol) in the same

manner as in Example 56.
$^1H$ -NMR (400 MHz, DMSO-$d_6$) d (ppm): 2.40 (s, 3H), 2.94 (s, 3H), 6.92 (d, 1H, J = 8.8 Hz), 7.34 (d, 1H, J = 8.6 Hz), 7.64 (d, 1H, J = 8.7 Hz), 7.66 (d, 1H, J = 9.0 Hz), 7.94 (s, 1H), 8.62 (br, 1H), 8.91 (s, 1H), 9.79 (br, 1H), 12.94 (br, 1H); ESI-MS (m/z): 305 $[M+H]^+$.

Example 90

Preparation of 2-{1-[2-(1H-indazol-6-ylamino)-8-methylquinazolin-7-yl]piperidin-4-yl}ethanol

[Chemical Formula 162]

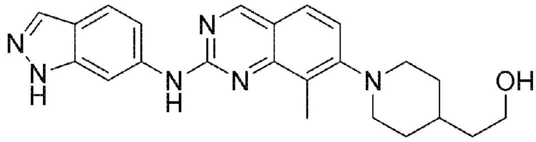

32 mg of the titled compound was obtained by reacting and treating the compound (70 mg, 0.239 mmol) of Example 44 and 1-(2-hydroxyethyl)piperidine (93 mg, 0.741 mmol) in the same manner as in Example 56.
$^1H$-NMR (400 MHz, DMSO-$d_6$) d (ppm): 2.66 (s, 3H), 3.2-3. 6 (m, 9H), 3.6-3.7 (m, 2H), 3.8-3.9 (m, 2H), 7.24 (d, 1H, J = 8.7 Hz), 7.46 (d, 1H, J = 8.8 Hz), 7.64 (d, 1H, J = 8.7 Hz), 7.79 (d, 1H, J = 8.6 Hz), 7.94 (s, 1H), 8.69 (s, 1H), 9.20 (s, 1H), 9.62 (br, 1H), 10.00 (s, 1H); ESI-MS (m/z): 404 $[M+H]^+$.

Example 91

Preparation of [6-(8-methylquinazolin-2-ylamino)-1H-indol-4-yl]methanol

[Chemical Formula 163]

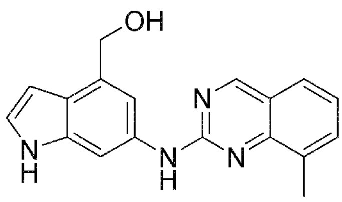

First Step

[Chemical Formula 164]

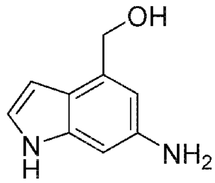

To a THF solution (15 mL) of 6-aminoindole-4-methyl carboxylate hydrochloride (0.9 g, 4.66 mmol), lithium aluminum hydride (300 mg, 7.89 mmol) was added while cooling to 0°C, followed by stirring at 0 to 5°C for 2 hours. An aqueous saturated ammonium chloride solution (1.5 mL) was gradually added to reaction mixture under ice cooling and the solvent was distilled off under reduced pressure. To the resulting residue, water (15 mL) was added, followed by extraction with ethyl acetate. The resulting organic layer was washed with a saturated brine solution and dried over anhydrous sodium sulfate, and then the solvent was distilled off under reduced pressure to obtain 110 mg of (6-amino-1H-indol-4-yl)methanol.
$^1$H-NMR (400 MHz, DMSO-$d_6$) d (ppm): 4.8-5.0 (m, 2H), 6.23 (s, 1H), 6.43 (s, 2H), 6.7-6.8 (m, 1H), 6.92 (s, 1H), 7.15 (d, 1H, J = 6.6 Hz), 10.40 (s, 1H).

Second Step

An n-butanol (2 mL) solution of 2-chloro-8-methylquinazoline (see Reference Example 1) (142 mg, 0.797 mmol), the compound (105 mg, 0.648 mmol) of the first step and triethylamine (100 mg, 1.0 mmol) was stirred at 100°C for 6 hours. The solvent was distilled off under reduced pressure and the residue was purified by preparative HPLC to obtain 18 mg of [6-(8-methylquinazolin-2-ylamino)-1H-indol-4-yl]methanol.
$^1$H-NMR (400 MHz, DMSO-$d_6$) d (ppm): 4.74 (d, 2H, J = 5.6 Hz), 5.07 (t, 1H, J = 5.6 Hz), 6.42 (s, 1H), 7.20 (d, 1H, J = 2.2 Hz), 7.25 (t, 1H, J = 7.4 Hz), 7.51 (s, 1H), 7.66 (d, 1H, J = 6.9 Hz), 7.71 (d, 1H, J = 7.9 Hz), 8.35 (s, 1H), 9.21 (s, 1H), 9.72 (s, 1H), 11.00 (s, 1H); ESI-MS (m/z): 305 $[M+H]^+$.

Example 92

Preparation of 1-{4-[2-(1H-indazol-6-ylamino)-8-methylquinazolin-7-yl]piperazin-1-yl}ethanone

[Chemical Formula 165]

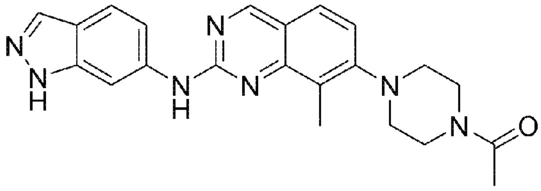

8 mg of the titled compound was obtained by reacting and treating the compound (55 mg, 0.188 mmol) of Example 44 and 1-acetylpiperazine (96 mg, 0.75 mmol) in the same manner as in Example 56.
$^1$H-NMR (400 MHz, DMSO-$d_6$) d (ppm): 2.07 (s, 3H), 2.65 (s, 3H), 2.9-3.1 (m, 4H), 3.6-3.7 (m, 4H), 7.20 (d, 1H, J = 8.8 Hz), 7.43 (d, 1H, J = 8.5 Hz), 7.63 (d, 1H, J = 8.7 Hz), 7.76 (d, 1H, J = 8.6 Hz), 7.93 (s, 1H), 8.69 (s, 1H), 9.17 (s, 1H), 9.97 (s, 1H), 12.91 (br, 1H); ESI-MS (m/z): 402 $[M+H]^+$.

Example 93

Preparation of N-(3-methyl-1H-indazol-6-yl)-7-fluoro-8-methylquinazoline-2-amine

[Chemical Formula 166]

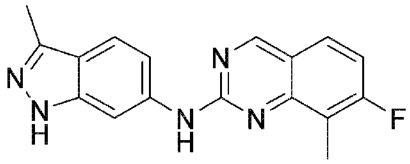

An n-butanol solution (0.8 mL) of 2-chloro-7-fluoro-8-methylquinazoline (see Reference Example 3) (79 mg, 0.40 mmol) and 3-methyl-1H-indazole-6-amine (see Example 34) (59 mg, 0.40 mmol) was reacted using a microwave synthesizer (manufactured by Biotage, Ltd., 130°C) for 45 minutes. The reaction solution was air-cooled to room temperature, and the precipitated solid was collected by filtration and then washed with ethanol to obtain 93 mg of the titled compound.
$^1$H-NMR (400 MHz, DMSO-$d_6$) d (ppm): 2.46 (s, 3H), 2.58 (d, 3H, J = 2.0 Hz), 7.28 (t, 1H, J = 9.2 Hz), 7.41 (dd, 1H, J = 8.5, 2.0 Hz), 7.60 (d, 1H, J = 8.4 Hz), 7.87 (dd, 1H, J = 8.8, 6.4 Hz), 8.61 (s, 1H), 9.30 (s, 1H), 10.16 (s, 1H), 12.61 (br, 1H); ESI-MS (m/z): 308 $[M+H]^+$.

Example 94

Preparation of {1-[2-(3-methyl-1H-indazol-6-ylamino)-8-methylquinazolin-7-yl]piperidin-4-yl}methanol

[Chemical Formula 167]

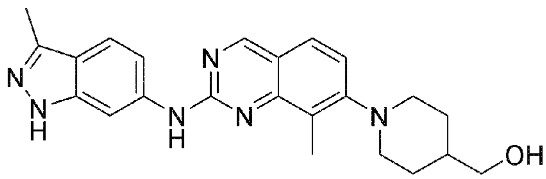

An NMP solution (0.5 mL) of the compound (21.5 mg, 0.07 mmol) of Example 93 and 4-piperidine methanol (202 mg, 1.75 mmol) was reacted for 3 hours using microwave synthesizer (manufactured by Biotage, Ltd., 220°C). The reaction solution was diluted with water, and the precipitated solid was collected by filtration and then washed with ethanol. The resulting solid was purified by silica gel chromatography (chloroform:methanol = 1:0 to 3:1) to obtain 3 mg of the titled compound.
$^1$H-NMR (400 MHz, DMSO-$d_6$) d (ppm): 1.3-1.5 (m, 2H), 1.5-1.6 (m, 1H), 1.8-1.9 (m, 2H), 2.44 (s, 3H), 2.59 (s, 3H), 2.7-2.9 (m, 2H), 3.2-3.5 (m, 4H), 4.53 (t, 1H, J = 5.2 Hz), 7.20 (d, 1H, J = 8.8 Hz), 7.39 (dd, 1H, J = 8.4, 1.6 Hz), 7.56 (d, 1H, J = 8.8 Hz), 7.71 (d, 1H, J = 8.8 Hz), 8.67 (s, 1H), 9.13 (s, 1H), 9.89 (s, 1H), 12.46 (br, 1H); ESI-MS (m/z): 403 $[M+H]^+$.

Example 95

Preparation of N-(4-methyl-1H-indazol-6-yl)-7-fluoro-8-methylquinazoline-2-amine

[Chemical Formula 168]

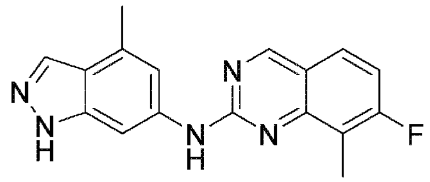

100 mg of the titled compound was obtained by reacting and treating 2-chloro-7-fluoro-8-methylquinazoline (see Reference Example 3) (79 mg, 0.40 mmol) and 4-methyl-1H-indazole-6-amine (see Example 37) (59 mg, 0.40 mmol) in the same manner as in Example 93.
$^1$H-NMR (400 MHz, DMSO-$d_6$) d (ppm): 2.53 (s, 3H), 2.59 (d, 3H, J = 2.0 Hz), 7.26 (d, 1H, J = 9.2 Hz), 7.29 (d, 1H, J = 5.2 Hz), 7.87 (dd, 1H, J = 8.8, 6.4 Hz), 7.99 (s, 1H), 8.48 (s, 1H), 9.29 (s, 1H), 10.07 (s, 1H), 12.91 (s, 1H); ESI-MS (m/z): 308 $[M+H]^+$.

Example 96

Preparation of N-(3-methyl-1H-indazol-6-yl)-7-methoxy-8-methylquinazoline-2-amine

[Chemical Formula 169]

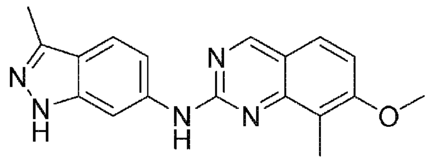

39.3 mg of the titled compound was obtained by reacting and treating 2-chloro-7-methoxy-8-methylquinazoline (see Reference Example 4) (50 mg, 0.24 mmol) and 3-methyl-1H-indazole-6-amine (see Example 34) (35.3 mg, 0.24 mmol) in the same manner as in Example 93.
$^1$H-NMR (400 MHz, DMSO-$d_6$) d (ppm): 2.46 (s, 3H), 2.53 (s, 3H), 3.99 (s, 3H), 7.28 (d, 1H, J = 9.2 Hz), 7.41 (dd, 1H, J = 8.8, 1.6 Hz), 7.59 (d, 1H, J = 8.4 Hz), 7.83 (d, 1H, J = 8.8 Hz), 8.63 (s, 1H), 9.17 (s, 1H), 9.98 (s, 1H), 12.66 (br, 1H); ESI-MS (m/z): 320 $[M+H]^+$.

Example 97

Preparation of N-(4-methyl-1H-indazol-6-yl)-7-methoxy-8-methylquinazoline-2-amine

[Chemical Formula 170]

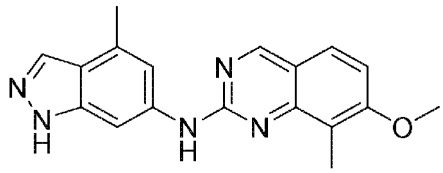

47 mg of the titled compound was obtained by reacting and treating 2-chloro-7-methoxy-8-methylquinazoline (see Reference Example 4) (50 mg, 0.24 mmol) and 4-methyl-1H-indazole-6-amine (see Example 37) (35.3 mg, 0.24 mmol) in the same manner as in Example 93.
$^1$H-NMR (400 MHz, DMSO-$d_6$) d (ppm): 2.52 (s, 3H), 2.54 (s, 3H), 3.99 (s, 3H), 7.2-7.3 (m, 2H), 7.82 (d, 1H, J = 8.8 Hz), 7.99 (d, 1H, J = 1.2 Hz), 8.51 (s, 1H), 9.17 (s, 1H), 9.92 (s, 1H), 12.94 (br, 1H); ESI-MS (m/z): 320 $[M+H]^+$.

Example 98

Preparation of 1-(2-hydroxyethyl)-3-[6-(8-methylquinazolin-2-ylamino)-1H-indazol-4-yl]urea

[Chemical Formula 171]

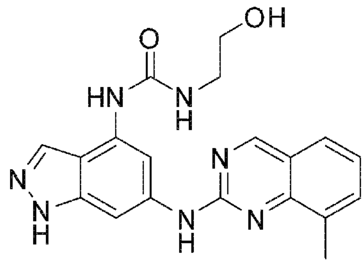

To an NMP solution (0.75 mL) of the compound (28 mg, 0.077 mmol) of Example 77, 2-aminoethanol (24 mg, 0.39 mmol) was added and the reaction was conducted for 60 minutes using a microwave synthesizer (manufactured by Biotage, Ltd., 180°C). The reaction mixture was air-cooled to room temperature and water was added, and then the precipitated solid was collected by filtration to obtain 3 mg of the titled compound.
$^1$H-NMR (400 MHz, DMSO-$d_6$) d: 2.69 (s, 3H), 3.1-3.3 (m, 2H), 3.4-3.6 (m, 2H), 4.82 (t, 1H, J = 2.4 Hz), 6.45-6.55 (m, 1H), 7.29 (t, 1H, J = 7.6 Hz), 7.70 (d, 1H, J = 6.0 Hz), 7.75 (d, 1H, J = 7.6 Hz), 7.94 (d, 1H, J = 1.6 Hz), 7.98 (s, 1H), 8.29 (s, 1H), 8.67 (s, 1H), 9.26 (s, 1H), 9.89 (s, 1H), 12.85 (s, 1H); ESI-MS (m/z): 378 $[M+H]^+$, 376 $[M-H]^-$.

Example 99

Preparation of 7-fluoro-N-(4-amino-1H-indazol-6-yl)-8-methylquinazoline-2-amine

[Chemical Formula 172]

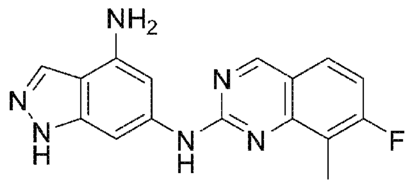

First Step

[Chemical Formula 173]

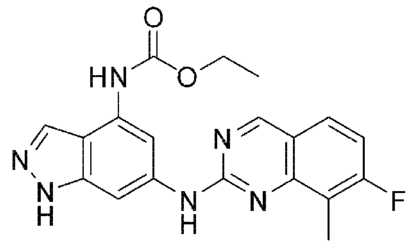

180 mg of ethyl 6-(7-fluoro-8-methylquinazolin-2-ylamino)-1H-indazol-4-ylcarbamate was obtained as a yellow solid by reacting and treating 2-chloro-7-fluoro-8-methylquinazoline (see Reference Example 3) (150 mg, 0.76 mmol) and ethyl 6-amino-1H-indazole-4-carbamate (see Example 51) (168 mg, 0.67 mmol) in the same manner as in Example 34.
$^1$H-NMR (400 MHz, DMSO-$d_6$) d: 1.31 (t, 3H, J = 7.2 Hz), 2.58 (d, 3H, J = 2.0 Hz), 4.20 (q, 2H, J = 7.2 Hz), 7.26 (t, 1H, J = 9.2 Hz), 7.86 (dd, 1H, J = 8.8, 6.4 Hz), 8.22 (d, 1H, J = 1.6 Hz), 8.27 (s, 1H), 9.28 (s, 1H), 9.77 (s, 1H), 10.10 (s, 1H), 12.88 (br, 1H); ESI-MS (m/z): 381 $[M+H]^+$.

Second Step

To a dioxane solution (15 mL) of the compound (120 mg, 0.32 mmol) of the first step, an aqueous 2N sodium hydroxide solution (3.0 mL) was added and the reaction was conducted for one hour using a microwave synthesizer (manufactured by Biotage, Ltd., 120°C). To the reaction solution, an aqueous 2N sodium hydroxide solution 1.5 mL was added and the reaction was conducted for 1.5 hours using a microwave synthesizer. The reaction mixture was diluted with ethyl acetate, washed with water and then dried over anhydrous sodium sulfate. The solvent was distilled off under reduced pressure and ice water was added to the resulting residue, and then the precipitated solid was collected by filtration to obtain 90 mg of 7-fluoro-N-(4-amino-1H-indazol-6-yl)-8-methylquinazoline-2-amine.
$^1$H-NMR (400 MHz, DMSO-$d_6$) d: 2.57 (d, 3H, J = 2.0 Hz), 5.64 (s, 2H), 6.51 (d, 1H, J = 1.2 Hz), 7.23 (dd, 1H, J = 9.6 Hz), 7.7-7.9 (m, 2H), 7.98 (s, 1H), 9.24 (s, 1H), 9.79 (s, 1H), 12.52 (s, 1H); ESI-MS (m/z): 309 $[M+H]^+$.

Example 100

Preparation of 2-[2-(4-amino-1H-indazol-6-ylamino)-8-methylquinazolin-7-ylamino]ethanol

[Chemical Formula 174]

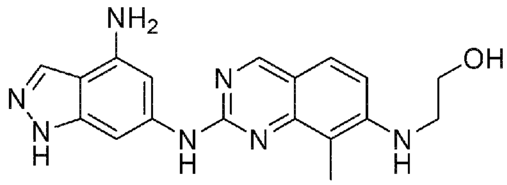

To an NMP solution of the compound (20 mg, 0.066 mmol) of Example 99, 2-aminoethanol (26 mg, 0.43 mmol) was added and the reaction was conducted for 60 minutes using a microwave synthesizer (manufactured by Biotage, Ltd., 180°C). To the reaction solution, 2-aminoethanol (20 mg) was added, followed by stirring at 140°C for 3 days. After cooling to room temperature, the reaction mixture was diluted with ethyl acetate, washed with water and then dried over anhydrous sodium sulfate. The solvent was distilled off under reduced pressure. The resulting residue was purified by silica gel chromatography (chloroform:methanol = 1:0 to 6:1) to obtain 3 mg of the titled compound.
$^1$H-NMR (400 MHz, DMSO-$d_6$) d: 2.42 (s, 3H), 3.3-3.45 (m, 2H), 3.62 (q, 2H, J = 5.6 Hz), 4.84 (t, 1H, J = 5.6 Hz), 5.56 (s, 2H), 5.74 (t, 1H, J = 5.8 Hz), 6.43 (d, 1H, J = 1.6 Hz), 6.90 (d, 1H, J = 9.2 Hz), 7.55 (d, 1H, J = 8.8 Hz), 7.89 (s, 1H), 7.95 (s, 1H), 8.82 (s, 1H), 9.26 (s, 1H), 12.47 (s, 1H); ESI-MS (m/z): 378 $[M+H]^+$, 376 $[M-H]^-$.

Example 101

Preparation of {1-[2-(4-amino-1H-indazol-6-ylamino)-8-methylquinazolin-7-yl]piperidin-4-yl}methanol

[Chemical Formula 175]

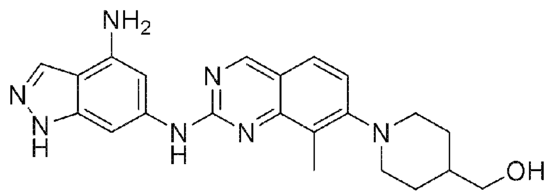

To an NMP solution of the compound (40 mg, 0.13 mmol) of Example 99, 4-piperidine methanol (75 mg, 0.65 mmol) was added and the reaction was conducted for 180 minutes using a microwave synthesizer (manufactured by Biotage, Ltd., 210°C). To the reaction solution, 4-piperidine methanol (30 mg) was added, followed by stirring at 140°C for one day. The reaction mixture was diluted with ethyl acetate, washed with water and then dried over anhydrous sodium sulfate. The solvent was distilled off under reduced pressure. The resulting residue was purified by silica gel chromatography (chloroform:methanol = 1:0 to 6:1) to obtain 10 mg of the titled compound.
$^1$H-NMR (400 MHz, DMSO-$d_6$) d: 1.3-1.45 (m, 2H), 1.45-1.6 (m, 1H), 1.75-1.9 (m, 2H), 2.57 (s, 3H), 2.7-2.85 (m, 2H), 3.25-3.4 (m, 4H), 4.52 (t, 1H, J = 5.3 Hz), 5.58 (s, 1H), 6.50 (d, 1H, J = 1.2 Hz), 7.17 (d, 1H, J = 8.8 Hz), 7.68 (d, 1H, J = 8.8 Hz), 7.91 (s, 1H), 7.96 (d, 1H, J = 1.2 Hz), 9.07 (s, 1H), 9.53 (s, 1H), 12.46 (s, 1H); ESI-MS (m/z): 404 $[M+H]^+$, 402 $[M-H]^-$.

Example 102

Preparation of N-(4-amino-1H-indazol-6-yl)-7-methoxy-8-methylquinazoline-2-amine

[Chemical Formula 176]

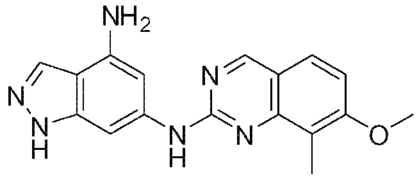

First Step

[Chemical Formula 177]

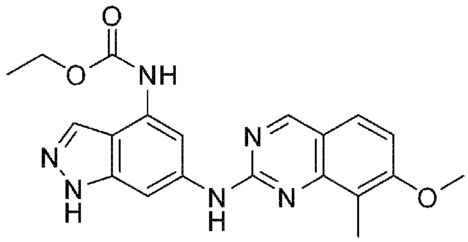

60 mg of ethyl 6-(7-methoxy-8-methylquinazolin-2-ylamino)-1H-indazol-4-ylcarbamate was obtained as a yellow solid by reacting and treating 2-chloro-7-methoxy-8-methylquinazoline (see Reference Example 4) (50 mg, 0.24 mmol) and ethyl 6-amino-1H-indazole-4-carbamate (see Example 51) (53 mg, 0.24 mmol) in the same manner as in Example 34.
$^1$H-NMR (400 MHz, DMSO-$d_6$) d: 1.31 (t, 3H, J = 7.2 Hz), 2.4-2.6 (m, 3H), 3.98 (s, 3H), 4.0-4.5 (m, 2H), 7.28 (d, 1H, J = 8.8 Hz), 7.83 (d, 1H, J = 8.8 Hz), 7.87 (s, 1H), 8.22 (d, 1H, J = 0.8 Hz), 8.31 (s, 1H), 9.17 (s, 1H), 9.76 (s, 1H), 9.98 (br, 1H), 12.94 (br, 1H); ESI-MS (m/z): 393 $[M+H]^+$.

Second Step

To a 1:1 solution (10 mL) of dioxane and methanol of the compound (60 mg, 0.15 mmol) of the first step, an aqueous 2N sodium hydroxide solution (1.5 mL) was added and the reaction was conducted for one hour using a microwave synthesizer (manufactured by Biotage, Ltd., 120°C). The reaction mixture was diluted with ethyl acetate, washed with water and then dried over anhydrous sodium sulfate. The solvent was distilled off under reduced pressure to obtain 12 mg of N-(4-amino-1H-indazol-6-yl)-7-methoxy-8-methylquinazoline-2-amine.
$^1$H-NMR (400 MHz, DMSO-$d_6$) d: 2.3-2.6 (m, 3H), 3.97 (s, 3H), 5.59 (s, 2H), 6.47 (d, 1H, J = 1.6 Hz), 7.23 (d, 1H, J = 8.8 Hz), 7.78 (d, 1H, J = 8.8 Hz), 7.90 (s, 1H), 7.96 (s, 1H), 9.11 (s, 1H), 9.56 (s, 1H), 12.49 (s, 1H); ESI-MS (m/z): 321 $[M+H]^+$, 319 $[M-H]^-$.

Example 103

Preparation of N-(3-methyl-1H-indazol-6-yl)-7-amino-8-methylquinazoline-2-amine

[Chemical Formula 178]

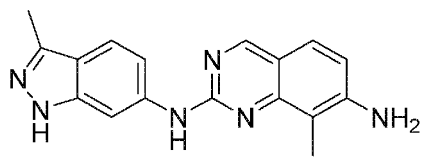

A DMF solution (1 mL) of the compound (40 mg, 0.13 mmol) of Example 93, sodium azide (85 mg, 1.3 mmol) and 18-crown 6-ether (34.4 mg, 0.13 mmol) was stirred at 130°C for 12 hours. To the reaction mixture, ice water was added and the filtrate obtained by filtration with cerite was extracted with ethyl acetate. The resulting organic layer was washed in turn with water and a saturated brine solution and then dried over sodium sulfate. The solvent was distilled off under reduced pressure and the residue was purified by silica gel chromatography (chloroform:methanol = 1:0 to 3: 1) to obtain 20 mg of the titled compound.
$^1$H-NMR (400 MHz, DMSO-$d_6$) d (ppm): 2.39 (s, 3H), 2.44 (s, 3H), 5.98 (s, 2H), 6.81 (d, 1H, J = 8.8 Hz), 7.34 (dd, 1H, J = 8.8, 1.6 Hz), 7.45 (d, 1H, J = 8.4 Hz), 7.53 (d, 1H, J = 8.8 Hz), 8.62 (d, 1H, J = 1.2 Hz), 8.80 (s, 1H), 9.58 (s, 1H), 12.43 (s, 1H); ESI-MS (m/z): 305 $[M+H]^+$.

Example 104

Preparation of {1-[2-(4-methyl-1H-indazol-6-ylamino)-8-methylquinazolin-7-yl]piperidin-4-yl}methanol

[Chemical Formula 179]

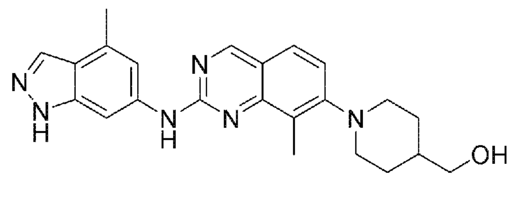

10.2 mg of the titled compound was obtained by reacting and treating the compound (30.7 mg, 0.1 mmol) of Example 95 and 4-piperidine methanol (288 mg, 2.5 mmol) in the same manner as in Example 94.
$^1$H-NMR (400 MHz, DMSO-$d_6$) d (ppm): 1.3-1.5 (m, 2H), 1.5-1.6 (m, 1H), 1.8-1.9 (m, 2H), 2.48 (s, 3H), 2.60 (s, 3H), 2.7-2.9 (m, 2H), 3.3-3.4 (m, 4H), 4.53 (t, 1H, J = 5.2 Hz), 7.20 (d, 1H, J = 8.4 Hz), 7.29 (s, 1H), 7.71 (d, 1H, J = 8.4 Hz), 7.96 (s, 1H), 8.52 (s, 1H), 9.12 (s, 1H), 9.81 (s, 1H), 12.84 (br, 1H); ESI-MS (m/z): 403 $[M+H]^+$.

Example 105

Preparation of N-(4-methyl-1H-indazol-6-yl)-7-amino-8-methylquinazoline-2-amine

[Chemical Formula 180]

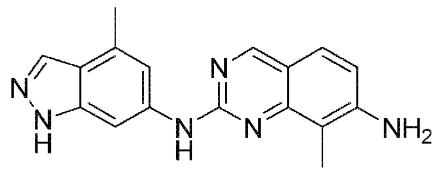

15.6 mg of the titled compound was obtained by reacting and treating the compound (40 mg, 0.13 mmol) of Example 95, sodium azide (85 mg, 1.3 mmol) and 18-crown 6-ether (34.4 mg, 0.13 mmol) in the same manner as in Example 103.
$^1$H-NMR (400 MHz, DMSO-$d_6$) d (ppm): 2.39 (s, 3H), 2.48 (s, 3H), 5.98 (s, 2H), 6.81 (d, 1H, J = 8.8 Hz), 7.22 (s, 1H), 7.44 (d, 1H, J = 8.8 Hz), 7.94 (s, 1H), 8.50 (s, 1H), 8.80 (s, 1H), 9.52 (s, 1H), 12.82 (s, 1H); ESI-MS (m/z): 305 $[M+H]^+$.

Example 106

Preparation of N-(1H-indazol-6-yl)-8-methyl-7-(piperazin-1-yl)-quinazoline-2-amine

[Chemical Formula 181]

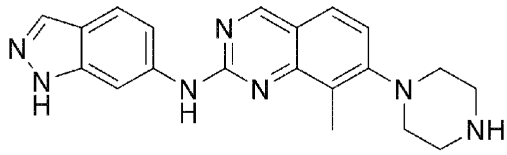

8 mg of the titled compound was obtained by reacting and treating the compound (50 mg, 0.170 mmol) of Example 44 and piperazine (58 mg, 0.682 mmol)in the same manner as in Example 56.
$^1$H-NMR (400 MHz, DMSO-$d_6$) d (ppm): 2.63 (s, 3H), 3.2-3.3 (m, 4H), 3.3-3.4 (m, 4H), 7.24 (d, 1H, J = 8.7 Hz), 7.46 (d, 1H, J = 9.1 Hz), 7.64 (d, 1H, J = 8.7 Hz), 7.79 (d, 1H J = 8.6 Hz), 7.94 (s, 1H), 8.70 (s, 1H), 8.83 (br, 1H), 9.21 (s, 1H), 10.03 (s, 1H), 12.93 (br, 1H); ESI-MS (m/z): 360 $[M+H]^+$.

Example 107

Preparation of 1-(2-(1H-indazol-6-ylamino)-8-methylquinazolin-7-yl)-3-ethylurea

[Chemical Formula 182]

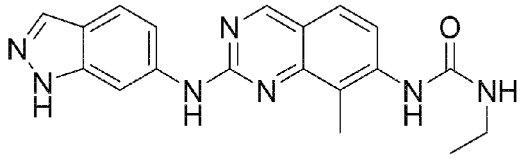

To a DMF solution (2 mL) of the compound (60 mg, 0.206 mmol) of Example 74, ethyl isocyanate (0.018 mL, 0.206 mmol) was added, followed by stirring at room temperature for 24 hours. The solvent was distilled off under reduced pressure and the residue was purified by preparative HPLC to obtain 6 mg of the titled compound.
$^1$H-NMR (400 MHz, DMSO-$d_6$) d (ppm): 1.15 (t, 3H, J = 7.1 Hz), 2.43 (s, 3H), 3.34-3.41 (m, 2H), 6.85 (d, 1H, J = 8.5 Hz), 7.22 (s, 1H), 7.48 (d, 1H, J = 8.6 Hz), 7.57 (d, 1H, J = 8.5 Hz), 7.70 (d, 1H, J = 8.6 Hz), 8.20 (s, 1H), 8.27 (t, 1H, J = 5.8 Hz), 8.84 (s, 1H), 9.43 (s, 1H), 9.86 (br, 1H); ESI-MS (m/z): 362 $[M+H]^+$.

Example 108

Preparation of 2-[2-(3-methyl-1H-indazol-6-ylamino)-8-methylquinazolin-7-ylamino]ethanol

[Chemical Formula 183]

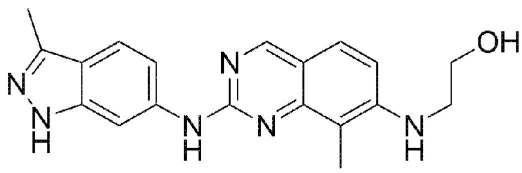

7.2 mg of the titled compound was obtained by reacting and treating the compound (10 mg, 0.033 mmol) of Example 93 and 2-aminoethanol (40 mg, 0.651 mmol) in the same manner as in Example 94.
$^1$H-NMR (400 MHz, DMSO-$d_6$) d (ppm): 2.43 (s, 3H), 2.49 (s, 3H), 3.3-3.4 (m, 2H), 3.5-3.6 (m, 2H), 4.83 (t, 1H, J = 5.6 Hz), 5.79 (t, 1H, J = 5.6 Hz), 6.93 (d, 1H, J = 8.8 Hz), 7.36 (dd, 1H, J = 8.4, 1.6 Hz), 7.54 (d, 1H, J = 8.8 Hz), 7.58 (d, 1H, J = 8.8 Hz), 8.59 (d, 1H, J = 1.2 Hz), 8.89 (s, 1H), 9.62 (s, 1H), 12.44 (s, 1H); ESI-MS (m/z): 349 $[M+H]^+$.

Example 109

Preparation of 2-[2-(4-methyl-1H-indazol-6-ylamino)-8-methylquinazolin-7-ylamino]ethanol

[Chemical Formula 184]

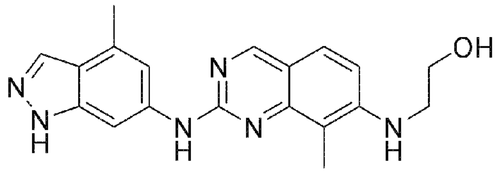

23.1 mg of the titled compound was obtained by reacting and treating the compound (30.7 mg, 0.1 mmol) of Example 95 and 2-aminoethanol (611 mg, 10 mmol) in the same manner as in Example 94.
$^1$H-NMR (400 MHz, DMSO-$d_6$) d (ppm): 2.44 (s, 3H), 2.50 (s, 3H), 3.3-3.5 (m, 2H), 3.5-3.7 (m, 2H), 4.85 (t, 1H, J = 5.6 Hz), 5.80 (t, 1H, J = 5.6 Hz), 6.93 (d, 1H, J = 8.8 Hz), 7.22 (s, 1H), 7.59 (d, 1H, J = 8.8 Hz), 7.95 (s, 1H), 8.50 (s, 1H), 8.86 (s, 1H), 9.55 (s, 1H), 12.85 (s, 1H); ESI-MS (m/z): 349 $[M+H]^+$.

Example 110

Preparation of N-(4-methyl-1H-indazol-6-yl)-7-hydroxy-8-methylquinazoline-2-amine

[Chemical Formula 185]

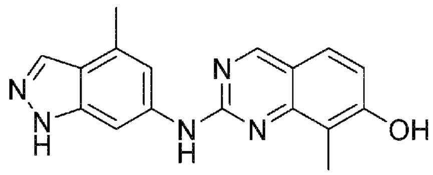

To a chloroform solution (1 mL) of the compound (10 mg, 0.031 mmol) of Example 97, borane tribromide (98%, 0.05 mL) was added, followed by stirring under a nitrogen gas flow for 4 hours. To the reaction mixture, ice water was added and the filtrate obtained by filtration with cerite was extracted with chloroform. The resulting organic layer was washed in turn with water and a saturated brine solution and then dried over sodium sulfate. The solvent was distilled off under reduced pressure, the residue was purified by silica gel chromatography (chloroform:methanol = 1:0 to 3:1) to obtain 0.6 mg of the titled compound.
$^1$H-NMR (400 MHz, $CD_3OD$) d (ppm): 2.58 (s, 3H), 2.59 (s, 3H), 6.95 (d, 1H, J = 8.8 Hz), 7.07 (s, 1H), 7.54 (d, 1H, J = 8.8 Hz), 7.97 (s, 1H), 8.64 (s, 1H), 8.89 (s, 1H); ESI-MS (m/z): 306 $[M+H]^+$.

Example 111

Preparation of 7-diethylamino-N-(1H-indazol-6-yl)-8-methylquinazoline-2-amine

[Chemical Formula 186]

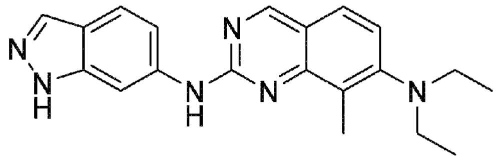

To an acetonitrile solution (0.86 mL) of the compound (60 mg, 0.206 mmol) of Example 74 and acetaldehyde (0.023 mL, 0.412 mmol), acetic acid (0.022 mL, 0.412 mmol) and sodium cyanoborohydride (40 mg, 0.663 mmol) were added at 0°C, followed by stirring under a nitrogen gas flow at room temperature. After 12 hours, acetaldehyde (0.023 mL, 0.412 mmol) and acetic acid (0.022 mL, 0.412 mmol) were added, followed by stirring for 12 hours. The reaction mixture was concentrated under reduced pressure to obtain a mixture of 7-diethylamino-N-(1H-indazol-6-yl)-8-methylquinazoline-2-amine and 7-ethylamino-N-(1H-indazol-6-yl)-8-methylquinazoline-2-amine. The resulting mixture was purified by preparative HPLC to obtain 3.0 mg of the titled compound (TLC: Rf = 0.8, ethyl acetate).
$^1$H-NMR (400 MHz, DMSO-$d_6$) d (ppm): 1.02 (t, 6H, J = 6.9 Hz), 1.2-1.3 (m, 4H), 2.63 (s, 3H), 7.42 (d, 1H, J = 8.5 Hz), 7.6-7.8 (m, 3H), 7.93 (s, 1H), 8.73 (s, 1H), 9.14 (br, 1H), 9.92 (br, 1H), 12.91(br, 1H); ESI-MS (m/z): 347 $[M+H]^+$.

Example 112

Preparation of 7-ethylamino-N-(1H-indazol-6-yl)-8-methylquinazoline-2-amine

[Chemical Formula 187]

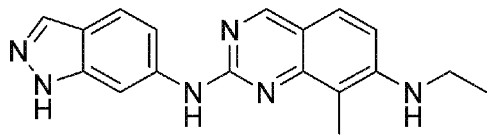

The reaction mixture of Example 111 was purified by preparative HPLC to obtain 3.1 mg of the titled compound (TLC: Rf = 0.6, ethyl acetate).
$^1$H-NMR (400 MHz, DMSO-$d_6$) d (ppm): 1.24 (t, 3H, J = 6.96 Hz), 2.42 (s, 3H), 3.34-3.35 (m, 2H), 6.93 (t, 1H, J = 8.2 Hz), 7.37 (d, 1H, J = 8.64 Hz), 7.61 (d, 2H, J = 8.44 Hz), 7.91 (s, 1H), 8.66 (s, 1H), 8.87 (s, 1H), 9.67 (s, 1H), 12.89 (bs, 1H); ESI-MS (m/z): 319 $[M+H]^+$.

Reference Example 7

Preparation of methyl 2-chloro-8-methylquinazoline-7-carboxylate

First Step

[Chemical Formula 188]

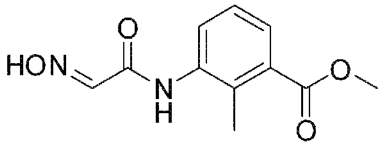

A 2 N hydrochloric acid solution (4 mL) of methyl 3-amino-2-methylbenzoate (2 g, 12.12 mmol) was added to an aqueous solution (80 mL) of 2,2,2-trichloro-1,1-ethanediol (2.39 g, 14.48 mmol), hydroxyl amine hydrochloride (2.99 g, 43.02 mmol) and sodium sulfate (48 g, 337.1 mmol), followed by stirring at 55°C for 18 hours. The reaction mixture was cooled to room temperature and the precipitated solid was collected by filtration. The resulting solid was dissolved in ethyl acetate and dried over sodium sulfate, and then the solvent was distilled off under reduced pressure. The residue was purified by silica gel chromatography (ethyl acetate:n-hexane = 2:3) to obtain 1.25 g of methyl 3-[2-(hydroxyimino) acetamide]-2-methylbenzoate.
$^1$H-NMR (400 MHz, DMSO-$d_6$) d (ppm): 2.31 (s, 3H), 3.83 (s, 3H), 7.31 (t, 1H, J = 7.8 Hz), 7.57 (d, 1H, J = 8.0 Hz), 7.60 (d, 1H, J = 7.8 Hz), 7.66 (s, 1H), 9.74 (s, 1H), 12.21 (s, 1H); ESI-MS (m/z): 237 $[M+H]^+$.

Second Step

[Chemical Formula 189]

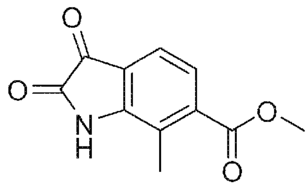

The product (5.8 g, 24.57 mmol) of the first step was gradually added to methanesulfonic acid (24 mL) heated to 80°C in advance, followed by stirring at 80°C for 2 hours. The reaction mixture was added to ice water and extracted with ethyl acetate, and then the organic layer was washed with a saturated brine solution. The resulting organic layer was dried over sodium sulfate and the solvent was distilled off under reduced pressure to obtain 4.5 g of methyl 7-methyl-2,3-dioxoindoline-6-carboxylate.
ESI-MS (m/z): 220 $[M+H]^+$.

Third Step

[Chemical Formula 190]

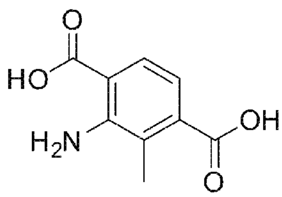

The product (4.5 g, 21 mmol) of the second step was suspended in an aqueous 1.5 N sodium hydroxide solution (100 mL) and 30% hydrogen peroxide water (2.46 mL) was added dropwise, followed by stirring at 23°C for 12 hours. The reaction solution was acidified by adding concentrated hydrochloric acid and then extracted with ethyl acetate. The organic layer was dried over sodium sulfate and the solvent was distilled off under reduced pressure to obtain 3.23 g of 2-amino-3-methylterephthalic acid.
ESI-MS (m/z): 196 $[M+H]^+$.

Fourth Step

[Chemical Formula 191]

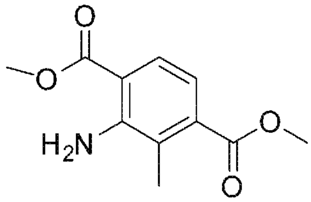

To a chloroform solution (55 mL) of the product (3.7 g, 18.97 mmol) of the third step, thionyl chloride (2.46 mL) and a catalytic amount of DMF (2 drops) were added, followed by reflux for 3 hours. To the reaction solution, methanol (20 mL) was added, followed by reflux for 10 hours. The reaction mixture was concentrated under reduced pressure and water (70 mL) was added to the resulting residue, followed by extraction with ethyl acetate. The resulting organic layer was dried over sodium sulfate and the solvent was distilled off under reduced pressure. The residue was purified by silica gel chromatography (ethyl acetate:n-hexane = 3:7) to obtain 1.95 g of dimethyl 2-amino-3-methyl terephthalate.
$^1$H-NMR (400 MHz, DMSO-$d_6$) d (ppm): 2.19 (s, 3H), 3.82 (s, 3H), 3.83 (s, 3H), 6.74 (br, 2H), 6.79 (d, 1H, J = 8.4 Hz), 7.68 (d, 1H, J = 8.4 Hz); ESI-MS (m/z): 224 $[M+H]^+$.

Fifth Step

[Chemical Formula 192]

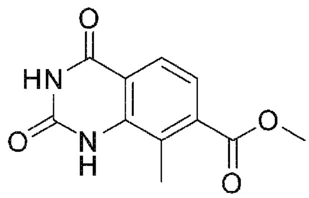

An acetic acid solution (21.3 mL) of the product (1.95 g, 8.74 mmol) of the fourth step was heated to 60°C and an aqueous solution (3 mL) of potassium cyanate (1.43 g, 17.65 mmol) was added. To the solid thus produced, acetic acid (4.42 mL) was added to give a suspension, followed by stirring at 75°C. After 10 hours, potassium cyanate (0.7 g) was added to the reaction solution, followed by stirring at 70°C for 35 hours. The reaction mixture was cooled to 0°C and the precipitated solid was collected by filtration, followed by washing with water and further air-drying. The resulting solid was suspended in methanol (8 mL) and then refluxed for one hour. After cooling to room temperature, the solid was collected by filtration and then washed with cold methanol to obtain 1.4 g of methyl 8-methyl-2,4-dioxo-1,2,3,4-tetrahydroquinazoline-7-carboxylate.
$^1$H-NMR (400 MHz, DMSO-$d_6$) d (ppm): 2.42 (s, 3H), 3.86 (s, 3H), 7.40 (d, 1H, J = 8.2 Hz), 7.84 (d, 1H, J = 8.2 Hz), 10.49 (s, 1H), 11.51 (s, 1H); ESI-MS (m/z): 235 $[M+H]^+$.

Sixth Step

[Chemical Formula 193]

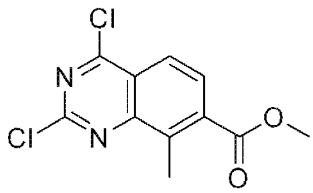

A mixture of the product (0.4 g, 1.7 mmol) of the fifth step, N,N-dimethylaniline (0.6 mL) and phosphoryl chloride (3.29 mL) was refluxed for 7 hours. After cooling to room temperature, the solvent was distilled off under reduced pressure and the residue was added to ice water, followed by extraction with ethyl acetate. The resulting organic layer was dried over anhydrous sodium sulfate and the solvent was distilled off under reduced pressure, and then the residue was purified by silica gel chromatography (ethyl acetate:hexane= 3:7) to obtain 0.38 g of methyl 2,4-dichloro-8-methylquinazoline-7-carboxyate.
$^1$H-NMR (400 MHz, $CDCl_3$) d (ppm) : 2.95 (s, 3H), 3.99 (s, 3H), 8.02 (d, 1H, J = 8.8 Hz), 8.12 (d, 1H, J = 8.8 Hz).

Seventh Step

[Chemical Formula 194]

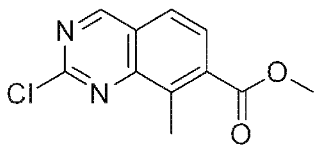

To an ethyl acetate solution (40 mL) of the product (0.5 g, 1.85 mmol) of the sixth step and N,N-diisopropylethylamine (0.35 mL, 0.28 mmol), 10% palladium carbon (60 mg) was added, followed by stirring under a hydrogen gas flow at room temperature for 2 hours. To the reaction mixture, water was added and insoluble substances were filtered through cerite, and then the organic layer was separated from the filtrate. The resulting organic layer was dried over anhydrous sodium sulfate and the solvent was distilled off under reduced pressure, and then the residue was purified by silica gel chromatography (ethyl acetate:hexane = 1:9) to obtain 0.145 g of methyl 2-chloro-8-methylquinazoline-7-carboxylate.
$^1$H-NMR (400 MHz, $CDCl_3$) d (ppm): 2.81 (s, 3H), 3.94 (s, 3H), 8.0 (d, 1H, J = 8.4 Hz), 8.15 (d, 1H, J = 8.5 Hz), 9.67 (s, 1H); ESI-MS (m/z): 237 $[M+H]^+$.

Example 113

Preparation of methyl 2-(1H-indazol-6-yl)-8-methylquinazoline-7-carboxylate

[Chemical Formula 195]

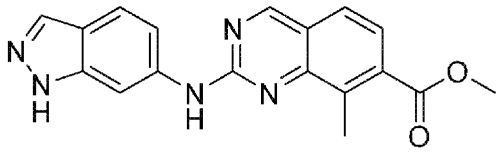

110 mg of the titled compound was obtained by reacting and treating methyl 2-chloro-8-methylquinazoline-7-carboxylate (see Reference Example 7) (150 mg, 0.63 mmol) and 6-aminoindazole (90 mg, 0.66 mmol) in the same manner as in Example 44.
$^1$H-NMR (400 MHz, DMSO-$d_6$) d (ppm): 2.88 (s, 3H), 3.96 (s, 3H), 7.44 (d, 1H, J = 8.7 Hz), 7.63 (d, 1H , J = 8.4 Hz), 7.66 (d, 1H, J = 8.8 Hz), 7.86 (d, 1H, J = 8.3 Hz), 7.95 (s, 1H), 8.68 (s, 1H), 9.39 (s, 1H), 10.25 (s, 1H), 12.97 (br, 1H); ESI-MS (m/z): 334 $[M+H]^+$.

Example 114

Preparation of 2-(1H-indazol-6-yl)-8-methylquinazoline-7-carboxylic acid

[Chemical Formula 196]

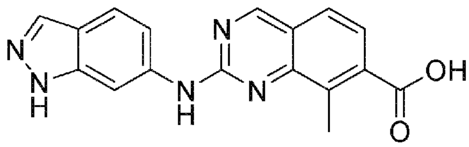

The compound (120 mg, 0.36 mmol) of Example 113 was dissolved in a 1:1 solution (10 mL) of methanol and THF and an aqueous 5 N sodium hydroxide solution (0.1 mL) was added, followed by stirring a room temperature for 30 hours. The reaction mixture was concentrated under reduced pressure and water (2 mL) was added to the resulting residue, followed by extraction with ethyl acetate. The resulting organic layer was dried over anhydrous sodium sulfate and the solvent was distilled off under reduced pressure to obtain 50 mg of the titled compound.
$^1$H-NMR (400 MHz, DMSO-$d_6$) d (ppm): 2.88 (s, 3H), 7.44 (d, 1H, J = 8.6 Hz), 7.60 (d, 1H, J = 8.0 Hz), 7.66 (d, 1H, J = 8.4 Hz), 7.82 (d, 1H, J = 8.0 Hz), 7.94 (s, 1H), 8.68 (s, 1H), 9.36 (s, 1H), 10.17 (s, 1H), 12.94 (br, 1H); ESI-MS (m/z): 320 $[M+H]^+$.

Example 115

Preparation of [2-(1H-indazol-6-yl)-8-methylquinazolin-7-yl]methanol

[Chemical Formula 197]

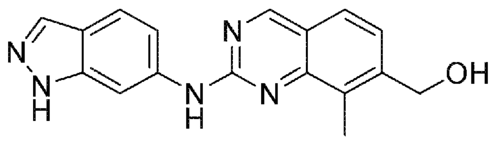

The compound (50 mg, 0.15 mmol) of Example 113 was added to a THF solution (8 mL) of lithium aluminum

hydride (18 mg, 0.54 mmol) under a nitrogen gas flow while cooling to 0°C, followed by stirring for one hour. The reaction mixture was diluted with ethyl acetate and insoluble substances were filtered through cerite. The filtrate was distilled off under reduced pressure to obtain 6 mg of the titled compound.
$^1$H-NMR (400 MHz, DMSO-$d_6$) d (ppm): 2.64 (s, 3H), 4.74 (d, 2H, J = 5.2 Hz), 5.36 (t, 1H, J = 5.2 Hz), 7.43 (d, 1H, J = 7.5 Hz), 7.53 (d, 1H, J = 8.1 Hz), 7.64 (d, 1H, J = 8.6 Hz), 7.77 (d, 1H, J = 8.1 Hz), 7.93 (s, 1H), 8.70 (s, 1H), 9.26 (s, 1H), 10.00 (s, 1H), 12.93 (s, 1H); ESI-MS (m/z): 306 $[M+H]^+$.

Example 116

Preparation of ethyl 1-methyl-6-(8-methylquinazolin-2-ylamino)-1H-benzo[d]imidazol-4-ylcarbamate

[Chemical Formula 198]

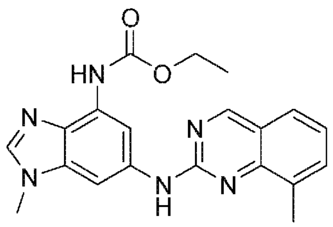

First Step

[Chemical Formula 199]

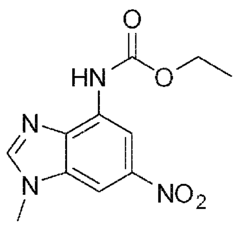

To an acetone solution (15 mL) of ethyl 6-nitro-1H-benzo[d]imidazol-4-ylcarbamate (see Example 77) (250 mg, 1.0 mmol), potassium carbonate (50 mg, 0.15 mmol) and methyl iodide (50 mg, 0.15 mmol) were added, followed by stirring at room temperature for 12 hours. The reaction mixture was concentrated under reduced pressure and water (2 mL) was added to the resulting residue, followed by extraction with ethyl acetate. The organic layer was dried over sodium sulfate and the solvent was distilled off under reduced pressure, and then the residue was purified by silica gel chromatography (ethyl acetate:n-hexane = 3:7) to obtain 185 mg of ethyl 1-methyl-6-nitro-1H-benzo[d]imidazol-4-ylcarbamate.
$^1$H-NMR (400 MHz, DMSO-$d_6$) d (ppm): 1.2-1.3 (m, 3H), 3.95 (s, 3H), 4.20 (q, 2H, J = 6.9 Hz), 8.3-8.4 (m, 1H), 8.50 (s, 1H), 8.6-8.7 (m, 1H); ESI-MS (m/z): 265 $[M+H]^+$.

Second Step

[Chemical Formula 200]

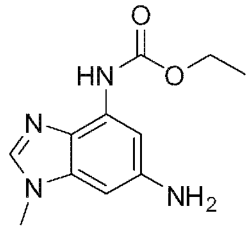

To a methanol-dichloromethane mixed solution (3:1, 20 mL) of the product (180 mg, 0.68 mmol) of the first step, 10% palladium carbon (18 mg) was added, followed by stirring under a hydrogen gas flow at room temperature for 2 hours. The reaction mixture was filtered through cerite and the solvent was distilled off under reduced pressure to obtain 42 mg of ethyl 6-amino-1-methyl-1H-benzo[d]imidazol-4-ylcarbamate.
ESI-MS (m/z): 235 $[M+H]^+$.

Third Step

60 mg of ethyl 1-methyl-6-(8-methylquinazolin-2-ylamino)-1H-benzo[d]imidazol-4-ylcarbamate was obtained by reacting and treating the product (50 mg, 0.21 mmol) of the second step and 6-aminoindazole (38 mg, 0.21 mmol) in the same manner as in Example 44.
$^1$H-NMR (400 MHz, DMSO-$d_6$) d (ppm): 1.29 (t, 3H, J = 7.0 Hz), 2.72 (s, 3H), 4.04 (s, 3H), 4.2-4.3 (m, 2H), 7.36 (t, 1H, J = 7.4 Hz), 7.76 (d, 1H, J = 7.0 Hz), 7.81 (d, 1H, J = 7.8 Hz), 7.92 (s, 1H), 8.95 (s, 1H), 9.35 (s, 1H), 9.93 (br, 1H), 10.45 (s, 1H); ESI-MS (m/z): 377 $[M+H]^+$.

Example 117

Preparation of N-(3-methyl-1H-indazol-6-yl)-7-hydroxy-8-methylquinazoline-2-amine

[Chemical Formula 201]

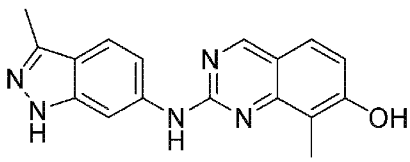

3.5 mg of the titled compound was obtained by reacting and treating the compound (10 mg, 0.031 mmol) of Example 96 in the same manner as in Example 44.
$^1$H-NMR (400 MHz, $CD_3OD$) d (ppm): 2.54 (s, 3H), 2.59 (s, 3H), 6.97 (d, 1H, J = 8.4 Hz), 7.23 (d, 1H, J = 8.4 Hz), 7.53 (d, 1H, J = 8.4 Hz), 7.59 (d, 1H, J = 8.8 Hz), 8.72 (s, 1H), 8.89 (s, 1H); ESI-MS (m/z): 306 $[M+H]^+$.

Reference Example 8

Preparation of 7-fluoro-8-methylquinazoline-2-amine

[Chemical Formula 202]

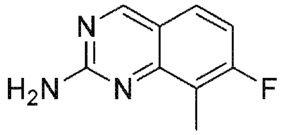

406 mg of the titled compound was obtained by reacting and treating 2-chloro-7-fluoro-8-methylquinazoline (see Reference Example 3) (0.50 g, 2.54 mmol) in the same manner as in Reference Example 6.
$^1$H-NMR (400 MHz, DMSO-$d_6$) d: 2.35 (d, 3H, J = 2.0 Hz), 6.97 (s, 2H), 7.10 (t, 1H, J = 9.2 Hz), 7.71 (dd, 1H, J = 8.6, 6.8 Hz), 9.06 (s, 1H); ESI-MS (m/z): 178 $[M+H]^+$.

Example 118

Preparation of 7-fluoro-N-(4-methoxy-1H-indazol-6-yl)-8-methylquinazoline-2-amine

[Chemical Formula 203]

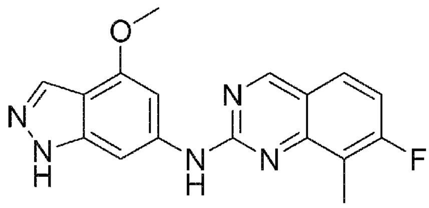

34 mg of the titled compound was obtained by reacting and treating 7-fluoro-8-methylquinazoline-2-amine (see Reference Example 8) (54.2 mg, 0.306 mmol) in the same manner as in Example 71.
$^1$H-NMR (400 MHz, DMSO-$d_6$) d (ppm): 2.54 (d, 3H, J = 2.0 Hz), 3.94 (s, 3H), 7.3-7.4 (m, 2H), 7.88 (dd, 1H, J = 8.8, 6.4 Hz), 7.93 (d, 1H, J = 1.2 Hz), 8.08 (s, 1H), 9.30 (s, 1H), 10.09 (s, 1H); ESI-MS (m/z): 324 $[M+H]^+$.

Example 119

Preparation of N-[6-(8-methylquinazolin-2-ylamino)-1H-indazol-4-yl]methanesulfonamide

[Chemical Formula 204]

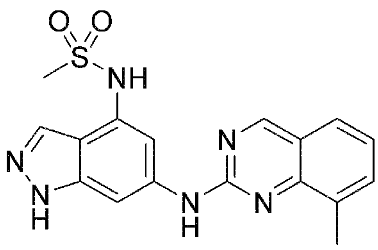

To a dimethylacetamide solution (1 mL) of the compound (15 mg, 0.052 mmol) of Example 52 and triethylamine (10.5 mg, 0.103 mmol), a dimethylacetamide solution (0.1 mL) of methanesulfonyl chloride (8.88 mg, 0.078 mmol) was added, followed by stirring at room temperature for 12 hours. The reaction mixture was diluted with ethyl acetate, washed

with water and then dried over sodium sulfate. The solvent was distilled off under reduced pressure and the residue was purified by silica gel chromatography (ethyl acetate:n-hexane = 1:1 to 6:1) to obtain 5.0 mg of the titled compound.
$^1$H-NMR (400 MHz, DMSO-$d_6$) d (ppm): 2.71 (s, 3H), 3.10 (s, 3H), 7.3-7.4 (m, 1H), 7.44 (d, 1H, J = 2.0 Hz), 7.71 (d, 1H, J = 7.2 Hz), 7.77 (d, 1H, J = 8.0 Hz), 8.19 (s, 1H), 8.48 (s, 1H), 9.30 (s, 1H), 9.97 (br, 1H), 10.06 (s, 1H), 12.97 (s, 1H); ESI-MS (m/z): 324 $[M+H]^+$.

Example 120

Preparation of 8-methyl-N-(4-amino-1-methyl-1H-benzo[d]imidazol-6-yl)quinazoline-2-amine

[Chemical Formula 205]

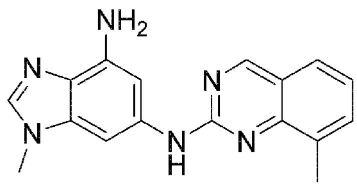

To a dioxane solution (1.2 mL) of the compound (45 mg, 0.12 mmol) of Example 116, an aqueous 2N sodium hydroxide solution (0.6 mL) was added, followed by reflux for 12 hours. To the reaction solution, methanol (2 mL) and an aqueous 2N sodium hydroxide solution (0.6 mL) were added, followed by reflux for 5 hours. The reaction mixture was concentrated under reduced pressure and water (2 mL) was added to the resulting residue, followed by extraction with ethyl acetate. The resulting organic layer was washed in turn with water and a saturated brine solution and dried over anhydrous sodium sulfate, and then the solvent was distilled off under reduced pressure. The resulting solid was suspended in diethylether and then collected by filtration to obtain 8 mg of the titled compound.
$^1$H-NMR (400 MHz, DMSO-$d_6$) d (ppm): 2.68 (s, 3H), 3.75 (s, 3H), 5.20 (s, 2H), 6.62 (s, 1H), 7.26 (t, 1H, J = 7.4 Hz), 7.68 (d, 1H, J = 7.2 Hz), 7.73 (d, 1H, J = 8.0 Hz), 7.86 (s, 1H), 8.15 (s, 1H), 9.22 (s, 1H), 9.74 (s, 1H); ESI-MS (m/z): 305 $[M+H]^+$.

Example 121

Preparation of 2-(1H-indazol-6-ylamino)-8-methylquinazoline-7-carboxamide

[Chemical Formula 206]

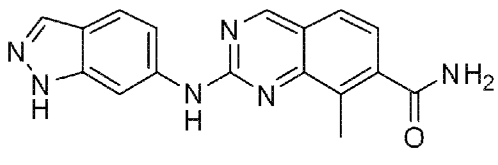

To a DMF solution (0.4 mL) of the compound (50 mg, 0.13 mmol) of Example 114 and HATU (90 mg, 0.195 mmol), ammonium chloride (34 mg, 0.26 mmol) and diisopropylethylamine (40 mg, 0.52 mmol) were added under ice cooling, followed by stirring at room temperature for 24 hours. The reaction mixture was diluted with ethyl acetate and then washed in turn with an aqueous 0.5 N sodium hydroxide solution and water. The resulting organic layer was dried over sodium sulfate and the solvent was distilled off under reduced pressure, and then the residue was purified by preparative HPLC to obtain 3.0 mg of the titled compound.
$^1$H-NMR (400 MHz, DMSO-$d_6$) d (ppm): 2.7 (s, 3H), 7.30 (d, 1H J = 7.44 Hz), 7.43 (d, 1H, J = 8.16 Hz), 7.64 (s, 2H),

7.81 (d, 1H, J = 7.36 Hz), 7.94 (s, 2H), 8.72 (s, 1H), 9.33 (s, 1H), 10.15 (s, 1H), 12.94 (s, 1H); ESI-MS (m/z): 319 $[M+H]^+$.

Example 122

Preparation of ethyl 6-(8-methylquinazolin-2-ylamino)-1H-indol-4-ylcarbamate

[Chemical Formula 207]

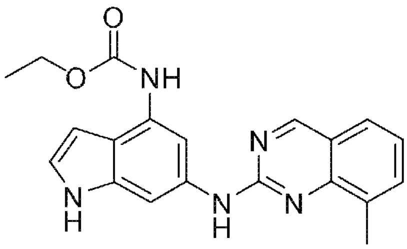

First Step

[Chemical Formula 208]

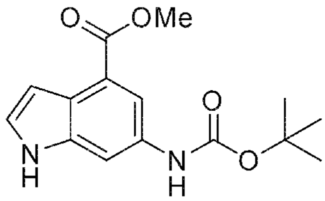

6-aminoindole-4-methyl carboxylate hydrochloride (1.5 g, 6.62 mmol) was added to a mixed solution of a THF solution (20 mL) and an aqueous 4 N sodium hydroxide solution (4 mL). To the reaction solution, di-tert-butyl dicarbonate (1.73 g, 7.92 mmol) was added, followed by stirring at room temperature for 3 hours. The organic layer was separated and washed with an aqueous saturated ammonium chloride solution (2 mL), and then the solvent was distilled off under reduced pressure. The resulting residue was washed with a mixed solution (1:20) of THF and heptane and then collected by filtration to obtain 1.83 g of methyl 6-(tert-butoxycarbonylamino)-1H-indole-4-carboxylate.
$^1$H-NMR (400 MHz, DMSO-$d_6$) d (ppm): 1.49 (s, 9H), 3.87 (s, 3H), 6.82 (s, 1H), 7.40 (s, 1H), 7.8-7.9 (m, 2H), 9.40 (br, 1H), 11.25 (s, 1H); ESI-MS (m/z): 291 $[M+H]^+$.

Second Step

[Chemical Formula 209]

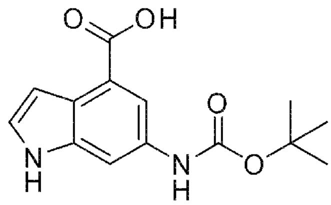

To a THF solution (15 mL) of the product (1.8 g, 6.206 mmol) of the first step, an aqueous 12 N sodium hydroxide solution (5 mL) was added, followed by stirring at room temperature for 12 hours and further stirring at 50°C for 4 days. THF was distilled off under reduced pressure and the residue was neutralized with citric acid, followed by extraction with ethyl acetate. The resulting organic layer was washed in turn with water and a saturated brine solution and dried over sodium sulfate, and then the solvent was distilled off under reduced pressure to obtain 1.38 g of 6-(tert-butoxycarbonylamino)-1H-indole-4-carboxylic acid.
$^1$H-NMR (400 MHz, DMSO-$d_6$) d (ppm): 1.49 (s, 9H), 6.82 (s, 1H), 7.35 (s, 1H), 7.81 (s, 1H), 7.87 (s, 1H), 9.34 (s, 1H), 11.17 (s, 1H), 12.52 (br, 1H); ESI-MS (m/z): 277 $[M+H]^+$.

Third Step

[Chemical Formula 210]

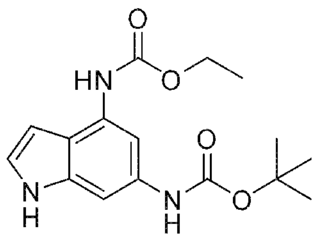

To a toluene solution (10 mL) of the product (0.1 g, 0.362 mmol) of the second step, triethylamine (51 mg, 0.5 mmol) was added and then diphenylphosphoric acid azide (120 mg, 0.436 mmol) was added. After the reaction mixture was refluxed for one hour, ethanol (0.5 mL) was added, followed by reflux for 2 hours. After cooling to room temperature, an aqueous 1N sodium hydroxide solution (10 mL) was added to the reaction mixture, followed by extraction with ethyl acetate. The resulting organic layer was washed in turn with water and a saturated brine solution and then dried over anhydrous sodium sulfate. The solvent was distilled off under reduced pressure, the residue was purified by silica gel chromatography (methanol:dichloromethane = 1:99) to obtain 108 mg of ethyl 6-(tert-butoxycarbonylamino)-1H-indol-4-ylcarbamate.
$^1$H-NMR (400 MHz, DMSO-$d_6$) d (ppm): 1.33 (t, 3H, J = 7.0 Hz), 1.51 (s, 9H), 4.25 (q, 2H, J = 7.0 Hz), 6.37 (s, 1H), 6.55 (br, 1H), 6.75 (br, 1H), 7.09 (s, 1H), 7.35 (br, 1H), 7.65 (s, 1H), 8.16 (br, 1H); ESI-MS (m/z): 320 $[M+H]^+$.

Fourth Step

[Chemical Formula 211]

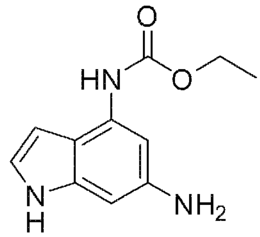

To a dichloromethane solution (5 mL) of the product (0.1 g, 0.313 mmol) of the third step, trifluoroacetic acid (1 mL) was added, followed by stirring at room temperature for 2 hours. The reaction mixture was concentrated under reduced pressure and an aqueous saturated sodium hydrogen carbonate solution (10 mL) was added to the resulting residue, followed by extraction with ethyl acetate. The organic layer was washed in turn with water and a saturated brine solution and dried over sodium sulfate, and then the solvent was distilled off under reduced pressure to obtain 57 mg of ethyl 6-amino-1H-indol-4-ylcarbamate.
ESI-MS (m/z): 220 $[M+H]^+$.

Fifth Step

[Chemical Formula 212]

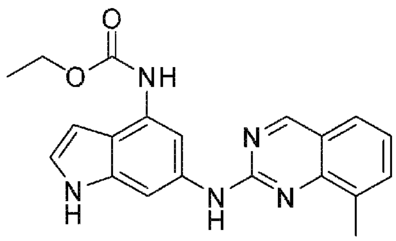

A DMSO solution (2 mL) of the product (0.123 g, 0.561 mmol) of the fourth step, 2-chloro-8-methylquinazoline (see Reference Example 1) (0.1 mg, 0.651 mmol) and cesium carbonate (0.183 g, 0.561 mmol) was stirred at 100°C for one hour and half. The reaction solution was cooled to room temperature and the precipitated solid was collected by filtration. The resulting solid was purified by preparative HPLC to obtain 50 mg of ethyl 6-(8-methylquinazolin-2-ylamino)-1H-indol-4-ylcarbamate.
$^1$H-NMR (400 MHz, DMSO-$d_6$) d (ppm): 1.28 (t, 3H, J = 7.2 Hz), 2.66 (s, 3H), 4.16 (q, 2H, J = 7.1 Hz), 6.64 (s, 1H), 7.13 (d, 1H, J = 2.3 Hz), 7.24 (t, 1H, J = 7.6 Hz), 7.66 (d, 1H, J = 6.9 Hz), 7.71 (d, 1H, J = 7.9 Hz), 7.86 (s, 1H), 8.14 (s, 1H), 9.21 (s, 1H), 9.24 (s, 1H), 9.69 (s, 1H), 10.99 (s, 1H); ESI-MS (m/z): 362 $[M+H]^+$.

Example 123

Preparation of 7-fluoro-8-methyl-N-(4-amino-1H-benzo[d]imidazol-6-yl)quinazoline-2-amine

[Chemical Formula 213]

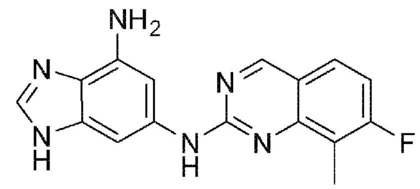

First Step

[Chemical Formula 214]

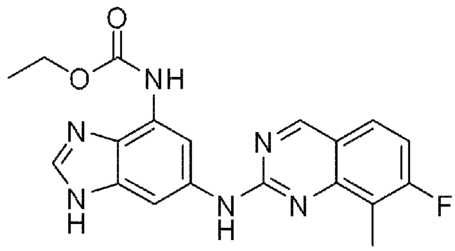

220 mg of ethyl 6-(7-fluoro-8-methylquinazolin-2-ylamino)-1H-benzo[d]imidazol-4-ylcarbamate was obtained by reacting and treating ethyl 6-amino-1H-benzo[d]imidazol-4-ylcarbamate (see Example 77) (180 mg, 0.81 mmol) and 2-chloro-7-fluoro-8-methylquinazoline (see Reference Example 3) (145 mg, 0.88 mmol) in the same manner as in Example 44.
$^1$H-NMR (400 MHz, DMSO-$d_6$) d (ppm): 1.2-1.4 (m, 3H), 2.56 (s, 3H), 4.2-4.3 (m, 2H), 7.2-7.4 (m, 1H), 7.8-7.9 (m, 1H), 8.05 (s, 1H), 8.66 (s, 1H), 9.32 (s, 1H), 9.42 (s, 1H), 9.97(s,1H), 10.43 (s, 1H), 14.67 (br,1H); ESI-MS (m/z): 381 $[M+H]^+$.

Second Step

To a dioxane solution (5.5 mL) of the product (220 mg, 0.5 mmol) of the first step, an aqueous 2 N sodium hydroxide solution (2.5 mL) was added, followed by reflux for 24 hours. The reaction mixture was extracted with ethyl acetate, washed in turn with water and a saturated brine solution and then dried over anhydrous sodium sulfate. The solvent was distilled off under reduced pressure to obtain 150 mg of 7-fluoro-8-methyl-N-(4-amino-1H-benzo[d]imidazol-6-yl)quinazoline-2-amine.
$^1$H-NMR (400 MHz, DMSO-$d_6$) d: 2.54 (s, 3H), 5.15 (s, 2H), 6.75 (s, 1H), 7.19 (t, 1H, J= 9.1Hz), 7.7-7.9 (m, 2H), 7.88 (s, 1H), 9.19 (s, 1H), 9.70 (s, 1H), 12.11 (s, 1H); ESI-MS (m/z): 309 $[M+H]^+$.

Example 124

Preparation of 2-[2-(4-amino-1H-benzo[d]imidazol-6-yl)-8-methylquinazolin-7-ylamino]ethanol

[Chemical Formula 215]

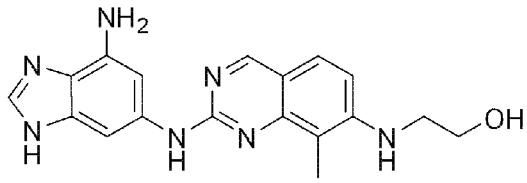

8 mg of the titled compound was obtained by reacting and treating the compound (75 mg, 0.24 mmol) of Example 123 and 2-aminoethanol (45 mg, 0.73 mmol) in the same manner as in Example 56.
$^1$H-NMR (400 MHz, DMSO-$d_6$) d (ppm): 2.40 (s, 3H), 3.4-3.8 (m, 4H), 6.9-7.0 (m, 2H), 7.61 (d, 1H, J = 8.7 Hz), 8.17 (s, 1H), 8.87 (s, 1H), 9.28 (s, 1H), 9.74 (br, 1H); ESI-MS (m/z): 350 $[M+H]^+$.

Example 125

Preparation of 8-methyl-N-(4-amino-1H-indol-6-yl)quinazoline-2-amine

[Chemical Formula 216]

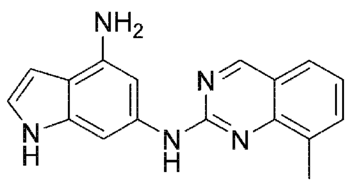

The compound (35 mg, 0.096 mmol) of Example 122 was dissolved in a dioxane/methanol 1:1 mixed solution (3 mL) and an aqueous 2N sodium hydroxide solution (1.5 mL) was added, followed by reflux for 24 hours. The reaction mixture was concentrated under reduced pressure and water (15 mL) was added to the resulting residue, followed by extraction with ethyl acetate. The resulting organic layer was washed in turn with water and a saturated brine solution and dried over anhydrous sodium sulfate, and then the solvent was distilled off under reduced pressure to obtain 21 mg of the titled compound.
$^1$H-NMR (400 MHz, DMSO-$d_6$) d: 2.65 (s, 3H), 5.06 (br, 2H), 6.44 (s, 1H), 6.56 (s, 1H), 6.98 (s, 1H), 7.21 (t, 1H, J = 7.4 Hz), 7.64 (d, 1H, J = 6.8 Hz), 7.68 (d, 1H, J = 7.8 Hz), 7.77 (s, 1H), 9.17 (s, 1H), 9.43 (s, 1H), 10.67 (br, 1H); ESI-MS (m/z): 290 $[M+H]^+$.

Example 126

Preparation of 7-fluoro-8-methyl-N-(4-amino-1-methyl-1H-benzo[d]imidazol-6-yl)quinazoline-2-amine

[Chemical Formula 217]

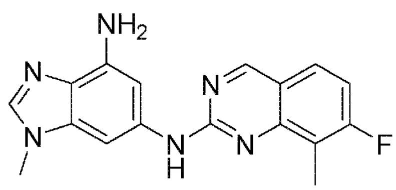

First Step

[Chemical Formula 218]

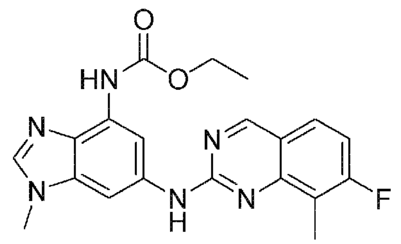

142 mg of ethyl 6-(7-fluoro-8-methylquinazolin-2-ylamino)-1-methyl-1H-benzo[d]imidazol-4-ylcarbamate was obtained by reacting and treating ethyl 6-amino-1-methyl-1H-benzo[d]imidazol-4-ylcarbamate (see Example 116) (100 mg, 0.427 mmol) and 2-chloro-7-fluoro-8-methylquinazoline (see Reference Example 3) (84 mg, 0.427 mmol) in the same manner as in Example 44.
$^1$H-NMR (400 MHz, DMSO-$d_6$) d (ppm): 1.29 (t, 3H, J = 7.0 Hz), 2.59 (s, 3H), 4.03 (s, 3H), 4.1-4.3 (m, 2H), 7.31 (t, 1H, J = 9.2 Hz), 7.8-8.0 (m, 2H), 8.83 (s, 1H), 9.3-9.4 (m, 1H), 9.90 (br, 1H), 10.50 (s, 1H); ESI-MS (m/z): 395 $[M+H]^+$.

Second Step

To a dioxane solution (4 mL) of the product (140 mg, 0.355 mmol) of the first step, an aqueous 2 N sodium hydroxide solution (2 mL) was added, followed by reflux for 12 hours. The reaction mixture was concentrated under reduced pressure and water (15 mL) was added to the resulting residue, followed by extraction with ethyl acetate. The resulting organic layer was washed in turn with water and a saturated brine solution and dried over anhydrous sodium sulfate, and then the solvent was distilled off under reduced pressure to obtain 70 mg of 7-fluoro-8-methyl-N-(4-amino-1-methyl-1H-benzo[d]imidazol-6-yl)quinazoline-2-amine.
$^1$H-NMR (400 MHz, DMSO-$d_6$) d: 2.55 (s, 3H), 3.75 (s, 3H), 5.23 (s, 2H), 6.64 (s, 1H), 7.21 (t, 1H, J = 9.1 Hz), 7.8-7.9 (m, 1H), 7.88 (s, 1H), 8.06 (s, 1H), 9.22 (s, 1H), 9.84 (s, 1H); ESI-MS (m/z): 323 $[M+H]^+$.

Example 127

Preparation of 2-[2-(4-methoxy-1H-indazol-6-ylamino)-8-methylquinazolin-7-ylamino]ethanol

[Chemical Formula 219]

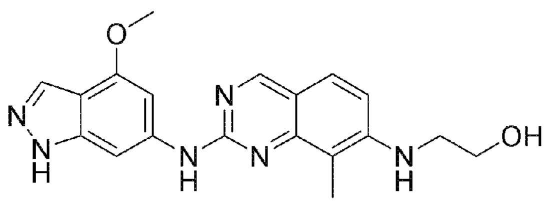

To an NMP solution (0.25 mL) of the compound (14 mg, 0.039 mmol) of Example 118, 2-aminoethanol (14 mg, 0.23 mmol) was added and the reaction was conducted for 3 hours using a microwave synthesizer (manufactured by Biotage, Ltd., 210°C). After cooling to room temperature, the reaction mixture was diluted with ethyl acetate, washed with water and then dried over anhydrous sodium sulfate. The solvent was distilled off under reduced pressure. The resulting residue was purified by silica gel chromatography (chloroform:methanol = 1:0 to 10:1) to obtain 4 mg of the titled compound.
$^1$H-NMR (400 MHz, DMSO-$d_6$) d: 2.44 (s, 3H), 3.3-3.5 (m, 2H), 3.5-3.7 (m, 2H), 3.92 (s, 3H), 4.83 (t, 1H, J = 5.6 Hz),

5.79 (t, 1H, J = 5.6 Hz), 6.93 (d, 1H, J = 8.8 Hz), 7.20 (d, 1H, J = 0.8 Hz), 7.59 (d, 1H, J = 8.8 Hz), 7.88 (d, 1H, J = 1.2 Hz), 8.07 (s, 1H), 8.87 (s, 1H), 9.56 (s, 1H), 12.84 (s, 1H); ESI-MS (m/z): 365 $[M+H]^+$.

Example 128

Preparation of {1-[2-(4-methoxy-1H-indazol-6-ylamino)-8-methylquinazolin-7-yl]piperidin-4-yl}methanol

[Chemical Formula 220]

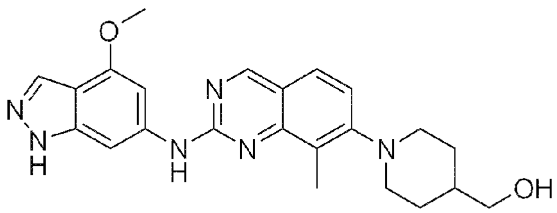

To an NMP solution (0.25 mL) of the compound (18 mg, 0.050 mmol) of Example 118, 4-piperidine methanol (35 mg, 0.30 mmol) was added and the reaction was conducted for 3 hours using a microwave synthesizer (manufactured by Biotage, Ltd., 210°C). After cooling to room temperature, the reaction mixture was diluted with ethyl acetate, washed with water and then dried over anhydrous sodium sulfate. The solvent was distilled off under reduced pressure to obtain a mixture of {1-[2-(4-methoxy-1H-indazol-6-ylamino)-8-methylquinazolin-7-yl]piperidin-4-yl}methanol and {1-[2-(4-hydroxy-1H-indazol-6-ylamino)-8-methylquinazolin-7-yl]piperidin-4-yl}methanol. The resulting mixture was purified by silica gel chromatography (chloroform:methanol = 1:0 to 3:1) to obtain 5.5 mg of the titled compound (TLC: Rf = 0.42, chloroform: methanol = 9:1).
$^1$H-NMR (400 MHz, DMSO-$d_6$) d: 1.3-1.5 (m, 2H), 1.5-1.6 (m, 1H), 1.7-1.9 (m, 2H), 2.59 (s, 3H), 2.7-2.9 (m, 2H), 3.3-3.5 (m, 4H), 3.95 (s, 3H), 4.52 (t, 1H, J = 5.2 Hz), 7.21 (d, 1H, J = 8.8 Hz), 7.34 (d, 1H, J = 1.2 Hz), 7.71 (d, 1H, J = 8.4 Hz), 7.89 (s, 1H), 8.05 (s, 1H), 9.13 (s, 1H), 9.83 (s, 1H), 12.84 (s, 1H); ESI-MS (m/z): 419 $[M+H]^+$.

Example 129

Preparation of {1-[2-(4-hydroxy-1H-indazol-6-ylamino)-8-methylquinazolin-7-yl]piperidin-4-yl}methanol

[Chemical Formula 221]

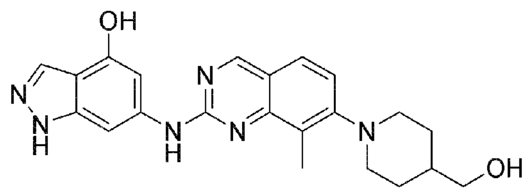

The reaction mixture of Example 128 was purified by silica gel chromatography (chloroform:methanol = 1:0 to 3:1) to obtain 3.2 mg of the titled compound (TLC: Rf = 0.27, chloroform:methanol = 9:1).
$^1$H-NMR (400 MHz, DMSO-$d_6$) d (ppm): 1.3-1.5 (m, 2H), 1.5-1.7 (m, 1H), 1.7-1.9 (m, 2H), 2.59 (s, 3H), 2.78 (t, 2H, J = 11.8 Hz), 3.3-3.5 (m, 4H), 4.5-4.6 (m, 1H), 6.77 (s, 1H), 7.20 (d, 1H, J = 8.8 Hz), 7.71 (d, 1H, J = 8.8 Hz), 7.91 (s, 1H), 8.23 (s, 1H), 9.11 (s, 1H), 9.77 (br, 1H), 9.95 (br, 1H), 12.73 (br, 1H); ESI-MS (m/z): 405 $[M+H]^+$.

Example 130

Preparation of N-[4-(aminomethyl)-1H-indazol-6-yl]-8-methylquinazoline-2-amine

[Chemical Formula 222]

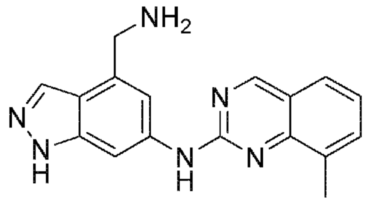

To a methanol solution (1 mL) of the compound (26.2 mg, 0.070 mmol of Example 80, an aqueous 2 N sodium hydroxide solution (0.4 mL) was added and the reaction was conducted for 3 hours using a microwave synthesizer (manufactured by Biotage, Ltd., 100-120°C). The reaction mixture was concentrated under reduced pressure and ethyl acetate was added to the resulting residue, followed by extraction with 2N hydrochloric acid. The resulting aqueous layer was alkalified by adding 4 N sodium hydroxide and then extracted with 2-butanol. The organic layer was distilled off under reduced pressure and the resulting residue was recrystallized from ethanol to obtain a mixture (21 mg) of the titled compound. To the resulting solid (13.6 mg), a 10% methanol hydrochloride solution was added, followed by stirring at room temperature overnight. The solution was neutralized by adding an aqueous saturated sodium hydrogen carbonate solution and then extracted with ethyl acetate. After drying over sodium sulfate, the solvent was distilled off under reduced pressure to obtain 5 mg of the titled compound.
$^{1}$H-NMR (400 MHz, DMSO-$d_6$) d (ppm): 2.71 (s, 3H), 3.51 (s, 2H), 4.01 (s, 2H), 7.31 (t, 1H, J = 7.2 Hz), 7.50 (s, 1H), 7.72 (d, 1H, J = 6.8 Hz), 7.76 (d, 1H, J = 8.0 Hz), 8.01 (s, 1H), 8.53 (s, 1H), 9.29 (s, 1H), 9.99 (s, 1H), 12.87 (s, 1H); ESI-MS (m/z): 305 $[M+H]^+$.

INDUSTRIAL APPLICABILITY

The 2-aminoquinazoline derivatives of the present invention or pharmaceutically acceptable salts thereof are effective as pharmaceuticals for the treatment of diseases which are known to be related to abnormal cell response mediated by protein kinases for example, autoimmune diseases, inflammatory diseases, bone diseases, metabolic disorders, neurological and neurodegenerative disorders, cancers, cardiovascular diseases, allergies and asthma, Alzheimer's disease, and hormonal-related disorders. Furthermore, the 2-aminoquinazoline derivatives of the present invention or pharmaceutically acceptable salts thereof are also useful for research of kinases in biological and pathologically phenomena, and intracellular signal transduction pathway mediated by such kinases; and for comparative evaluations of novel kinase inhibitor.

## Claims

**1.** A 2-aminoquinazoline derivative represented by the following formula (I):

[Chemical Formula 1]

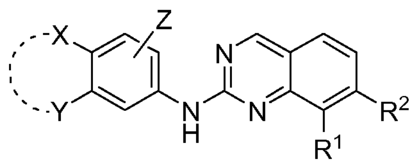

(I)

wherein $R^1$ represents a lower alkyl group which may be substituted with a halogen atom, or a halogen atom; $R^2$ represents a hydrogen atom, a substituted or unsubstituted lower alkyl group, a halogen atom, a hydroxyl group, a substituted or unsubstituted lower alkoxy group, a substituted or unsubstituted amino group, a substituted or unsubstituted acylamino group, a carboxyl group, a lower alkoxycarbonyl group, a carbamoyl group, or a substituted or unsubstituted lower alkylureido group; and X, Y and Z each independently represents a hydrogen atom, a substituted or unsubstituted lower alkyl group, a halogen atom, a hydroxyl group, a carboxyl group, a lower alkoxycarbonyl group, a cyano group, a carbamoyl group, a substituted or unsubstituted lower alkoxy group, a substituted or unsubstituted amino group, a substituted or unsubstituted lower alkoxycarbonylamino group, a substituted or unsubstituted lower alkylaminocarbonyl group, a lower alkylsulfonylamino group, a substituted or unsubstituted lower alkylureido group, or a substituted or unsubstituted acylamino group, or X and Y may be combined to form a 5- to 6-membered ring forming a bicyclic fused ring, wherein the 5- to 6-membered ring may optionally have a substituent, provided that when X and Y are not combined to form a fused ring, $R^2$ represents a hydrogen atom and, when X and Y are combined to form a fused ring, a saturated or unsaturated, bicyclic alicyclic or heterocyclic fused ring can be formed; or a pharmaceutically acceptable salt thereof.

**2.** The 2-aminoquinazoline derivative according to claim 1, wherein in the formula (I), $R^1$ is a lower alkyl group which may be substituted with a halogen atom, or a pharmaceutically acceptable salt thereof.

**3.** The 2-aminoquinazoline derivative according to claim 1, wherein in the formula (I), $R^1$ is a methyl group, or a pharmaceutically acceptable salt thereof.

**4.** The 2-aminoquinazoline derivative according to claim 1, wherein in the formula (I), $R^1$ is a halogen atom, or a pharmaceutically acceptable salt thereof.

**5.** The 2-aminoquinazoline derivative according to claim 1, wherein in the formula (I), $R^1$ is a methyl group and $R^2$ is a hydrogen atom, or a pharmaceutically acceptable salt thereof.

**6.** The 2-aminoquinazoline derivative according to claim 1, wherein in the formula (I), $R^1$ is a methyl group and $R^2$ is a hydroxyl group or a substituted or unsubstituted amino group, or a pharmaceutically acceptable salt thereof.

**7.** The 2-aminoquinazoline derivative according to any one of claims 1 to 6, wherein in the formula (I), X and Y are combined to form a bicyclic fused ring, or a pharmaceutically acceptable salt thereof.

**8.** The 2-aminoquinazoline derivative according to claim 7, wherein the bicyclic fused ring is a heterocyclic fused ring, or a pharmaceutically acceptable salt thereof.

**9.** The 2-aminoquinazoline derivative according to claim 8, wherein the heterocyclic fused ring is a 1H-indazol-6-yl group, or a pharmaceutically acceptable salt thereof.

**10.** The 2-aminoquinazoline derivative according to claim 8, wherein the heterocyclic fused ring is a 1H-indol-6-yl group, or a pharmaceutically acceptable salt thereof.

**11.** The 2-aminoquinazoline derivative according to claim 8, wherein the heterocyclic fused ring is a 1H-benzo[d]imidazol-6-yl group, or a pharmaceutically acceptable salt thereof.

**12.** The 2-aminoquinazoline derivative according to any one of claims 9 to 11, wherein hydrogen atoms of the heterocyclic fused ring each independently may be substituted with Z, or a pharmaceutically acceptable salt thereof.

**13.** The 2-aminoquinazoline derivative according to claim 12, wherein Z each independently represents a methyl group, an amino group or a hydroxyl group, or a pharmaceutically acceptable salt thereof.

**14.** A 2-aminoquinazoline derivative represented by the following formula (II):

[Chemical Formula 2]

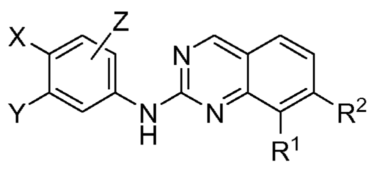

(II)

wherein $R^1$ represents a lower alkyl group which may be substituted with a halogen atom, or a halogen atom; $R^2$ represents a hydrogen atom; and X, Y and Z each independently represents a hydrogen atom, a substituted or unsubstituted lower alkyl group, a halogen atom, a hydroxyl group, a carboxyl group, a cyano group, a carbamoyl group, a substituted or unsubstituted lower alkoxy group, a substituted or unsubstituted amino group, or a substituted or unsubstituted acylamino group; or a pharmaceutically acceptable salt thereof.

**15.** The 2-aminoquinazoline derivative according to claim 14, wherein in the formula (II), $R^1$ is a lower alkyl group which may be substituted with a halogen atom, or a pharmaceutically acceptable salt thereof.

**16.** The 2-aminoquinazoline derivative according to claim 14, wherein in the formula (II), $R^1$ is a halogen atom; or a pharmaceutically acceptable salt thereof.

**17.** The 2-aminoquinazoline derivative according to any one of claims 14 to 16, wherein X and Y each independently represents a hydrogen atom, a hydroxyl group, a substituted or unsubstituted lower alkoxy group, a substituted or unsubstituted amino group, or a substituted or unsubstituted acylamino group, or a pharmaceutically acceptable salt thereof.

**INTERNATIONAL SEARCH REPORT**

International application No. PCT/JP2008/073866

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D239/84, A61K31/517, A61K31/5377, A61P3/00, A61P5/00, A61P9/00, A61P11/06, A61P19/08, A61P25/00, A61P25/28, A61P29/00, A61P35/00, A61P37/06, A61P37/08, A61P43/00, C07D403/12, C07D405/12, C07D413/12, C07D417/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
WPI, CAplus(STN), REGISTRY(STN), JSTPlus(JDreamII), JMEDPlus(JDreamII)

C. DOCUMENTS CONSIDERED TO BE RELEVANT

| Category* | Citation of document, with indication, where appropriate, of the relevant passages | Relevant to claim No. |
|---|---|---|
| A | WO 2007/117607 A2 (NOVARTIS VACCINES & DIAGNOSTICS, INC.), 18 October, 2007 (18.10.07), (Family: none) | 1-17 |
| A | WO 01/38315 A1 (WARNER-LAMBERT CO.), 31 May, 2001 (31.05.01), & JP 2003-514901 A & US 6982260 B1 & EP 1235815 A & AU 1462101 A & BR 15718 A & CA 2386955 A | 1-17 |
| A | WO 2006/118256 A1 (Kyowa Hakko Kogyo Kabushiki Kaisha), 09 November, 2006 (09.11.06), & WO 2006/118256 A1 | 1-17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 January, 2009 (16.01.09) | 27 January, 2009 (27.01.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2008/073866

C (Continuation). DOCUMENTS CONSIDERED TO BE RELEVANT

| Category* | Citation of document, with indication, where appropriate, of the relevant passages | Relevant to claim No. |
|---|---|---|
| A | WO 2006/105056 A2 (FMC CORP.), 05 October, 2006 (05.10.06), (Family: none) | 1-17 |
| P,A | WO 2008/079988 A2 (NOVARTIS VACCINES & DIAGNOSTICS, INC.), 03 July, 2008 (03.07.08), & WO 2008/079988 A3 | 1-17 |

Form PCT/ISA/210 (continuation of second sheet) (April 2007)

**INTERNATIONAL SEARCH REPORT**

International application No.
PCT/JP2008/073866

Continuation of A. CLASSIFICATION OF SUBJECT MATTER
(International Patent Classification (IPC))

*C07D239/84*(2006.01)i, *A61K31/517*(2006.01)i, *A61K31/5377*(2006.01)i, *A61P3/00*(2006.01)i, *A61P5/00*(2006.01)i, *A61P9/00*(2006.01)i, *A61P11/06*(2006.01)i, *A61P19/08*(2006.01)i, *A61P25/00*(2006.01)i, *A61P25/28*(2006.01)i, *A61P29/00*(2006.01)i, *A61P35/00*(2006.01)i, *A61P37/06*(2006.01)i, *A61P37/08*(2006.01)i, *A61P43/00*(2006.01)i, *C07D403/12*(2006.01)i, *C07D405/12*(2006.01)i, *C07D413/12*(2006.01)i, *C07D417/12*(2006.01)i

(According to International Patent Classification (IPC) or to both national classification and IPC)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2001038315 A
- WO 2005037285 A
- WO 2006039718 A
- WO 2006118256 A
- WO 2007117607 A
- WO 0121598 A
- JP 2502462 B
- US 6040488 A
- US 20040209904 A
- WO 2007014927 A
- WO 9823609 A
- WO 2005117819 A
- WO 2001017982 A
- US 2006194801 A
- JP 04295441 B
- WO 2005084315 A


### Non-patent literature cited in the description


- **Shchemelinin et al.** *Folia Biol,* 2006, vol. 52, 81
- **Scheijen, B. ; Griffin J. D.** *Oncogene,* 2002, vol. 21, 3314
- **Lyman, S. ; Jacobsen, S.** *Blood,* 1998, vol. 91, 1101
- **Nigg et al.** *Nat. Rev. Mol. Cell. Biol.,* 2001, vol. 2, 21
- **Keen, N. ; Taylor, S.** *Nature Review Cancer,* 2004, vol. 4, 927
- **Zhou et al.** *Nat. Genet.,* 1998, vol. 20, 189
- **Tanaka et al.** *Cancer Res.,* 1999, vol. 59, 2041
- **Han et al.** *Cancer Res.,* 2002, vol. 62, 2890
- **Shuai, K. ; Liu, B.** *Nature Review Immunol.,* 2003, vol. 3, 900
- **Seidel et al.** *Oncogene,* 2000, vol. 19, 2645
- **Gordon et al.** *Nature,* 1990, vol. 346, 274
- **Galli.** *N., Engl. J. Med.,* 1993, vol. 328, 257
- **Taylor et al.** *Mol. Cell. Biol.,* 1995, vol. 15, 4149
- **Yousefi et al.** *J. Exp. Med.,* 1996, vol. 183, 1407
- **T. W. Greene.** Protective Groups in Organic Synthesis. John Wiley & Sons, Inc, 1999
- *J. Med. Chem.,* 1991, vol. 34, 217
- *J. Org. Chem.,* 2006, vol. 71, 3959
- **T. W. Greene.** Protective Groups in Organic Synthesis. John Wiley & Sons, Inc, 1999, 249
- *J. Org. Chem.,* 1960, vol. 25, 942